# EUROPEAN PATENT SPECIFICATION

(11) **EP 2 822 555 B1**
(45) Date of publication and mention of the grant of the patent: **08.11.2017**
(21) Application number: 13709722.6
(22) Date of filing: 06.03.2013
(51) Int. Cl.: A61K 31/436, A61K 31/00, A61K 31/519, A61P 25/02, A61P 25/00, A61K 31/35, A61K 31/5025

(54) **INHIBITION OF ADAPTOR ASSOCIATED KINASE 1 FOR THE TREATMENT OF PAIN**
HEMMUNG VON ADAPTER-ASSOZIIERTER KINASE 1 ZUR BEHANDLUNG VON SCHMERZEN
INHIBITION DE LA KINASE 1 ASSOCIÉE À UN ADAPTATEUR POUR LE TRAITEMENT DE LA DOULEUR

(30) Priority: 09.03.2012 US 201261608849 P
(43) Date of publication of application: 14.01.2015
(60) Divisional application: 17175353.6
(73) Proprietor: Lexicon Pharmaceuticals, Inc., The Woodlands, TX 77381 (US)
(72) Inventor: LANTHORN, Thomas Herbert, The Woodlands, Texas 77382 (US); SAVELIEVA, Katerina, The Woodlands, Texas 77381 (US); ZAMBROWICZ, Brian, The Woodlands, Texas 77382 (US)
(74) Representative: Abel & Imray
(86) International application number: PCT/US2013/029258
(87) International publication number: WO 2013/134336

(56) References cited:
- WO-A1-2008/052734
- WO-A2-2007/025090
- WO-A2-2009/140128
- Srinivas G Rao: "Current progress in the pharmacological therapy of fibromyalgia", Expert Opinion on Investigational Drugs, vol. 18, no. 10, 1 October 2009 (2009-10-01), pages 1479-1493, XP055201832, ISSN: 1354-3784, DOI: 10.1517/13543780903203771

## Description

### 1. FIELD OF THE INVENTION

This invention relates to methods of treating pain by inhibiting adaptor associated kinase 1 (AAK1).

### 2. BACKGROUND OF THE INVENTION

Pain can generally be divided into three different types: acute, inflammatory, and neuropathic. Acute pain by its very nature generally is short lasting and intense. Inflammatory pain on the other hand may last for much longer periods of time and its intensity is more graded. Inflammation may occur for many reasons, including tissue damage, autoimmune response, and pathogen invasion. The third class of pain is neuropathic and is presumed to involve nerve damage that results in reorganization of neuronal proteins and circuits to yield a pathologic "sensitized" state that can produce chronic pain lasting for years. This type of pain is particularly difficult to treat with existing therapies.

Pain, particularly neuropathic and intractable pain, is a large unmet medical need. Millions of individuals suffer from severe pain that is not well controlled by current therapeutics. The current drugs used to treat pain include NSAIDS, COX2 inhibitors, opioids, tricyclic antidepressants, and anticonvulsants. Neuropathic pain has been particularly difficult to treat as it does not respond well to opioids until high doses are reached. Gabapentin is currently the favored therapeutic for the treatment of neuropathic pain, although it works in only 60% of patients, where it shows modest efficacy.

Adaptor associated kinase 1 (AAK1) is a member of the Ark1/Prk1 family of serine/threonine kinases. AAK1 mRNA exists in two splice forms termed short and long. The long form predominates and is highly expressed in brain and heart (Henderson and Conner, Mol. Biol. Cell. 2007, 18, 2698-2706). AAK1 is enriched in synaptosomal preparations and is co-localized with endocytic structures in cultured cells. AAK1 modulates clatherin coated endocytosis, a process that is important in synaptic vesicle recycling and receptor-mediated endocytosis. AAK1 associates with the AP2 complex, a hetero-tetramer which links receptor cargo to the clatherin coat. The binding of clatherin to AAK1 stimulates AAK1 kinase activity (Conner et. al., Traffic 2003, 4, 885-890; Jackson et. al., J. Cell. Biol. 2003, 163, 231-236). AAK1 phosphorylates the mu-2 subunit of AP-2, which promotes the binding of mu-2 to tyrosine containing sorting motifs on cargo receptors (Ricotta et. al., J. Cell Bio. 2002, 156, 791-795; Conner and Schmid, J. Cell Bio. 2002, 156, 921-929). Mu2 phosphorylation is not required for receptor uptake, but phosphorylation enhances the efficiency of internalization (Motely et. al., Mol. Biol. Cell. 2006, 17, 5298-5308).

AAK1 has been identified as an inhibitor of Neuregulin-1/ErbB4 signaling in PC12 cells. Loss of AAK1 expression through RNA interference mediated gene silencing or treatment with the kinase inhibitor K252a (which inhibits AAK1 kinase activity) results in the potentiation of Neuregulin-1 induced neurite outgrowth. These treatments result in increased expression of ErbB4 and accumulation of ErbB4 in or near the plasma membrane (Kuai et. al., Chemistry and Biology 2011, 18, 891-906). NRG1 and ErbB4 are putative schizophrenia susceptibility genes (Buonanno, Brain Res. Bull. 2010, 83, 122-131). SNPs in both genes have been associated with multiple schizophrenia endophenotypes (Greenwood et. al., Am. J. Psychiatry 2011, 168, 930-946). Neuregulin 1 and ErbB4 KO mouse models have shown schizophrenia relevant morphological changes and behavioral phenotypes (Jaaro-Peled et. al., Schizophrenia Bulletin 2010, 36, 301-313; Wen et. al., Proc. Natl. Acad. Sci. USA. 2010, 107, 1211-1216). In addition, a single nucleotide polymorphism in an intron of the AAK1 gene has been associated with the age of onset of Parkinson's disease (Latourelle et. al., BMC Med. Genet. 2009, 10, 98). Srinivas (2009) Exp. Opin. Investig. Drugs 18(10) 1479-1493 provides a review of pharmacological therapy of fibromyalgia.

### 3. SUMMARY OF THE INVENTION

This invention is based upon Applicants' seminal discovery that inhibition of AAK1 can mitigate pain, and upon the subsequent discovery of multiple classes of AAK1 inhibitors.

The invention itself relates to an inhibitor of adaptor associated kinase 1 (AAK1) activity according to claim 1 for use in a method of treating or managing fibromyalgia. Subject-matter which is not encompassed by the scope of claim 1 does not form part of the presently claimed invention.

### 4. BRIEF DESCRIPTION OF THE FIGURES

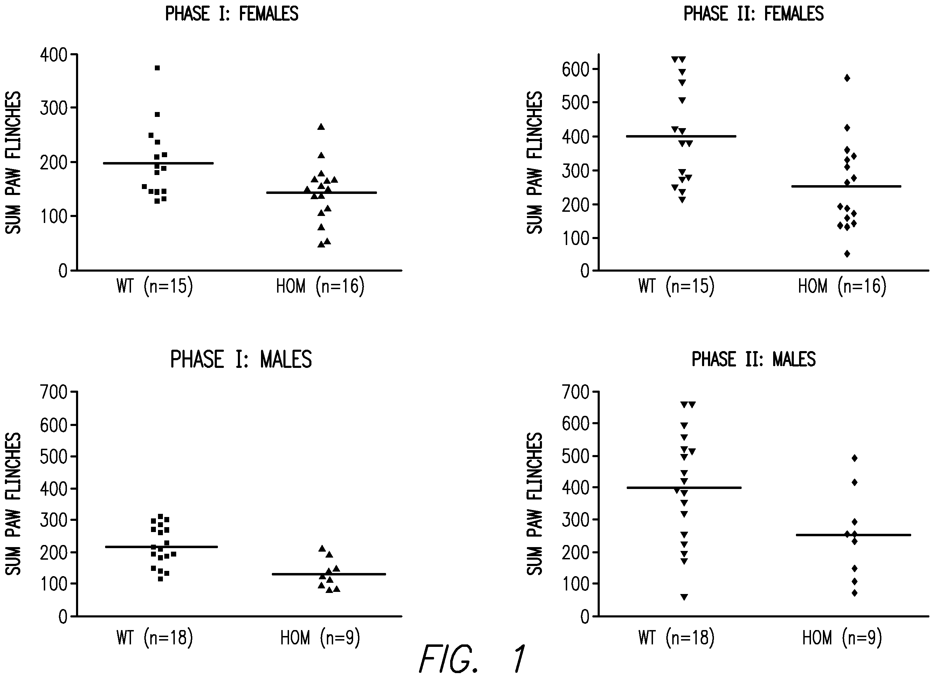

Aspects of the invention are illustrated in Figure 1, which shows results obtained from a formalin pain model using AAK1 homozygous (-/-) knockout mice and their wild-type (+/+) littermates. The AAK1 homozygous (-/-) knockout mice show a clear reduction in both acute and tonic pain response as compared to their wild-type (+/+) littermates.

### 5. DETAILED DESCRIPTION OF THE INVENTION

This invention is based on Applicants' discovery that AAK1 knockout mice exhibit a high resistance to pain. That discovery prompted research that ultimately led to the discovery of a wide range of AAK1 inhibitors that may be used in the treatment and management of fibromyalgia. In short, this invention provides an entirely new mechanism by which fibromyalgia may be treated or managed, as well as compounds useful therein.

### 5.1. DEFINITIONS

Unless otherwise indicated, the phrases "compounds of the invention," "compounds of the present disclosure," and the like refer to the compounds disclosed herein.

Unless otherwise indicated, the term "hydrocarbyl" means an aliphatic or alicyclic moiety having an all-carbon backbone and consisting of carbon and hydrogen atoms. Examples of hydrocarbyl groups include those having 1-20, 1-12, 1-6, and 1-4 carbon atoms (referred to as C₁₋₂₀ hydrocarbyl, C₁₋₁₂ hydrocarbyl, C₁₋₆ hydrocarbyl, and C₁₋₄ hydrocarbyl, respectively). Particular examples include alkyl, alkenyl, alkynyl, aryl, benzyl, cycloalkyl, cycloalkenyl, cycloalkylalkyl, cycloalkenylalkyl, napthyl, phenyl, and phenylethyl.

Examples of alkyl moeites include straight-chain and branched moieties having 1-20, 1-12, 1-6, 1-4 and 1-3 carbon atoms (referred to as C₁₋₂₀ alkyl, C₁₋₁₂ alkyl, C₁₋₆ alkyl, C₁₋₄ alkyl and C₁₋₃ alkyl, respectively). Particular examples include methyl, ethyl, propyl, isopropyl, n-butyl, t-butyl, isobutyl, pentyl, hexyl, isohexyl, heptyl, 4,4-dimethylpentyl, octyl, 2,2,4-trimethylpentyl, nonyl, decyl, undecyl and dodecyl.

Examples of alkenyl moieties include straight-chain and branched C₂₋₂₀, C₂₋₁₂ and C₂₋₆ alkenyl. Particular examples include vinyl, allyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, 1-hexenyl, 2-hexenyl, 3-hexenyl, 1-heptenyl, 2-heptenyl, 3-heptenyl, 1-octenyl, 2-octenyl, 3-octenyl, 1-nonenyl, 2-nonenyl, 3-nonenyl, 1-decenyl, 2-decenyl and 3-decenyl.

Examples of alkynyl moeites include include straight-chain and branched C₂₋₂₀, C₂₋₁₂ and C₂₋₆ alkynyl. Particular examples include ethynyl and 2-propynyl (propargyl).

Examples of aryl moeites include anthracenyl, azulenyl, fluorenyl, indan, indenyl, naphthyl, phenyl and phenanthrenyl.

Examples of cycloalkyl moeites include C₃₋₁₂, C₃₋₇, C₄₋₆ and C₆ cycloalkyl. Particular examples include cyclopropyl, cyclobutyl, cyclopentyl, cyclohexyl and adamantyl.

Unless otherwise indicated, the term "halo" encompass fluoro, chloro, bromo, and iodo.

Unless otherwise indicated, the term "heterocarbyl" refers to a moiety having a backbone made up of one or more carbon atoms and one or more heteroatoms. Particular heteroatoms are nitrogen, oxygen and sulfur. A heterocarbyl moieties can be thought of as a hydrocarbyl moiety wherein at least one carbon atom, CH, CH₂, or CH₃ group is replaced with one or more heteroatoms and the requisite number of hydrogen atoms to satisy valencies. Examples of heterocarbyl include 2-20, 2-12, 2-8, 2-6 and 2-4 membered heterocarbyl moieties, wherein the number range refers to the sum total of carbon, nitrogen, oxygen, and/or sulfur atoms in the moiety. The term "2-12 membered heterocarbyl" thus refers to a heterocarbyl moiety having a total of 2-12 carbon, nitrogen, oxygen, and/or sulfur atoms. Particular heterocarbyl moeites include straight chain and branched heteroalkyl, heteroalkenyl, and heteroalkynyl, as well as heterocycle and heteroaryl.

Examples of heteroalkyl moieties include 2-8-membered, 2-6-membered and 2-4-membered heteroalkyl moieties. Particular examples include alkoxyl, acyl (e.g., formyl, acetyl, benzoyl), alkylamino (e.g., di-(C₁₋₃-alkyl)amino), arylamino, aryloxime, carbamates, carbamides, alkylcarbonyl, arylcarbonyl, aminocarbonyl, alkylaminocarbonyl, alkylsulfanyl, arylsulfanyl, alkylsulfinyl, arylsulfinyl, alkylsulfonyl, arylsulfonyl, alkylsulfonylamino, and arylsulfonylamino.

Unless otherwise indicated, the term "heterocycle" refers to a cyclic (monocyclic or polycyclic) heterocarbyl moieity which may be aromatic, partially aromatic or non-aromatic. Heterocycles include heteroaryls. Examples include 4-10-membered, 4-7-membered, 6-membered, and 5-membered heterocycles. Particular examples include benzo[1,3]dioxolyl, 2,3-dihydrobenzo[1,4]dioxinyl, cinnolinyl, furanyl, hydantoinyl, morpholinyl, oxetanyl, oxiranyl, piperazinyl, piperidinyl, pyrrolidinonyl, pyrrolidinyl, tetrahydrofuranyl, tetrahydropyranyl, tetrahydropyridinyl, tetrahydropyrimidinyl, tetrahydrothiophenyl, tetrahydrothiopyranyl and valerolactamyl. Because the term "heterocycle" refers to a ring, standing alone it does not encompass moieities such as oxazolidinone and imidazolidinone: such moieties are considered substituted heterocycles, *viz.* heterocycles substituted with oxo.

Examples of heteroaryl moieties include acridinyl, benzimidazolyl, benzofuranyl, benzoisothiazolyl, benzoisoxazolyl, benzoquinazolinyl, benzothiazolyl, benzoxazolyl, furyl, imidazolyl, indolyl, isothiazolyl, isoxazolyl, oxadiazolyl, oxazolyl, phthalazinyl, pyrazinyl, pyrazolyl, pyridazinyl, pyridyl, pyrimidinyl, pyrimidyl, pyrrolyl, quinazolinyl, quinolinyl, tetrazolyl, thiazolyl, and triazinyl.

Unless otherwise indicated, the term "include" has the same meaning as "include, but are not limited to," and the term "includes" has the same meaning as "includes, but is not limited to." Similarly, the term "such as" has the same meaning as the term "such as, but not limited to."

Unless otherwise indicated, the terms "manage," "managing" and "management" encompass preventing the recurrence of the specified disease or disorder in a patient who has already suffered from the disease or disorder, and/or lengthening the time that a patient who has suffered from the disease or disorder remains in remission. The terms encompass modulating the threshold, development and/or duration of the disease or disorder, or changing the way that a patient responds to the disease or disorder.

Unless otherwise indicated, a "therapeutically effective amount" of a compound is an amount sufficient to provide a therapeutic benefit in the treatment or management of a disease or condition, or to delay or minimize one or more symptoms associated with the disease or condition. A "therapeutically effective amount" of a compound means an amount of therapeutic agent, alone or in combination with other therapies, that provides a therapeutic benefit in the treatment or management of the disease or condition. The term "therapeutically effective amount" can encompass an amount that improves overall therapy, reduces or avoids symptoms or causes of a disease or condition, or enhances the therapeutic efficacy of another therapeutic agent.

Unless otherwise indicated, the terms "treat," "treating" and "treatment" contemplate an action that occurs while a patient is suffering from the specified disease or disorder, which reduces the severity of the disease or disorder, or retards or slows the progression of the disease or disorder.

Unless otherwise indicated, one or more adjectives immediately preceding a series of nouns is to be construed as applying to each of the nouns. For example, the phrase "optionally substituted alky, aryl, or heteroaryl" has the same meaning as "optionally substituted alky, optionally substituted aryl, or optionally substituted heteroaryl."

### 5.2. COMPOUNDS

Compounds that may be used to inhibit AAK1 are disclosed in U.S. provisional patent application nos. 61/608,758, 61/608,765, and 61/608,737, all filed March 9, 2012, as well as in U.S. patent application nos. 13/777,144, filed February 26, 2013, 13/785,271, filed March 5, 2013, and 13/785,355, also filed March 5, 2013. Compounds encompassed by the present invention are as defined by claim 1.

Compounds of the invention can have one or more asymmetric centers. Unless otherwise indicated, this invention encompasses all stereoisomers of the compounds, as well as mixtures thereof. Individual stereoisomers of compounds can be prepared synthetically from commercially available starting materials which contain chiral centers or by preparation of mixtures of enantiomeric products followed by separation such as conversion to a mixture of diastereomers followed by separation or recrystallization, chromatographic techniques, or direct separation of enantiomers on chiral chromatographic columns. Starting compounds of particular stereochemistry are either commercially available or can be made and resolved by techniques known in the art.

Certain compounds of the present disclosure may also exist in different stable conformational forms which may be separable. Torsional asymmetry due to restricted rotation about an asymmetric single bond, for example because of steric hindrance or ring strain, may permit separation of different conformers. The present disclosure includes each conformational isomer of these compounds and mixtures thereof.

The present disclosure is intended to include all isotopes of atoms occurring in the present compounds. Isotopes include those atoms having the same atomic number but different mass numbers. By way of general example and without limitation, isotopes of hydrogen include deuterium and tritium. Isotopes of carbon include ¹³C and ¹⁴C. Isotopically-labeled compounds of the invention can generally be prepared by conventional techniques known to those skilled in the art or by processes analogous to those described herein, using an appropriate isotopically-labeled reagent in place of the non-labeled reagent otherwise employed. Such compounds may have a variety of potential uses, for example as standards and reagents in determining biological activity. In the case of stable isotopes, such compounds may have the potential to favorably modify biological, pharmacological, or pharmacokinetic properties.

The compounds of the present disclosure can exist as pharmaceutically acceptable salts. The term "pharmaceutically acceptable salt," as used herein, represents salts or zwitterionic forms of the compounds of the present disclosure which are water or oil-soluble or dispersible, which are, within the scope of sound medical judgment, suitable for use in contact with the tissues of patients without excessive toxicity, irritation, allergic response, or other problem or complication commensurate with a reasonable benefit/risk ratio, and are effective for their intended use. The salts can be prepared during the final isolation and purification of the compounds or separately by reacting a suitable nitrogen atom with a suitable acid. Representative acid addition salts include acetate, adipate, alginate, citrate, aspartate, benzoate, benzenesulfonate, bisulfate, butyrate, camphorate, camphorsulfonate; digluconate, dihydrobromide, diydrochloride, dihydroiodide, glycerophosphate, hemisulfate, heptanoate, hexanoate, formate, fumarate, hydrochloride, hydrobromide, hydroiodide, 2-hydroxyethanesulfonate, lactate, maleate, mesitylenesulfonate, methanesulfonate, naphthylenesulfonate, nicotinate, 2-naphthalenesulfonate, oxalate, palmoate, pectinate, persulfate, 3-phenylproprionate, picrate, pivalate, propionate, succinate, tartrate, trichloroacetate, trifluoroacetate, phosphate, glutamate, bicarbonate, para-toluenesulfonate, and undecanoate. Examples of acids which can be employed to form pharmaceutically acceptable addition salts include inorganic acids such as hydrochloric, hydrobromic, sulfuric, and phosphoric, and organic acids such as oxalic, maleic, succinic, and citric.

Basic addition salts can be prepared during the final isolation and purification of the compounds by reacting a carboxy group with a suitable base such as the hydroxide, carbonate, or bicarbonate of a metal cation or with ammonia or an organic primary, secondary, or tertiary amine. The cations of pharmaceutically acceptable salts include lithium, sodium, potassium, calcium, magnesium, and aluminum, as well as nontoxic quaternary amine cations such as ammonium, tetramethylammonium, tetraethylammonium, methylamine, dimethylamine, trimethylamine, triethylamine, diethylamine, ethylamine, tributylamine, pyridine, N,N-dimethylaniline, N-methylpiperidine, N-methylmorpholine, dicyclohexylamine, procaine, dibenzylamine, N,N-dibenzylphenethylamine, and N,N'-dibenzylethylenediamine. Other representative organic amines useful for the formation of base addition salts include ethylenediamine, ethanolamine, diethanolamine, piperidine, and piperazine.

Particular compounds inhibit AAK1 with an IC₅₀ of less than 0.1, 0.01 or 0.001 µM as measured in the P81 filter plate assay described below in the Examples. Particular compounds inhibit AAK1 with an IC₅₀ of less than 0.1, 0.01 or 0.001 µM as measured in the HEK281 cell-based assay described described below in the Examples.

### 5.2.1. Imidazo[1,2-b]pyridazine-based Inhibitors

One class of compounds that may be used in embodiments of the invention is disclosed in U.S. patent application no. 13/785,271, filed March 5, 2013, entitled "Imidazo[1,2-b]pyridazine-based Compounds, Compositions Comprising Them, and Methods of Their Use." That application encompasses compounds of the formula: 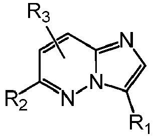 and pharmaceutically acceptable salts thereof, wherein: R₁ is R_{1A} or optionally substituted C₁₋₁₂ hydrocarbyl or 2-12-membered heterocarbyl, which optional substitution is with one or more R_{1A}; each R_{1A} is independently -OR_{1C}, -N(R_{1C})₂, -C(O)R_{1C}, -C(O)OR_{1C}, -C(O)N(R_{1C})₂, -N(R_{1C})C(O)OR_{1C}, cyano, halo, or optionally substituted C₁₋₁₂ hydrocarbyl or 2-12-membered heterocarbyl, which optional substitution is with one or more R_{1B}; each R_{1B} is independently -OR_{1C}, - -N(R_{1C})₂, -C(O)R_{1C}, -C(O)OR_{1C}, - C(O)N(R_{1C})₂, -N(R_{1C})C(O)OR_{1C}, cyano or halo; each R_{1C} is independently hydrogen or optionally substituted C₁₋₁₂ hydrocarbyl or 2-12-membered heterocarbyl, which optional substitution is with one or more of cyano, halo or hydroxyl; R₂ is -NR_{2A}R_{2B}, wherein R_{2A} is hydrogen and R_{2B} is optionally substituted C₁₋₁₂ hydrocarbyl or 2-12-membered heterocarbyl, which optional substitution is with one or more R_{2C}; or R_{2A} and R_{2B} are taken together to form a 4-7-membered heterocycle optionally substituted with one or more R_{2C}; each R_{2C} is independently -OR_{2D}, -N(R_{2D})₂, -C(O)R_{2D}, -C(O)OR_{2D}, -C(O)N(R_{2D})₂, -N(R_{2D})C(O)OR_{2D}, cyano, halo, or optionally substituted C₁₋₁₂ hydrocarbyl or 2-12-membered heterocarbyl, which optional substitution is with one or more with one or more R_{2D}; each R_{2D} is independently hydrogen or optionally substituted C₁₋₁₂ hydrocarbyl or 2-12-membered heterocarbyl, which optional substitution is with one or more of cyano, halo or hydroxyl; and R₃ is hydrogen or C₁₋₆ alkyl optionally substituted with one or more of cyano, halo or hydroxyl.

In particular compounds, R₁ is R_{1A}. In particular compounds, R₁ is optionally substituted C₁₋₁₂ hydrocarbyl. In particular compounds, R₁ is optionally substituted phenyl. In particular compounds, R₁ is optionally substituted 2-12-membered heterocarbyl (e.g., 2-8 membered heterocarbyl, 2-6 membered heterocarbyl, 2-6 membered heterocarbyl). In particular compounds, R₁ is optionally substituted pyridinyl, thiophen, or imidazol.

In particular compounds, R_{1A} is halo. In some, R_{1A} is -OR_{1C}, -N(R_{1C})₂, -C(O)R_{1C}, -C(O)OR_{1C}, or -C(O)N(R_{1C})₂. In some, R_{1A} is -OR_{1C}. In some, R_{1B} is -N(R_{1C})₂, -OR_{1C}, halo.

In particular compounds, R_{2A} and R_{2B} are taken together to form a 4-7-membered heterocycle optionally substituted with one or more R_{2C}.

In particular compounds, R_{1C} is hydrogen. In some, R_{1C} is C₁₋₁₂ hydrocarbyl (e.g., C₁₋₆ hydrocarbyl, C₁₋₄ hydrocarbyl such as methyl, ethyl, propyl).

In particular compounds, R_{2C} is -C(O)OR₂D, -C(O)N(R_{2D})₂, or -N(R_{2D})C(O)OR₂D.

In particular compounds, R_{2D} is hydrogen. In some, R_{2D} is C₁₋₁₂ hydrocarbyl (e.g., C₁₋₆ hydrocarbyl, C₁₋₄ hydrocarbyl such as methyl, ethyl, propyl).

In particular compounds, R₃ is hydrogen.

One embodiement of the invention encompasses compounds of the formula: 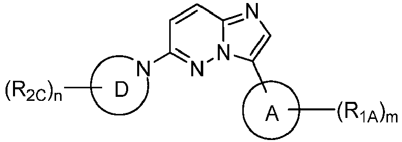 and pharmaceutically acceptable salt thereof, wherein: A is cyclic C₁₋₁₂ hydrocarbyl or 4-7-membered heterocycle; D is 4-7-membered heterocycle; each R_{1A} is independently-OR_{1C}, -N(R_{1C})₂, -C(O)R_{1C}, -C(O)OR_{1C}, -C(O)N(R_{1C})₂, -N(R_{1C})C(O)OR_{1C}, cyano, halo, or optionally substituted C₁₋₁₂ hydrocarbyl or 2-12-membered heterocarbyl, which optional substitution is with one or more R_{1B}; each R_{1B} is independently -OR_{1C}, N(R_{1C})₂, -C(O)R_{1C}, -C(O)OR_{1C}, -C(O)N(R_{1C})₂, -N(R_{1C})C(O)OR_{1C}, cyano or halo; each R_{1C} is independently hydrogen or optionally substituted C₁₋₁₂ hydrocarbyl or 2-12-membered heterocarbyl, which optional substitution is with one or more of cyano, halo or hydroxyl; each R_{2C} is independently -OR_{2D}, -N(R_{2D})₂, -C(O)R_{2D}, -C(O)OR_{2D}, -C(O)N(R_{2D})₂, -N(R_{2D})C(O)OR_{2D}, cyano, halo, or optionally substituted C₁₋₁₂ hydrocarbyl or 2-12-membered heterocarbyl, which optional substitution is with one or more with one or more R_{2D}; each R_{2D} is independently hydrogen or optionally substituted C₁₋₁₂ hydrocarbyl or 2-12-membered heterocarbyl, which optional substitution is with one or more of cyano, halo or hydroxyl; n is 1-3; and m is 0-3.

In particular compounds, R_{2C} is not hydroxyl or optionally substituted phenyl or pyridinyl.

In particular compounds, when D is diazapine and A is pyridinyl, R_{2C} is not -C(O)O-tert-butyl.

In particular compounds, when D is piperazin, A is phenyl and R_{1A} is chloro, R_{2C} is not -C(O)O-tert-butyl.

In particular compounds, when D is piperidinyl, A is pyridinyl and R_{1A} is chloro, R_{2C} is not --NHC(O)O-tert-butyl.

In particular compounds, when D is piperidinyl, A is pyridinyl and R_{1A} is -NHCH₂CH₂CH(CH₃)₂, R_{2C} is not NH₂.

One embodiment of the invention encompasses compounds of the formula: 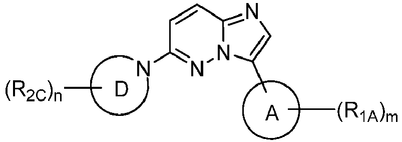 and pharmaceutically acceptable salt thereof, wherein: A is cyclic C₁₋₁₂ hydrocarbyl or 4-7-membered heterocycle; D is 4-7-membered heterocycle; each R_{1A} is independently-OR_{1C}, -N(R_{1C})₂, -C(O)R_{1C}, -C(O)OR_{1C}, -C(O)N(R_{1C})₂, -N(R_{1C})C(O)OR_{1C}, cyano, halo, or optionally substituted C₁₋₁₂ hydrocarbyl or 2-12-membered heterocarbyl, which optional substitution is with one or more R_{1B}; each R_{1B} is independently-OR_{1C}, -N(R_{1C})₂, -C(O)R_{1C}, -C(O)OR_{1C}, -C(O)N(R_{1C})₂, -N(R_{1C})C(O)OR_{1C}, cyano or halo; each R_{1C} is independently hydrogen or optionally substituted C₁₋₁₂ hydrocarbyl or 2-12-membered heterocarbyl, which optional substitution is with one or more of cyano, halo or hydroxyl; each R_{2C} is independently -OR_{2D}, -N(R_{2D})₂, -C(O)R_{2D}, -C(O)OR_{2D}, -C(O)N(R_{2D})₂, -N(R_{2D})C(O)OR_{2D}, cyano, halo, or optionally substituted C₁₋₁₂ hydrocarbyl or 2-12-membered heterocarbyl, which optional substitution is with one or more with one or more R_{2D}; each R_{2D} is independently hydrogen or optionally substituted C₁₋₁₂ hydrocarbyl or 2-12-membered heterocarbyl, which optional substitution is with one or more of cyano, halo or hydroxyl; n is 1-3; and m is 0-3.

In particular compounds encompassed by this embodiment: 1) R_{2C} is not hydroxyl or optionally substituted phenyl or pyridinyl; 2) when D is diazapine and A is pyridinyl, R_{2C} is not --C(0)0-tert-butyl; 3) when D is piperazin, A is phenyl and R_{1A} is chloro, R_{2C} is not -C(O)O-tert-butyl; 4) when D is piperidinyl, A is pyridinyl and R_{1A} is chloro, R_{2C} is not -NHC(O)O-tert-butyl; and 5) when D is piperidinyl, A is pyridinyl and R_{1A} is -NHCH₂CH₂CH(CH₃)₂, R_{2C} is not NH₂.

In particular compounds, D is piperazin or pyrrolidin.

In particular compounds, n is 1.

In particular compounds, m is 1.

In particular compounds, A is pyridinyl, thiophen, or imidazol.

Particular compounds of the invention are of the formula: 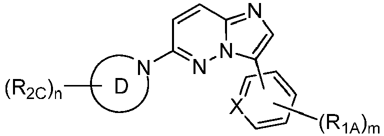 wherein X is CH or N.

One embodiment of the invention encompasses compounds of the formula: 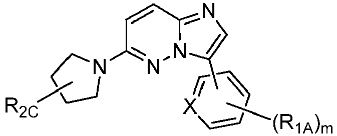 and pharmaceutically acceptable salt thereof, wherein: X is CH or N; each R_{1A} is independently -OR_{1C}, -N(R_{1C})₂, -C(O)R_{1C}, -C(O)OR_{1C}, -C(O)N(R_{1C})₂, -N(R_{1C})C(O)OR_{1C}, cyano, halo, or optionally substituted C₁₋₁₂ hydrocarbyl or 2-12-membered heterocarbyl, which optional substitution is with one or more R_{1B}; each R_{1B} is independently -OR_{1C}, -N(R_{1C})₂, -C(O)R_{1C}, -C(O)OR_{1C}, -C(O)N(R_{1C})₂, -N(R_{1C})C(O)OR_{1C}, cyano or halo; each R_{1C} is independently hydrogen or optionally substituted C₁₋₁₂ hydrocarbyl or 2-12-membered heterocarbyl, which optional substitution is with one or more of cyano, halo or hydroxyl; each R_{2C} is independently -OR_{2D}, -N(R_{2D})₂, -C(O)R_{2D}, -C(O)OR_{2D}, -C(O)N(R_{2D})₂, -N(R_{2D})C(O)OR_{2D}, cyano, halo, or optionally substituted C₁₋₁₂ hydrocarbyl or 2-12-membered heterocarbyl, which optional substitution is with one or more with one or more R_{2D}; each R_{2D} is independently hydrogen or optionally substituted C₁₋₁₂ hydrocarbyl or 2-12-membered heterocarbyl, which optional substitution is with one or more of cyano, halo or hydroxyl; and m is 0-3.

In some compounds of this embodiment, R_{2C} is not optionally substituted phenyl or pyridinyl.

Particular compounds of the invention are of the formula:

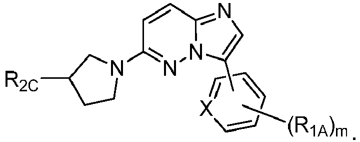

Particular compounds of the invention are of the formula:

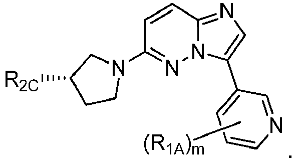

Particular compounds of the invention are of the formula:

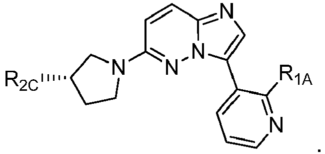

One embodiment of the invention encompasses compounds of the formula: 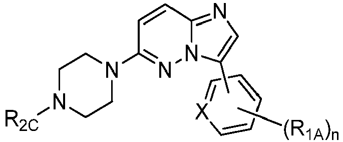 and pharmaceutically acceptable salts thereof, wherein: X is CH or N; each R_{1A} is independently -OR_{1C}, -N(R_{1C})₂, -C(O)R_{1C}, -C(O)OR_{1C}, -C(O)N(R_{1C})₂, -N(R_{1C})C(O)OR_{1C}, cyano, halo, or optionally substituted C₁₋₁₂ hydrocarbyl or 2-12-membered heterocarbyl, which optional substitution is with one or more R_{1B}; each R_{1B} is independently -OR_{1C}, N(R_{1C})₂, -C(O)R_{1C}, -C(O)OR_{1C}, -C(O)N(R_{1C})₂, -N(R_{1C})C(O)OR_{1C}, cyano or halo; each R_{1C} is independently hydrogen or optionally substituted C₁₋₁₂ hydrocarbyl or 2-12-membered heterocarbyl, which optional substitution is with one or more of cyano, halo or hydroxyl; each R_{2C} is independently -OR_{2D}, -N(R_{2D})₂, -C(O)R_{2D}, -C(O)OR_{2D}, -C(O)N(R_{2D})₂, -N(R_{2D})C(O)OR_{2D}, cyano, halo, or optionally substituted C₁₋₁₂ hydrocarbyl or 2-12-membered heterocarbyl, which optional substitution is with one or more with one or more R_{2D}; each R_{2D} is independently hydrogen or optionally substituted C₁₋₁₂ hydrocarbyl or 2-12-membered heterocarbyl, which optional substitution is with one or more of cyano, halo or hydroxyl; and m is 0-3.

In some compounds of this embodiment, when X is CH, m is 1 and R_{1A} is chloro, R_{2C} is not t-butyl.

In some compounds of this embodiment, R_{2C} is not optionally substituted phenyl or pyridinyl.

Particular compounds of the invention are of the formula:

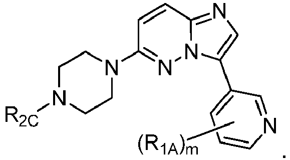

Particular compounds of the invention are of the formula:

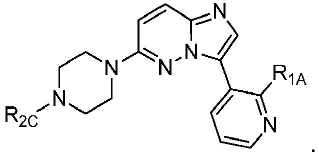

In particular compounds, R_{1A} is -OR_{1C}.

In particular compounds, R_{1C} is optionally substituted C₁₋₁₂ hydrocarbyl (e.g., C₁₋₆ hydrocarbyl, C₁₋₄ hydrocarbyl).

In particular compounds, R_{2C} is -C(O)OR_{2D}, -C(O)N(R_{2D})₂, or -N(R_{2D})C(O)OR₂D.

In particular compounds, each R_{2D} is independently hydrogen or C₁₋₁₂ hydrocarbyl (e.g., C₁₋₆ hydrocarbyl, C₁₋₄ hydrocarbyl).

### 5.2.2. Pyrazolo[1,5-a]pyrimidine-based Inhibitors

Another class of compounds that may be used in embodiments of the invention is disclosed in U.S. patent application no. 13/785,355, filed March 5, 2013, entitled "Pyrazolo[1,5-a]pyrimidine-based Compounds, Compositions Comprising Them, and Methods of Their Use." That application encompasses compounds of the formula: 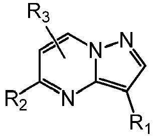 and pharmaceutically acceptable salts thereof, wherein: R₁ is R_{1A} or optionally substituted C₁₋₁₂ hydrocarbyl or 2-12-membered heterocarbyl, which optional substitution is with one or more R_{1A}; each R_{1A} is independently -OR_{1C}, -N(R_{1C})₂, -C(O)R_{1C}, -C(O)OR_{1C}, -C(O)N(R_{1C})₂, -N(R_{1C})C(O)OR_{1C}, cyano, halo, or optionally substituted C₁₋₁₂ hydrocarbyl or 2-12-membered heterocarbyl, which optional substitution is with one or more R_{1B}; each R_{1B} is independently -OR_{1C}, - -N(R_{1C})₂, -C(O)R_{1C}, -C(O)OR_{1C}, -C(O)N(R_{1C})₂, -N(R_{1C})C(O)OR_{1C}, cyano or halo; each R_{1C} is independently hydrogen or optionally substituted C₁₋₁₂ hydrocarbyl or 2-12-membered heterocarbyl, which optional substitution is with one or more of cyano, halo or hydroxyl; R₂ is -NR_{2A}R_{2B}, wherein R_{2A} is hydrogen and R_{2B} is optionally substituted C₁₋₁₂ hydrocarbyl or 2-12-membered heterocarbyl, which optional substitution is with one or more R_{2C}; or R_{2A} and R_{2B} are taken together to form a 4-7-membered heterocycle optionally substituted with one or more R_{2C}; each R_{2C} is independently -OR_{2D}, -N(R_{2D})₂, -C(O)R_{2D}, -C(O)OR_{2D}, - C(O)N(R_{2D})₂, -N(R_{2D})C(O)OR_{2D}, cyano, halo, or optionally substituted C₁₋₁₂ hydrocarbyl or 2-12-membered heterocarbyl, which optional substitution is with one or more with one or more R_{2D}; each R_{2D} is independently hydrogen or optionally substituted C₁₋₁₂ hydrocarbyl or 2-12-membered heterocarbyl, which optional substitution is with one or more of cyano, halo or hydroxyl; and R₃ is hydrogen or C₁₋₆ alkyl optionally substituted with one or more of cyano, halo or hydroxyl.

In particular compounds, R₁ is R_{1A}. In some, R₁ is optionally substituted C₁₋₁₂ hydrocarbyl. In some, R₁ is optionally substituted phenyl. In some, R₁ is optionally substituted 2-12-membered heterocarbyl (e.g., 2-8 membered heterocarbyl, 2-6 membered heterocarbyl, 2-6 membered heterocarbyl). In some, R₁ is optionally substituted pyridinyl, thiophen, or imidazol.

In particular compounds, R_{1A} is halo. In some, R_{1A} is -OR_{1C}, -N(R_{1C})₂, -C(O)R_{1C}, -C(O)OR_{1C}, or -C(O)N(R_{1C})₂. In some, R_{1A} is -OR_{1C}.

In particular compounds, R_{1B} is -N(R_{1C})₂, -OR_{1C}, halo.

In particular compounds, R_{2A} and R_{2B} are taken together to form a 4-7-membered heterocycle optionally substituted with one or more R_{2C}.

In particular compounds, R_{1C} is hydrogen. In some, R_{1C} is C₁₋₁₂ hydrocarbyl (e.g., C₁₋₆ hydrocarbyl, C₁₋₄ hydrocarbyl such as methyl, ethyl, propyl). In some, R_{2C} is -C(O)OR_{2D}, -C(O)N(R_{2D})₂, or -N(R_{2D})C(O)OR_{2D}.

In particular compounds, R_{2D} is hydrogen.

In particular compounds, R_{2D} is C₁₋₁₂ hydrocarbyl (e.g., C₁₋₆ hydrocarbyl, C₁₋₄ hydrocarbyl such as methyl, ethyl, propyl).

In particular compounds, R₃ is hydrogen.

One embodiment of the invention encompasses compounds of the formula: 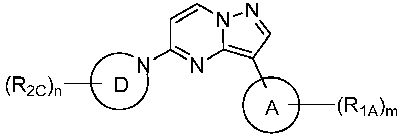 and pharmaceutically acceptable salt thereof, wherein: A is cyclic C₁₋₁₂ hydrocarbyl or 4-7-membered heterocycle; D is 4-7-membered heterocycle; each R_{1A} is independently-OR_{1C}, -N(R_{1C})₂, -C(O)R_{1C}, - C(O)OR_{1C}, -C(O)N(R_{1C})₂, -N(R_{1C})C(O)OR_{1C}, cyano, halo, or optionally substituted C₁₋₁₂ hydrocarbyl or 2-12-membered heterocarbyl, which optional substitution is with one or more R_{1B}; each R_{1B} is independently-OR_{1C}, -N(R_{1C})₂, -C(O)R_{1C}, -C(O)OR_{1C}, -C(O)N(R_{1C})₂, -N(R_{1C})C(O)OR_{1C}, cyano or halo; each R_{1C} is independently hydrogen or optionally substituted C₁₋₁₂ hydrocarbyl or 2-12-membered heterocarbyl, which optional substitution is with one or more of cyano, halo or hydroxyl; each R_{2C} is independently -OR_{2D}, -N(R_{2D})₂, -C(O)R_{2D}, -C(O)OR_{2D}, -C(O)N(R_{2D})₂, -N(R_{2D})C(O)OR_{2D}, cyano, halo, or optionally substituted C₁₋₁₂ hydrocarbyl or 2-12-membered heterocarbyl, which optional substitution is with one or more with one or more R_{2D}; each R_{2D} is independently hydrogen or optionally substituted C₁₋₁₂ hydrocarbyl or 2-12-membered heterocarbyl, which optional substitution is with one or more of cyano, halo or hydroxyl; n is 1-3; and m is 0-3.

In particular compounds, D is not piperidinyl.

In particular compounds, R_{2C} is not -N(R_{2D})₂.

In particular compounds, A is not phenyl.

In particular compounds, m is 1.

In particular compounds, R_{2D} is not ethyl.

In particular compounds, when D is piperidinyl, A is phenyl, and R_{2C} is -N(R_{2D})₂, R_{2D} is not ethyl.

In particular compounds, D is piperazin or pyrrolidin.

In particular compounds, n is 1.

In particular compounds, m is 1.

In particular compounds, A is pyridinyl, thiophen, or imidazol.

Particular compounds are of the formula: 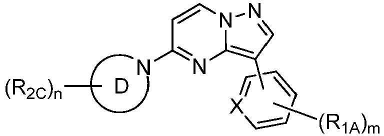 wherein X is CH or N.

Another embodiment of the invention encompasses compounds of the formula: 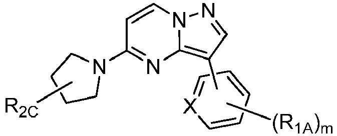 and pharmaceutically acceptable salt thereof, wherein: X is CH or N; each R_{1A} is independently -OR_{1C}, -N(R_{1C})₂, -C(O)R_{1C}, -C(O)OR_{1C}, -C(O)N(R_{1C})₂, -N(R_{1C})C(O)OR_{1C}, cyano, halo, or optionally substituted C₁₋₁₂ hydrocarbyl or 2-12-membered heterocarbyl, which optional substitution is with one or more R_{1B}; each R_{1B} is independently -OR_{1C}, -N(R_{1C})₂, -C(O)R_{1C}, -C(O)OR_{1C}, -C(O)N(R_{1C})₂, -N(R_{1C})C(O)OR_{1C}, cyano or halo; each R_{1C} is independently hydrogen or optionally substituted C₁₋₁₂ hydrocarbyl or 2-12-membered heterocarbyl, which optional substitution is with one or more of cyano, halo or hydroxyl; each R_{2C} is independently -OR_{2D}, -N(R_{2D})₂, -C(O)R_{2D}, -C(O)OR_{2D}, -C(O)N(R_{2D})₂, -N(R_{2D})C(O)OR_{2D}, cyano, halo, or optionally substituted C₁₋₁₂ hydrocarbyl or 2-12-membered heterocarbyl, which optional substitution is with one or more with one or more R_{2D}; each R_{2D} is independently hydrogen or optionally substituted C₁₋₁₂ hydrocarbyl or 2-12-membered heterocarbyl, which optional substitution is with one or more of cyano, halo or hydroxyl; and m is 0-3.

In particular compounds, R_{2C} is not optionally substituted phenyl or pyridinyl.

Particular compounds are of the formula:

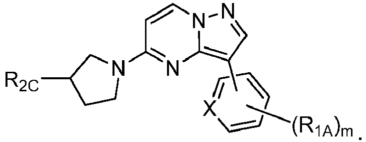

Particular compounds are of the formula:

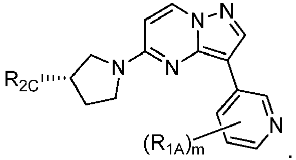

Particular compounds are of the formula:

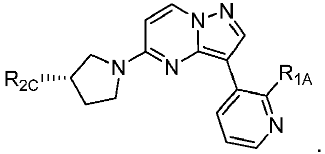

Another embodiment of the invention encompasses compounds of the formula: 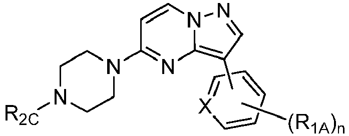 and pharmaceutically acceptable salt thereof, wherein: X is CH or N; each R_{1A} is independently -OR_{1C}, -N(R_{1C})₂, -C(O)R_{1C}, -C(O)OR_{1C}, -C(O)N(R_{1C})₂, -N(R_{1C})C(O)OR_{1C}, cyano, halo, or optionally substituted C₁₋₁₂ hydrocarbyl or 2-12-membered heterocarbyl, which optional substitution is with one or more R_{1B}; each R_{1B} is independently -OR_{1C}, -N(R_{1C})₂, -C(O)R_{1C}, -C(O)OR_{1C}, -C(O)N(R_{1C})₂, -N(R_{1C})C(O)OR_{1C}, cyano or halo; each R_{1C} is independently hydrogen or optionally substituted C₁₋₁₂ hydrocarbyl or 2-12-membered heterocarbyl, which optional substitution is with one or more of cyano, halo or hydroxyl; each R_{2C} is independently -OR_{2D}, -N(R_{2D})₂, -C(O)R_{2D}, -C(O)OR_{2D}, -C(O)N(R_{2D})₂, -N(R_{2D})C(O)OR_{2D}, cyano, halo, or optionally substituted C₁₋₁₂ hydrocarbyl or 2-12-membered heterocarbyl, which optional substitution is with one or more with one or more R_{2D}; each R_{2D} is independently hydrogen or optionally substituted C₁₋₁₂ hydrocarbyl or 2-12-membered heterocarbyl, which optional substitution is with one or more of cyano, halo or hydroxyl; and m is 0-3.

Particular compounds are of the formula:

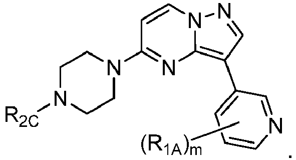

Particular compounds are of the formula:

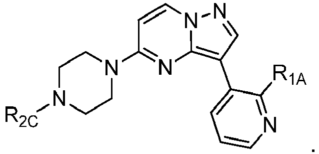

In particular compounds, R_{1A} is -OR_{1C},

In particular compounds, R_{1C} is optionally substituted C₁₋₁₂ hydrocarbyl (e.g., C₁₋₆ hydrocarbyl, C₁₋₄ hydrocarbyl).

In particular compounds, R_{2C} is -C(O)OR_{2D}, -C(O)N(R_{2D})₂, or -N(R_{2D})C(O)OR₂D.

In particular compounds, R_{2D} is independently hydrogen or C₁₋₁₂ hydrocarbyl (e.g., C₁₋₆ hydrocarbyl, C₁₋₄ hydrocarbyl).

### 5.2.3. Aryl ether-based Inhibitors

Another class of compounds that may be used in embodiments of the invention is disclosed in U.S. patent application no. 13/777,144, filed February 26, 2013, entitled "Aryl Ether-Base Kinase Inhibitors." That application encompasses compounds of the formula: 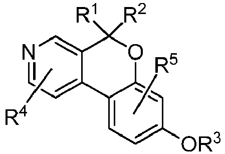 and pharmaceutically acceptable salts thereof, wherein: R¹ and R² are independently selected from hydrogen, C₃-C₆cycloalkyl, and C₁-C₃alkyl wherein the C₁-C₃alkyl is optionally substituted with one, two, or three groups independently selected from C₁-C₃alkoxy, C₁-C₃alkylamino, amino, cyano, C₁-C₃dialkylamino, halo, and hydroxy; or R¹ and R₂ together are oxo; R₃ is C₁-C₃alkyl-Y or C₂-C₈alkyl, wherein the C₂-C₈alkyl is optionally substituted with one, two, or three groups independently selected from C₁-C₃alkoxy, C₁-C₃alkylamino, C₁-C₃alkoxyC₂-C₃alkylamino, amino, aryl, halo, C₁-C₃haloalkylamino, C₁-C₃haloalkylcarbonylamino, hydroxy, -NR^{x}R^{y}, and C₃-C₈cycloalkyl, wherein the cycloalkyl is further optionally substituted with one, two, or three groups independently selected from C₁-C₃alkoxy, C₁-C₃alkyl, C₁-C₃alkylamino, C₁-C₃alkoxyC₂-C₃alkylamino, amino, aryl, arylC₁-C₃alkyl, halo, C₁-C₃haloalkyl, C₁-C₃haloalkylamino and hydroxy; R⁴ is selected from hydrogen, C₁-C₃alkoxy, C₁-C₃alkoxycarbonylamino, C₁-C₃alkyl, C₁-C₃alkylamino, C₁-C₃alkylcarbonylamino, amino, arylamino, arylcarbonylamino, C₃-C₆cycloalkylamino, C₃-C₆cycloalkylcarbonylamino, C₃-C₆cycloalkyloxy, halo, C₁-C₃haloalkoxy, C₂-C₃haloalkylamino, C₂-C₃haloalkylcarbonylamino, and hydroxy; R⁵ is selected from hydrogen, C₁-C₃alkyl, cyano, C₃cycloalkyl, and halo; R^{x} and R^{y}, together with the nitrogen atom to which they are attached, form a three- to six-membered ring; and Y is selected from 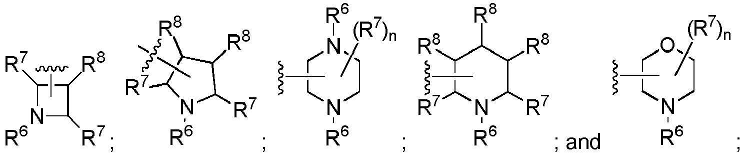 wherein R⁶ is selected from hydrogen, C₁-C₆alkyl, C₃-C₆cycloalkyl, and C₁-C₆alkylcarbonyl; n is 0, 1, 2, or 3; each R⁷ is independently selected from hydrogen, C₁-C₆alkyl, aryl, arylC₁-C₃alkyl, C₃-C₆cycloalkyl, halo, and C₁-C₃haloalkyl; and each R⁸ is independently selected from hydrogen, C₁-C₃alkoxy and hydroxy.

In particular compunds, R¹ and R² are each hydrogen. In some, one of R¹ and R² is hydrogen and the other is C₁-C₃alkyl. In some, one of R¹ and R² is hydrogen and the other is C₃-C₆cycloalkyl. In some, R¹ and R² together are oxo.

In particular compunds, R³ is C₂-C₈alkyl, optionally substituted with an amino group. In some, R³ is C₂-C₈alkyl, optionally substituted with an amino group and an aryl group, wherein the aryl is phenyl.

In particular compunds, R⁴ is hydrogen. In some, R⁴ is amino. In some, R⁴ is C₁-C₃alkylcarbonylamino.

In particular compounds, R⁵ is hydrogen. In some, R⁵ is halo.

### 5.3. METHODS OF USE

This invention is based on the seminal discovery that inhibition of adaptor associated kinase 1 (AAK1)-both as a result of genetic mutation and by the administration of an AAK1 inhibitor-can alleviate pain.

Thus, disclosed is a method of treating or managing pain, which comprises inhibiting AAK1 in patient in need thereof. Particular types of pain include chronic pain, acute pain, and neuropathic pain. Particular types of neuropathic pain include fibromyalgia and peripheral neuropathy (e.g., diabetic neuropathy). The inhibition is achieved by administering to a patient a therapeutically effective amount of an AAK1 inhibitor encompassed by claim 1.

### 5.4. EXAMPLES

Certain aspects of the invention can be understood from the following examples.

### 5.4.1. AAK1 Knockout Mice

Mice homozygous (-/-) for the disruption of the AAK1 gene were prepared by two methods; gene trapping and homologous recombination.

Gene trapping is a method of random insertional mutagenesis that uses a fragment of DNA coding for a reporter or selectable marker gene as a mutagen. Gene trap vectors have been designed to integrate into introns or genes in a manner that allows the cellular splicing machinery to splice vector encoded exons to cellular mRNAs. Commonly, gene trap vectors contain selectable marker sequences that are preceded by strong splice acceptor sequences and are not preceded by a promoter. Thus, when such vectors integrate into a gene, the cellular splicing machinery splices exons from the trapped gene onto the 5' end of the selectable marker sequence. Typically, such selectable marker genes can only be expressed if the vector encoding the gene has integrated into an intron. The resulting gene trap events are subsequently identified by selecting for cells that can survive selective culture.

Embryonic stem cells (Lex-1 cells from derived murine strain A129), were mutated by a process involving the insertion of at least a portion of a genetically engineered vector sequence into the gene of interest, the mutated embryonic stem cells were microinjected into blastocysts which were subsequently introduced into pseudopregnant female hosts and carried to term using established methods. See, e.g., "Mouse Mutagenesis", 1998, Zambrowicz et al., eds., Lexicon Press, The Woodlands, TX. The resulting chimeric animals were subsequently bred to produce offspring capable of germline transmission of an allele containing the engineered mutation in the gene of interest.

AAK1-gene disrupted mice were also made by homologous recombination. In this case, the second coding exon of the murine AAK1 gene (see GenBank Accession Number NM_177762) was removed by methods known in the art. See, e.g., U.S. Patent Nos. 5,487,992, 5,627,059, and 5,789,215.

Mice homozygous (-/-) for the disruption of the AAK1 gene were studied in conjunction with mice heterozygous (+/-) for the disruption of the AAK1 gene, and wild-type (+/+) litter mates. During this analysis, the mice were subject to a medical work-up using an integrated suite of medical diagnostic procedures designed to assess the function of the major organ systems in a mammalian subject. Homozygous (-/-) "knockout" mice were studied in conjunction with their heterozygous (+/-) and wild-type (+/+) litter mates. Disruption of the AAK1 gene was confirmed by Southern analysis. Expression of the murine homolog of AAK1 was detected by RT-PCR in murine brain; spinal cord; eye; thymus; spleen; lung; kidney; liver; skeletal muscle; bone; stomach, small intestine and colon; heart; adipose; asthmatic lung; LPS liver; blood; banded heart; aortic tree; prostate; and mammary gland (5 week virgin, mature virgin, 12 DPC, 3 day post-partum (lactating), 3 day post-weaning (early involution), and 7 day post-weaning (late involution)).

AAK1 homozygous (-/-) and their wild-type (+/+) littermates were tested using the formalin paw test in order to assess their acute and tonic nociceptive responses. For these tests, Automatic Nociception Analyzers (purchased from the Ozaki lab at University of California, San Diego) were used. A metal band was placed around the left hind paw of each mouse 30 minutes prior to testing. After the 30-minute acclimation period, 20 µl of 5% formalin is subcutaneously injected in the dorsal surface of the left hind paw. Mice were individually housed in cylindrical chambers for 45 minutes. A computer recorded flinches per minute, total flinches for phase I (acute phase = first 8 minutes), and total flinches for phase II (tonic phase = time between minutes 20 - 40) through an electromagnetic field. See Yaksh TL, Ozaki G, McCumber D, Rathbun M, Svensson C, Malkmus S, Yaksh MC. An automated flinch detecting system for use in the formalin nociceptive bioassay. J Appl Physiol., 2001; 90:2386-402.

As shown in Figure 1, phase 1 and phase 2 data were obtained using homozygous (-/-) mice females (n = 16), wild-type females (n = 15), homozygous (-/-) mice males (n = 9), and wild-type males (n = 18). In all groups and in both phases, the AAK1 homozygous (-/-) mice exhibited significantly less recorded paw flinching than their wild-type (+/+) littermates.

### 5.4.2. Caliper Assay

The assays were performed in U-bottom 384-well plates. The final assay volume was 30 µl prepared from 15 µl additions of enzyme and substrates (fluoresceinated peptide (5-FAM)-Aha-KEEQSQITSQVTGQIGWR-NH2 and ATP) and test compounds in assay buffer (10 mM Tris-HCL pH 7.4, 10 mM MgCl₂, 0.01% Tween-20 and 1.0 mM DTT). The reactions were initiated by the combination of bacterially expressed, GST-Xa-hAAK1 with substrates and test compounds. The reactions were incubated at room temperature for 3 hours and terminated by adding 60 µl of 35 mM EDTA buffer to each sample. The reactions were analyzed on the Caliper LabChip 3000 (Caliper, Hopkinton, MA) by electrophoretic separation of the fluorescent substrate and phosphorylated product. Inhibition data were calculated by comparison to EDTA quenched control reactions for 100% inhibition and vehicle-only reactions for 0% inhibition. The final concentration of reagents in the assays are ATP, 22 µM; (5-FAM)-Aha-KEEQSQITSQVTGQIGWR-NH2, 1.5 µM; GST-Xa-hAAK1, 3.5 nM; and DMSO, 1.6%. Dose response curves were generated to determine the concentration required inhibiting 50% of kinase activity (IC₅₀). Compounds were dissolved at 10 mM in dimethylsulfoxide (DMSO) and evaluated at eleven concentrations. IC₅₀ values were derived by non-linear regression analysis.

### 5.4.3. P81 Filter Plate Assay

Compounds were serially diluted into a Labcyte LDV plate (Labcyte, cat# LP-0200) using a Mutiprobe (PerkinElmer) and Biomek FX (Beckman Coulter) so that the highest compound concentration was at 96 µM. Compounds were then pinged (75 nL per well) into a Greiner 384-well reaction plate (Greiner, # 781076) using an ECHO 550 Liquid Handler (Labcyte). A total of 12µl reaction buffer (IMAP buffer containing Tween and DTT, from Molecular Devices) was then added to each well of columns 1 and 13 for the negative controls and 12µl of 2X AAK1 (0.2 nM full-length human protein, NCBI accession no. NP_055726.2) was added to the remaining wells. Enzyme was then pre-incubated with compound for 10 minutes at RT. Reactions were initiated upon Minitrak (PerkinElmer) addition of 12µl substrate mix containing 2X Mu2 (0.2 µM, full length human protein), 2x cold ATP (2 µM), and 1.3 µCi of hot ³³P-ATP. Reactions proceeded for one hour at RT. Meanwhile, Millipore 384-well P81 filter plates (Millipore, catalog # MZPHN0W10) were placed on a plate washer (Zoom ZW, from Titertek) and pre-wet with 50µl 1% phosphoric acid. Kinase reactions were then stopped upon addition of 24µl of 2% phosphoric acid to each well and the Minitrak was then used to transfer 40µl from each well into the pre-wet Millipore 384-well P81 filter plates. Reaction mixtures were incubated for 10 minutes at RT in the P81 plates, followed by washing five times with 100µl/well of 1% phosphoric acid using the Zoom filter washer. The bottom of each filter plate was sealed followed by addition of 20µl Microscint 40 to each well, sealing the top of the plates with Flashplate cover, and then waiting one hour until reading on the TopCount (PerkinElmer).

### 5.4.4. HEK281 Cell-Based Assay

HEK293F cells were cultured in media containing DMEM (Gibco, cat. #11965), 10% FBS (SAFC Biosciences, cat. #12103C), 1X GPS (glutamine, penicillin and streptomycin). On day one, cells were plated on a 10cm dish so that they are ∼80% confluent at time of transfection. Roughly 12 million cells were in a 10cm dish at time of transfection. On day two, each dish was transfected with 48 ug DNA and 144 ul Lipofectamine 2000 (Invitrogen, cat.# 11668-019). The DNA was comprised of a mixture (per 10cm dish) containing 3 ug AAK1/HA/pIRES (full length human, NCBI accession no. NP_055726.2), 45 µg Flag/AP2MI/pcDNA (full length human), and 1.5 ml OPTI-MEM. The Lipofectamine 2000 is made up of a mixture (per 10cm dish) containing 144 µl Lipofectamine 2000 and 1.5 ml OPTI-MEM. Each mixture was transferred to individual 15ml tubes and incubated at RT for 5 minutes, and then the two mixes were combined and incubated at RT for 20 minutes. Growth media was then aspirated from each 10cm plate and replaced with 10ml of DMEM+10% FBS (no GPS). Finally, 3 ml DNA/Lipofectamine mix was added to each 10cm dish and mix gently followed by incubate of plate overnight at 37°C and 5% CO₂.

On day three, compounds were diluted in 100% DMSO at 1000X final concentration, followed by 3-fold serial dilutions for a total of 5 concentrations tested. Four compounds were tested per 10cm dish. One ul of each compound dilution was then pipetted into a deep-well, 96-well plate, followed by addition of 500 µl DMEM + 0.5% FBS into each well for a 2X final concentration of each compound. Cells were resuspended in a 10cm dish by simple pipetting (HEK293 cells come off the plate that easy at this point) and then transferred to a 50 ml conical tube and pelleted by centrifugation at 1000rpm for 5 min. Cell pellets were then resuspended in 2.75 ml DMEM + 0.5% FBS per 10cm dish and 100 µl of cell suspension transferred into each well of 96-well TC plate. Finally, 100 µl of 2X compound diluted in DMEM + 0.5% FBS was then added into wells containing cell suspension for a 1X final concentration. Plates were then incubated at 37°C and 5% CO₂ for 3 hours followed by transferring of cell suspensions from each well into 12-tube PCR strips. The PCR strips were spun in a tip rack at 1000rpm for 5 minutes to pellet cells and media was then removed by pipetting without disturbing the cell pellet.

To prepare for Western Blot analysis, cell pellets were resuspend in 40ul 1X LDS-PAGE sample buffer (Invitrogen, cat.# NP0008) + 2X Halt phophatase and protease inhibitor cocktail (Thermo Scientific, cat.#1861284), followed by sonicating each with microtip sonicator set at 5 for 8-10 seconds. Five ul of 10X NuPage Sample Reducing Agent (with 50 mM DTT) was to each sample followed by heat denaturing at 70C for 10 min on PCR machine. A total of 10µl per sample was loaded into each lane of a 4-20% Tris-Glycine Criterion 26-well gel (Biorad, cat.# 345-0034) for the phospho-mu2 blot and 10µl per lane in a 4-12% Bis-Tris (+MES buffer) NuPAGE 26-well gel (Invitrogen, cat.# WG1403BX10) for the mu2 blot. For controls, 2ng of phospho-mu2 or 20ng mu2/Flag proteins were loaded in the last well of each gel. After SDS-PAGE, samples on each gel were transferred to PVDF membrane using an iBlot and membranes were blocked for one hour in TBST + 5% milk, followed by wash 3X for 5-10 min with TBST. Criterion gels were probed with rabbit anti-phospho-mu2 (1:5000; a rabbit polyclonal antibody produced by New England Peptide and affinity purified at Lexicon) in TBST + 5% BSA, whereas, NuPAGE gels were probed with mouse anti-Flag (1:500; Sigma, cat.# F1804) in TBST + 5% milk, and these primary antibodies were incubated overnight at 4°C on a rocker.

On day four, Western blots were washed 3X for 5-10 minutes with TBST, probe with anti-rabbit-HRP (1:2000; BioRad, cat.# 170-6515) or anti-mouse-HRP (1:2000; Biorad, cat.# 170-6516) in TBST + 5% milk for 1 hour at RT, washed 3X for 10 minutes with TBST, and developed with ECL reagent (GE Healthcare, cat.# RPN2132) on a Versadoc. Finally, the camera was set up to take a picture every 30 seconds for 10 minutes and the best image saved for each blot with no saturated signal (when the signal is saturated, the bands will be highlighted red). A volume analysis on each band was performed to obtain density values. Percent inhibition was calculated for each sample by first normalizing to total Mu2 expression levels and then comparing to 0% and 100% controls. IC₅₀ values were then calculated using Excel fitting software.

### 5.4.5. Imidazo[1,2-b]pyridazine-based Inhibitor In Vitro Data

*In vitro* data obtained for various compounds of the invention are provided below in Table 1, wherein "MW" means molecular weight, "P81 Assay" refers to the P81 filter plate assay described above, "CBA" refers to the HEK281 cell-based assay described above, "--" means that results for the given assay were not obtained, "*" means less than or equal to 1.0 µM, "**" means a value of less than or equal to 0.1 µM, and "***" means less than or equal to 0.01 µM.

**Table 1**

| **Compound** | **MW** | **CBA IC₅₀** | **P81 IC₅₀** |
|---|---|---|---|
| (2S)-ethyl 2-(((3-(4-(pyrrolidin-2-yl)phenyl)imidazo[1,2-b]pyridazin-6-yl)amino)methyl)pyrrolidine-1-carboxylate | 434.5 | ** | -- |
| (2S)-methyl 2-(((3-(4-(pyrrolidin-2-yl)phenyl)imidazo[1,2-b]pyridazin-6-yl)amino)methyl)pyrrolidine-1-carboxylate | 420.5 | ** | -- |
| (2S)-tetrahydrofuran-3-yl 2-(((3-(pyridin-4-yl)imidazo[1,2-b]pyridazin-6-yl)amino)methyl)pyrrolidine-1-carboxylate | 408.5 | -- | *** |
| (2S)-tetrahydrofuran-3-yl 2-(((3-bromoimidazo[1,2-b]pyridazin-6-yl)amino)methyl)pyrrolidine-1-carboxylate | 410.3 | -- | *** |
| (4-(3-(2-methoxyphenyl)imidazo[1,2-b]pyridazin-6-yl)piperazin-1-yl)(pyrrolidin-1-yl)methanone | 406.5 | ** | *** |
| (4-(3-(2-methoxyphenyl)imidazo[1,2-b]pyridazin-6-yl)piperazin-1-yl)(piperidin-1-yl)methanone | 420.5 | ** | *** |
| (4-(3-(2-methoxypyridin-3-yl)imidazo[1,2-b]pyridazin-6-yl)piperazin-1-yl)(pyrrolidin-1-yl)methanone | 407.5 | ** | *** |
| (4-(3-(2-methoxypyridin-3-yl)imidazo[1,2-b]pyridazin-6-yl)piperazin-1-yl)(piperidin-1-yl)methanone | 421.5 | ** | *** |
| (4-(3-(3-methoxypyridin-4-yl)imidazo[1,2-b]pyridazin-6-yl)piperazin-1-yl)(pyrrolidin-1-yl)methanone | 407.5 | ** | *** |
| (4-(3-(pyridin-2-yl)imidazo[1,2-b]pyridazin-6-yl)piperazin-1-yl)(pyrrolidin-1-yl)methanone | 377.4 | ** | *** |
| (4-(3-phenylimidazo[1,2-b]pyridazin-6-yl)piperazin-1-yl)(piperidin-1-yl)methanone | 390.5 | ** | *** |
| (4-(3-phenylimidazo[1,2-b]pyridazin-6-yl)piperazin-1-yl)(pyrrolidin-1-yl)methanone | 376.5 | * | *** |
| (4-(6-((2-(cyclopentyloxy)ethyl)amino)imidazo[1,2-b]pyridazin-3-yl)phenyl)methanol | 352.4 | -- | *** |
| (4-(6-(butylthio)imidazo[1,2-b]pyridazin-3-yl)phenyl)methanamine | 312.4 | * | -- |
| (4-aminopiperidin-1-yl)(4-(6-(butylamino)imidazo[1,2-b]pyridazin-3-yl)phenyl)methanone | 392.5 | * | -- |
| (R)-isopropyl methyl(2-((3-(4-(pyrrolidin-2-yl)phenyl)imidazo[1,2-b]pyridazin-6-yl)amino)ethyl)carbamate | 422.5 | ** | -- |
| (R)-N-butyl-3-(4-(pyrrolidin-2-yl)phenyl)imidazo[1,2-b]pyridazin-6-amine | 335.4 | * | -- |
| (S)-1-(2-(((3-(2,4-dimethylthiazol-5-yl)imidazo[1,2-b]pyridazin-6-yl)amino)methyl)pyrrolidin-1-yl)-3-methylbutan-1-one | 412.6 | *** | *** |
| (S)-1-(2-(((3-(2-chlorophenyl)imidazo[1,2-b]pyridazin-6-yl)amino)methyl)pyrrolidin-1-yl)-3-methylbutan-1-one | 411.9 | -- | -- |
| (S)-1-(2-(((3-(2-fluoropyridin-4-yl)imidazo[1,2-b]pyridazin-6-yl)amino)methyl)pyrrolidin-1-yl)-3-methylbutan-1-one | 396.5 | -- | *** |
| (S)-1-(2-(((3-(2-methoxyphenyl)imidazo[1,2-b]pyridazin-6-yl)amino)methyl)pyrrolidin-1-yl)-3-methylbutan-1-one | 407.5 | *** | -- |
| (S)-1-(2-(((3-(2-methoxyphenyl)imidazo[1,2-b]pyridazin-6-yl)amino)methyl)pyrrolidin-1-yl)ethanone | 365.4 | ** | -- |
| (S)-1-(2-(((3-(3-chlorophenyl)imidazo[1,2-b]pyridazin-6-yl)amino)methyl)pyrrolidin-1-yl)-3-methylbutan-1-one | 411.9 | *** | *** |
| (S)-1-(2-(((3-(3-fluorophenyl)imidazo[1,2-b]pyridazin-6-yl)amino)methyl)pyrrolidin-1-yl)-3-methylbutan-1-one | 395.5 | *** | *** |
| (S)-1-(2-(((3-(3-methoxyphenyl)imidazo[1,2-b]pyridazin-6-yl)amino)methyl)pyrrolidin-1-yl)-3-methylbutan-1-one | 407.5 | ** | *** |
| (S)-1-(2-(((3-(3-methoxypyridin-4-yl)imidazo[1,2-b]pyridazin-6-yl)amino)methyl)pyrrolidin-1-yl)ethanone | 366.4 | ** | -- |
| (S)-1-(2-(((3-(3-methoxypyridin-4-yl)imidazo[1,2-b]pyridazin-6-yl)amino)methyl)pyrrolidin-1-yl)-3-methylbutan-1-one | 408.5 | *** | -- |
| (S)-1-(2-(((3-(4-(aminomethyl)phenyl)imidazo[1,2-b]pyridazin-6-yl)amino)methyl)pyrrolidin-1-yl)-3-methylbutan-1-one | 406.5 | *** | -- |
| (S)-1-(2-(((3-(4-(aminomethyl)phenyl)imidazo[1,2-b]pyridazin-6-yl)amino)methyl)pyrrolidin-1-yl)-2,2-dimethylpropan-1-one | 406.5 | ** | *** |
| (S)-1-(2-(((3-(4-(aminomethyl)phenyl)imidazo[1,2-b]pyridazin-6-yl)amino)methyl)pyrrolidin-1-yl)butan-1-one | 392.5 | *** | *** |
| (S)-1-(2-(((3-(4-(aminomethyl)phenyl)imidazo[1,2-b]pyridazin-6-yl)amino)methyl)pyrrolidin-1-yl)-3,3-dimethylbutan-1-one | 420.6 | ** | *** |
| (S)-1-(2-(((3-(4-fluorophenyl)imidazo[1,2-b]pyridazin-6-yl)amino)methyl)pyrrolidin-1-yl)-3-methylbutan-1-one | 395.5 | ** | *** |
| (S)-1-(2-(((3-(4-methoxyphenyl)imidazo[1,2-b]pyridazin-6-yl)amino)methyl)pyrrolidin-1-yl)-3-methylbutan-1-one | 407.5 | * | *** |
| (S)-1-(2-(((3-(cyclopent-1-en-1-yl)imidazo[1,2-b]pyridazin-6-yl)amino)methyl)pyrrolidin-1-yl)-3-methylbutan-1-one | 367.5 | -- | *** |
| (S)-1-(2-(((3-(pyridin-4-yl)imidazo[1,2-b]pyridazin-6-yl)amino)methyl)pyrrolidin-1-yl)butan-1-one | 364.4 | -- | *** |
| (S)-1-(2-(((3-bromoimidazo[1,2-b]pyridazin-6-yl)amino)methyl)pyrrolidin-1-yl)-3-methylbutan-1-one | 380.3 | ** | -- |
| (S)-1-(3,3-dimethylbutyl)-5-(((3-(2-methoxyphenyl)imidazo[1,2-b]pyridazin-6-yl)amino)methyl)pyrrolidin-2-one | 421.5 | * | *** |
| (S)-1-(3,3-dimethylbutyl)-5-(((3-(2-methoxypyridin-3-yl)imidazo[1,2-b]pyridazin-6-yl)amino)methyl)pyrrolidin-2-one | 422.5 | *** | *** |
| (S)-1-(3,3-dimethylbutyl)-5-(((3-(pyridin-4-yl)imidazo[1,2-b]pyridazin-6-yl)amino)methyl)pyrrolidin-2-one | 392.5 | -- | *** |
| (S)-1-(3,3-dimethylbutyl)-5-(((3-phenylimidazo[1,2-b]pyridazin-6-yl)amino)methyl)pyrrolidin-2-one | 391.5 | * | *** |
| (S)-1-methylcyclopentyl 2-(((3-bromoimidazo[1,2-b]pyridazin-6-yl)amino)methyl)pyrrolidine-1-carboxylate | 422.3 | * | *** |
| (S)-2-(((3-bromoimidazo[1,2-b]pyridazin-6-yl)amino)methyl)-N-(tert-butyl)pyrrolidine-1-carboxamide | 395.3 | ** | *** |
| (S)-2,2,2-trifluoroethyl (1-(3-(2-methoxypyridin-3-yl)imidazo[1,2-b]pyridazin-6-yl)pyrrolidin-3-yl)(methyl)carbamate | 450.4 | ** | *** |
| (S)-2-amino-N-(4-(6-(butylamino)imidazo[1,2-b]pyridazin-3-yl)benzyl)-4-methylpentanamide | 408.5 | ** | -- |
| (S)-2-cyclopropyl-N-(1-(3-(2-hydroxypyridin-3-yl)imidazo[1,2-b]pyridazin-6-yl)pyrrolidin-3-yl)-N-methylacetamide | 392.5 | -- | *** |
| (S)-2-methoxyethyl (1-(3-(2-methoxypyridin-3-yl)imidazo[1,2-b]pyridazin-6-yl)pyrrolidin-3-yl)(methyl)carbamate | 426.5 | -- | ** |
| (S)-3-(4-(aminomethyl)phenyl)-N-((tetrahydrofuran-2-yl)methyl)imidazo[1,2-b]pyridazin-6-amine | 323.4 | ** | -- |
| (S)-3-(6-(((1-(3-methylbutanoyl)pyrrolidin-2-yl)methyl)amino)imidazo[1,2-b]pyridazin-3-yl)benzonitrile | 402.5 | ** | *** |
| (S)-3,3,3-trifluoro-1-(2-(((3-phenylimidazo[1,2-b]pyridazin-6-yl)amino)methyl)pyrrolidin-1-yl)propan-1-one | 403.4 | *** | *** |
| (S)-3,3,3-trifluoro-N-(1-(3-(2-hydroxypyridin-3-yl)imidazo[1,2-b]pyridazin-6-yl)pyrrolidin-3-yl)-N-methylpropanamide | 420.4 | -- | *** |
| (S)-3,3-dimethyl-1-(2-(((3-(pyridin-4-yl)imidazo[1,2-b]pyridazin-6-yl)amino)methyl)pyrrolidin-1-yl)butan-1-one | 392.5 | ** | *** |
| (S)-3-methyl-1-(2-(((3-(2-methylpyridin-4-yl)imidazo[1,2-b]pyridazin-6-yl)amino)methyl)pyrrolidin-1-yl)butan-1-one | 392.5 | ** | *** |
| (S)-3-methyl-1-(2-(((3-(m-tolyl)imidazo[1,2-b]pyridazin-6-yl)amino)methyl)pyrrolidin-1-yl)butan-1-one | 391.5 | * | *** |
| (S)-3-methyl-1-(2-(((3-(pyridin-4-yl)imidazo[1,2-b]pyridazin-6-yl)amino)methyl)pyrrolidin-1-yl)butan-1-one | 378.5 | ** | -- |
| (S)-3-methyl-1-(2-(((3-phenylimidazo[1,2-b]pyridazin-6-yl)amino)methyl)pyrrolidin-1-yl)butan-1-one | 377.5 | ** | *** |
| (S)-4-(6-(((1-(3-methylbutanoyl)pyrrolidin-2-yl)methyl)amino)imidazo[1,2-b]pyridazin-3-yl)benzonitrile | 402.5 | *** | *** |
| (S)-5-(((3-(4-(aminomethyl)phenyl)imidazo[1,2-b]pyridazin-6-yl)amino)methyl)-1-isopentylpyrrolidin-2-one | 406.5 | ** | *** |
| (S)-5-(((3-(4-(aminomethyl)phenyl)imidazo[1,2-b]pyridazin-6-yl)amino)methyl)-1-(3,3-dimethylbutyl)pyrrolidin-2-one | 420.6 | ** | *** |
| (S)-5-(((3-bromoimidazo[1,2-b]pyridazin-6-yl)amino)methyl)-1-(3,3-dimethylbutyl)pyrrolidin-2-one | 394.3 | ** | *** |
| (S)-cyclobutyl 2-(((3-(2-methoxyphenyl)imidazo[1,2-b]pyridazin-6-yl)amino)methyl)pyrrolidine-1-carboxylate | 421.5 | ** | *** |
| (S)-cyclobutyl 2-(((3-(pyridin-4-yl)imidazo[1,2-b]pyridazin-6-yl)amino)methyl)pyrrolidine-1-carboxylate | 392.5 | *** | *** |
| (S)-cyclobutyl 2-(((3-bromoimidazo[1,2-b]pyridazin-6-yl)amino)methyl)pyrrolidine-1-carboxylate | 394.3 | ** | *** |
| (S)-cyclopentyl 2-(((3-(4-(aminomethyl)phenyl)imidazo[1,2-b]pyridazin-6-yl)amino)methyl)pyrrolidine-1-carboxylate | 434.5 | *** | *** |
| (S)-cyclopentyl 2-(((3-(pyridin-4-yl)imidazo[1,2-b]pyridazin-6-yl)amino)methyl)pyrrolidine-1-carboxylate | 406.5 | *** | *** |
| (S)-cyclopentyl 2-(((3-bromoimidazo[1,2-b]pyridazin-6-yl)amino)methyl)pyrrolidine-1-carboxylate | 408.3 | *** | *** |
| (S)-cyclopentyl 2-(((3-cyanoimidazo[1,2-b]pyridazin-6-yl)amino)methyl)pyrrolidine-1-carboxylate | 354.4 | -- | *** |
| (S)-cyclopentyl 2-(((3-phenylimidazo[1,2-b]pyridazin-6-yl)amino)methyl)pyrrolidine-1-carboxylate | 405.5 | ** | *** |
| (S)-cyclopropylmethyl 2-(((3-(pyridin-4-yl)imidazo[1,2-b]pyridazin-6-yl)amino)methyl)pyrrolidine-1-carboxylate | 392.5 | -- | *** |
| (S)-cyclopropylmethyl 2-(((3-bromoimidazo[1,2-b]pyridazin-6-yl)amino)methyl)pyrrolidine-1-carboxylate | 394.3 | -- | *** |
| (S)-ethyl (1-(3-(2-methoxypyridin-3-yl)imidazo[1,2-b]pyridazin-6-yl)pyrrolidin-3-yl)(methyl)carbamate | 396.4 | ** | *** |
| (S)-ethyl 2-(((3-(2-methoxyphenyl)imidazo[1,2-b]pyridazin-6-yl)amino)methyl)pyrrolidine-1-carboxylate | 395.5 | ** | -- |
| (S)-ethyl 2-(((3-(3-methoxypyridin-4-yl)imidazo[1,2-b]pyridazin-6-yl)amino)methyl)pyrrolidine-1-carboxylate | 396.4 | ** | -- |
| (S)-ethyl 2-(((3-(4-(aminomethyl)phenyl)imidazo[1,2-b]pyridazin-6-yl)amino)methyl)pyrrolidine-1-carboxylate | 394.5 | *** | -- |
| (S)-ethyl 2-(((3-(pyridin-4-yl)imidazo[1,2-b]pyridazin-6-yl)amino)methyl)pyrrolidine-1-carboxylate | 366.4 | ** | -- |
| (S)-ethyl 2-(((3-bromoimidazo[1,2-b]pyridazin-6-yl)amino)methyl)pyrrolidine-1-carboxylate | 368.2 | ** | -- |
| (S)-isopropyl (1-(3-(2-(difluoromethoxy)pyridin-3-yl)imidazo[1,2-b]pyridazin-6-yl)pyrrolidin-3-yl)(methyl)carbamate | 446.5 | -- | *** |
| (S)-isopropyl (1-(3-(2-methoxy-6-methylpyridin-3-yl)imidazo[1,2-b]pyridazin-6-yl)pyrrolidin-3-yl)(methyl)carbamate | 424.5 | *** | *** |
| (S)-isopropyl (1-(3-(2-methoxypyridin-3-yl)imidazo[1,2-b]pyridazin-6-yl)pyrrolidin-3-yl)(methyl)carbamate | 410.5 | *** | *** |
| (S)-isopropyl (1-(3-(3,6-dimethoxypyridazin-4-yl)imidazo[1,2-b]pyridazin-6-yl)pyrrolidin-3-yl)(methyl)carbamate | 441.5 | ** | *** |
| (S)-isopropyl (1-(3-bromoimidazo[1,2-b]pyridazin-6-yl)pyrrolidin-3-yl)(methyl)carbamate | 382.3 | -- | *** |
| (S)-isopropyl 2-(((3-(2-methoxyphenyl)imidazo[1,2-b]pyridazin-6-yl)amino)methyl)pyrrolidine-1-carboxylate | 409.5 | *** | -- |
| (S)-isopropyl 2-(((3-(3-methoxypyridin-4-yl)imidazo[1,2-b]pyridazin-6-yl)amino)methyl)pyrrolidine-1-carboxylate | 410.5 | *** | -- |
| (S)-isopropyl 2-(((3-(4-(aminomethyl)phenyl)imidazo[1,2-b]pyridazin-6-yl)amino)methyl)pyrrolidine-1-carboxylate | 408.5 | *** | -- |
| (S)-isopropyl 2-(((3-(prop-1-en-2-yl)imidazo[1,2-b]pyridazin-6-yl)amino)methyl)pyrrolidine-1-carboxylate | 343.4 | ** | -- |
| (S)-isopropyl 2-(((3-bromoimidazo[1,2-b]pyridazin-6-yl)amino)methyl)pyrrolidine-1-carboxylate | 382.3 | * | -- |
| (S)-isopropyl 2-(((3-chloroimidazo[1,2-b]pyridazin-6-yl)amino)methyl)pyrrolidine-1-carboxylate | 337.8 | ** | -- |
| (S)-isopropyl 2-(((3-cyanoimidazo[1,2-b]pyridazin-6-yl)amino)methyl)pyrrolidine-1-carboxylate | 328.4 | * | *** |
| (S)-isopropyl 2-(((3-cyclopropylimidazo[1,2-b]pyridazin-6-yl)amino)methyl)pyrrolidine-1-carboxylate | 343.4 | * | -- |
| (S)-isopropyl 2-(((3-vinylimidazo[1,2-b]pyridazin-6-yl)amino)methyl)pyrrolidine-1-carboxylate | 329.4 | *** | -- |
| (S)-isopropyl methyl(1-(3-(pyridin-3-yl)imidazo[1,2-b]pyridazin-6-yl)pyrrolidin-3-yl)carbamate | 380.4 | ** | *** |
| (S)-isopropyl methyl(1-(3-(pyridin-4-yl)imidazo[1,2-b]pyridazin-6-yl)pyrrolidin-3-yl)carbamate | 380.4 | - | ** |
| (S)-isopropyl methyl(2-((3-(4-(pyrrolidin-2-yl)phenyl)imidazo[1,2-b]pyridazin-6-yl)amino)ethyl)carbamate | 422.5 | ** | -- |
| (S)-methyl 2-(((3-(2-methoxyphenyl)imidazo[1,2-b]pyridazin-6-yl)amino)methyl)pyrrolidine-1-carboxylate | 381.4 | ** | -- |
| (S)-methyl 2-(((3-(3-methoxypyridin-4-yl)imidazo[1,2-b]pyridazin-6-yl)amino)methyl)pyrrolidine-1-carboxylate | 382.4 | ** | -- |
| (S)-methyl 2-(((3-(4-(aminomethyl)phenyl)imidazo[1,2-b]pyridazin-6-yl)amino)methyl)pyrrolidine-1-carboxylate | 380.4 | ** | -- |
| (S)-methyl 2-(((3-bromoimidazo[1,2-b]pyridazin-6-yl)amino)methyl)pyrrolidine-1-carboxylate | 354.2 | * | -- |
| (S)-N-(1-(3-(2-hydroxypyridin-3-yl)imidazo[1,2-b]pyridazin-6-yl)pyrrolidin-3-yl)-N-methylcyclopentanecarboxamide | 406.5 | -- | ** |
| (S)-N-(1-(3-(2-hydroxypyridin-3-yl)imidazo[1,2-b]pyridazin-6-yl)pyrrolidin-3-yl)-2-methoxy-N-methylacetamide | 382.4 | -- | * |
| (S)-N-(1-(3-(2-hydroxypyridin-3-yl)imidazo[1,2-b]pyridazin-6-yl)pyrrolidin-3-yl)-N,3-dimethylbutanamide | 394.5 | -- | ** |
| (S)-N-(1-(3-(2-hydroxypyridin-3-yl)imidazo[1,2-b]pyridazin-6-yl)pyrrolidin-3-yl)-N,3,3-trimethylbutanamide | 408.5 | * | ** |
| (S)-N-(1-(3-(2-hydroxypyridin-3-yl)imidazo[1,2-b]pyridazin-6-yl)pyrrolidin-3-yl)-N-methylbutyramide | 380.4 | -- | ** |
| (S)-N-(1-(3-(2-methoxyphenyl)imidazo[1,2-b]pyridazin-6-yl)pyrrolidin-3-yl)-N-methylpyrrolidine-1-carboxamide | 420.5 | ** | *** |
| (S)-N-(1-(3-(2-methoxypyridin-3-yl)imidazo[1,2-b]pyridazin-6-yl)pyrrolidin-3-yl)-N-methylpyrrolidine-1-carboxamide | 421.5 | ** | *** |
| (S)-N-(1-(3-(2-methoxypyridin-3-yl)imidazo[1,2-b]pyridazin-6-yl)pyrrolidin-3-yl)-N-methylbutyramide | 394.5 | -- | ** |
| (S)-N-(1-(3-bromoimidazo[1,2-b]pyridazin-6-yl)pyrrolidin-3-yl)-N-methylbutyramide | 366.3 | -- | * |
| (S)-N-(1-(3-bromoimidazo[1,2-b]pyridazin-6-yl)pyrrolidin-3-yl)-N-methylpyrrolidine-1-carboxamide | 393.3 | -- | ** |
| (S)-N-(tert-butyl)-2-(((3-(2-methoxyphenyl)imidazo[1,2-b]pyridazin-6-yl)amino)methyl)pyrrolidine-1-carboxamide | 422.5 | ** | *** |
| (S)-N-(tert-butyl)-2-(((3-(pyridin-4-yl)imidazo[1,2-b]pyridazin-6-yl)amino)methyl)pyrrolidine-1-carboxamide | 393.5 | ** | *** |
| (S)-N-(tert-butyl)-2-(((3-phenylimidazo[1,2-b]pyridazin-6-yl)amino)methyl)pyrrolidine-1-carboxamide | 392.5 | ** | *** |
| (S)-N-butyl-3-(4-(pyrrolidin-2-yl)phenyl)imidazo[1,2-b]pyridazin-6-amine | 335.4 | ** | -- |
| (S)-N-cyclopentyl-2-(((3-(2-methoxyphenyl)imidazo[1,2-b]pyridazin-6-yl)amino)methyl)pyrrolidine-1-carboxamide | 434.5 | * | *** |
| (S)-neopentyl 2-(((3-(4-(aminomethyl)phenyl)imidazo[1,2-b]pyridazin-6-yl)amino)methyl)pyrrolidine-1-carboxylate | 436.5 | *** | *** |
| (S)-neopentyl 2-(((3-(pyridin-4-yl)imidazo[1,2-b]pyridazin-6-yl)amino)methyl)pyrrolidine-1-carboxylate | 408.5 | ** | *** |
| (S)-neopentyl 2-(((3-bromoimidazo[1,2-b]pyridazin-6-yl)amino)methyl)pyrrolidine-1-carboxylate | 410.3 | ** | -- |
| (S)-N-methyl-N-(1-(3-(pyridin-2-yl)imidazo[1,2-b]pyridazin-6-yl)pyrrolidin-3-yl)pyrrolidine-1-carboxamide | 391.5 | -- | ** |
| (S)-N-methyl-N-(1-(3-(pyridin-3-yl)imidazo[1,2-b]pyridazin-6-yl)pyrrolidin-3-yl)butyramide | 364.4 | -- | * |
| (S)-N-methyl-N-(1-(3-(pyridin-4-yl)imidazo[1,2-b]pyridazin-6-yl)pyrrolidin-3-yl)pyrrolidine-1-carboxamide | 391.5 | -- | ** |
| (S)-N-methyl-N-(1-(3-(pyridin-4-yl)imidazo[1,2-b]pyridazin-6-yl)pyrrolidin-3-yl)butyramide | 364.4 | -- | ** |
| (S)-N-neopentyl-2-(((3-phenylimidazo[1,2-b]pyridazin-6-yl)amino)methyl)pyrrolidine-1-carboxamide | 406.5 | ** | *** |
| (S)-propyl 2-(((3-bromoimidazo[1,2-b]pyridazin-6-yl)amino)methyl)pyrrolidine-1-carboxylate | 382.3 | -- | *** |
| (S)-tert-butyl (1-(3-(2-methoxyphenyl)imidazo[1,2-b]pyridazin-6-yl)pyrrolidin-3-yl)(methyl)carbamate | 423.5 | ** | *** |
| (S)-tert-butyl (1-(3-(2-methoxypyridin-3-yl)imidazo[1,2-b]pyridazin-6-yl)pyrrolidin-3-yl)(methyl)carbamate | 424.5 | ** | *** |
| (S)-tert-butyl (1-(3-bromoimidazo[1,2-b]pyridazin-6-yl)pyrrolidin-3-yl)(methyl)carbamate | 396.3 | * | *** |
| (S)-tert-butyl 2-(((3-(2-methoxyphenyl)imidazo[1,2-b]pyridazin-6-yl)amino)methyl)pyrrolidine-1-carboxylate | 423.5 | *** | -- |
| (S)-tert-butyl 2-(((3-(3-methoxypyridin-4-yl)imidazo[1,2-b]pyridazin-6-yl)amino)methyl)piperidine-1-carboxylate | 438.5 | ** | -- |
| (S)-tert-butyl 2-(((3-(4-(aminomethyl)phenyl)imidazo[1,2-b]pyridazin-6-yl)amino)methyl)pyrrolidine-1-carboxylate | 422.5 | *** | *** |
| (S)-tert-butyl 2-(((3-(4-(aminomethyl)phenyl)imidazo[1,2-b]pyridazin-6-yl)amino)methyl)piperidine-1-carboxylate | 436.5 | *** | -- |
| (S)-tert-butyl 2-(((3-(4-carbamoylphenyl)imidazo[1,2-b]pyridazin-6-yl)amino)methyl)pyrrolidine-1-carboxylate | 436.5 | *** | *** |
| (S)-tert-butyl 2-(((3-(difluoromethyl)imidazo[1,2-b]pyridazin-6-yl)amino)methyl)pyrrolidine-1-carboxylate | 367.4 | * | *** |
| (S)-tert-butyl 2-(((3-(methylcarbamoyl)imidazo[1,2-b]pyridazin-6-yl)amino)methyl)pyrrolidine-1-carboxylate | 374.4 | ** | *** |
| (S)-tert-butyl 2-(((3-(pyridin-4-yl)imidazo[1,2-b]pyridazin-6-yl)amino)methyl)piperidine-1-carboxylate | 408.5 | ** | -- |
| (S)-tert-butyl 2-(((3-(trifluoromethyl)imidazo[1,2-b]pyridazin-6-yl)amino)methyl)pyrrolidine-1-carboxylate | 385.4 | ** | -- |
| (S)-tert-butyl 2-(((3-bromoimidazo[1,2-b]pyridazin-6-yl)amino)methyl)pyrrolidine-1-carboxylate | 396.3 | ** | -- |
| (S)-tert-butyl 2-(((3-bromoimidazo[1,2-b]pyridazin-6-yl)amino)methyl)azetidine-1-carboxylate | 382.3 | ** | *** |
| (S)-tert-butyl 2-(((3-chloroimidazo[1,2-b]pyridazin-6-yl)amino)methyl)pyrrolidine-1-carboxylate | 351.8 | ** | -- |
| (S)-tert-butyl 2-(((3-ethylimidazo[1,2-b]pyridazin-6-yl)amino)methyl)pyrrolidine-1-carboxylate | 345.4 | ** | -- |
| (S)-tert-butyl 2-(((3-iodoimidazo[1,2-b]pyridazin-6-yl)amino)methyl)pyrrolidine-1-carboxylate | 443.3 | *** | -- |
| (S)-tert-butyl 2-(((3-methylimidazo[1,2-b]pyridazin-6-yl)amino)methyl)pyrrolidine-1-carboxylate | 331.4 | ** | *** |
| (S)-tert-butyl 2-(((3-phenylimidazo[1,2-b]pyridazin-6-yl)amino)methyl)pyrrolidine-1-carboxylate | 393.5 | *** | *** |
| (S)-tert-butyl 2-(2-((3-(4-(aminomethyl)phenyl)imidazo[1,2-b]pyridazin-6-yl)amino)ethyl)pyrrolidine-1-carboxylate | 436.5 | ** | -- |
| (S)-tert-butyl 3-(((3-(3-methoxypyridin-4-yl)imidazo[1,2-b]pyridazin-6-yl)amino)methyl)pyrrolidine-1-carboxylate | 424.5 | * | -- |
| (S)-tert-butyl 3-(((3-(4-(aminomethyl)phenyl)imidazo[1,2-b]pyridazin-6-yl)amino)methyl)pyrrolidine-1-carboxylate | 422.5 | ** | -- |
| (S)-tert-butyl 3-(((3-bromoimidazo[1,2-b]pyridazin-6-yl)amino)methyl)pyrrolidine-1-carboxylate | 396.3 | * | -- |
| (S)-tert-butyl methyl(1-(3-(pyridin-3-yl)imidazo[1,2-b]pyridazin-6-yl)pyrrolidin-3-yl)carbamate | 394.5 | -- | *** |
| (S)-tert-butyl methyl(1-(3-(pyridin-4-yl)imidazo[1,2-b]pyridazin-6-yl)pyrrolidin-3-yl)carbamate | 394.5 | * | *** |
| (S)-vinyl 2-(((3-(2-methoxyphenyl)imidazo[1,2-b]pyridazin-6-yl)amino)methyl)pyrrolidine-1-carboxylate | 393.4 | ** | *** |
| 1-(2-((3-(4-(aminomethyl)phenyl)imidazo[1,2-b]pyridazin-6-yl)amino)ethyl)-3-(tert-butyl)imidazolidin-2-one | 407.5 | ** | -- |
| 1-(3-(3-(3-methoxypyridin-4-yl)imidazo[1,2-b]pyridazin-6-yl)phenyl)-4-methylpentan-1-one | 400.5 | ** | -- |
| 1-(3-(3-(4-(aminomethyl)phenyl)imidazo[1,2-b]pyridazin-6-yl)phenyl)ethanone | 342.4 | ** | -- |
| 1-(3-(3-(4-(aminomethyl)phenyl)imidazo[1,2-b]pyridazin-6-yl)phenyl)-4-methylpentan-1-one | 398.5 | ** | -- |
| 1-(4-(3-(3-methoxypyridin-4-yl)imidazo[1,2-b]pyridazin-6-yl)piperazin-1-yl)-3,3-dimethylbutan-1-one | 408.5 | ** | -- |
| 1-(5-(6-((2-(methyl(phenyl)amino)ethyl)amino)imidazo[1,2-b]pyridazin-3-yl)thiophen-2-yl)ethanone | 391.5 | ** | -- |
| 1-(5-(6-((3-(methylamino)propyl)amino)imidazo[1,2-b]pyridazin-3-yl)thiophen-2-yl)ethanone | 329.4 | * | -- |
| 1-(5-(6-((3-methoxypropyl)amino)imidazo[1,2-b]pyridazin-3-yl)thiophen-2-yl)ethanone | 330.4 | * | -- |
| 1-(5-(6-(butyl(methyl)amino)imidazo[1,2-b]pyridazin-3-yl)thiophen-2-yl)ethanone | 328.4 | ** | -- |
| 1-(5-(6-(isobutylamino)imidazo[1,2-b]pyridazin-3-yl)thiophen-2-yl)ethanone | 314.4 | * | -- |
| 1-(5-(6-(propylamino)imidazo[1,2-b]pyridazin-3-yl)thiophen-2-yl)ethanone | 300.4 | * | -- |
| 1-(tert-butyl)-3-(2-((3-(3-methoxypyridin-4-yl)imidazo[1,2-b]pyridazin-6-yl)amino)ethyl)imidazolidin-2-one | 409.5 | ** | -- |
| 2,2-dimethyl-1-(4-(3-phenylimidazo[1,2-b]pyridazin-6-yl)piperazin-1-yl)propan-1-one | 363.5 | -- | ** |
| 2-fluoroethyl isopropyl(2-((3-(3-methoxypyridin-4-yl)imidazo[1,2-b]pyridazin-6-yl)amino)ethyl)carbamate | 416.4 | * | -- |
| 3-(2,4-dimethylthiazol-5-yl)-N-(2-isobutoxyethyl)imidazo[1,2-b]pyridazin-6-amine | 345.5 | * | -- |
| 3-(2-aminopyridin-4-yl)-N-(3-(tert-butoxy)propyl)imidazo[1,2-b]pyridazin-6-amine | 340.4 | * | -- |
| 3-(2-aminopyridin-4-yl)-N-butylimidazo[1,2-b]pyridazin-6-amine | 282.3 | * | -- |
| 3-(2-methoxypyridin-3-yl)-N-((tetrahydrofuran-3-yl)methyl)imidazo[1,2-b]pyridazin-6-amine | 325.4 | * | -- |
| 3-(3-(4-(aminomethyl)phenyl)imidazo[1,2-b]pyridazin-6-yl)-N-isobutylbenzamide | 399.5 | ** | -- |
| 3-(3-methoxypyridin-4-yl)-N-(4,4,4-trifluorobutyl)imidazo[1,2-b]pyridazin-6-amine | 351.3 | *** | -- |
| 3-(4-(((2-methoxyethyl)amino)methyl)phenyl)-N-pentylimidazo[1,2-b]pyridazin-6-amine | 367.5 | * | -- |
| 3-(4-((2-(dimethylamino)ethoxy)methyl)phenyl)-N-(3,3-dimethylbutyl)imidazo[1,2-b]pyridazin-6-amine | 395.5 | ** | -- |
| 3-(4-((2-aminoethoxy)methyl)phenyl)-N-(3,3-dimethylbutyl)imidazo[1,2-b]pyridazin-6-amine | 367.5 | ** | -- |
| 3-(4-(1H-tetrazol-5-yl)phenyl)-N-butylimidazo[1,2-b]pyridazin-6-amine | 334.4 | * | -- |
| 3-(4-(2-aminoethoxy)phenyl)-N-butylimidazo[1,2-b]pyridazin-6-amine | 325.4 | ** | -- |
| 3-(4-(3-aminopropoxy)phenyl)-N-(3,3-dimethylbutyl)imidazo[1,2-b]pyridazin-6-amine | 367.5 | ** | -- |
| 3-(4-(3-aminopropoxy)phenyl)-N-(3-phenylpropyl)imidazo[1,2-b]pyridazin-6-amine | 401.5 | ** | -- |
| 3-(4-(aminomethyl)-3-fluorophenyl)-N-butylimidazo[1,2-b]pyridazin-6-amine | 313.4 | * | -- |
| 3-(4-(aminomethyl)phenyl)-N-((1-(2,2,2-trifluoroethyl)pyrrolidin-2-yl)methyl)imidazo[1,2-b]pyridazin-6-amine | 404.4 | ** | *** |
| 3-(4-(aminomethyl)phenyl)-N-((tetrahydrofuran-3-yl)methyl)imidazo[1,2-b]pyridazin-6-amine | 323.4 | * | -- |
| 3-(4-(aminomethyl)phenyl)-N-(1-oxaspiro[4.4]nonan-3-yl)imidazo[1,2-b]pyridazin-6-amine | 363.5 | ** | -- |
| 3-(4-(aminomethyl)phenyl)-N-(2-(cyclopentyloxy)ethyl)imidazo[1,2-b]pyridazin-6-amine | 351.4 | ** | -- |
| 3-(4-(aminomethyl)phenyl)-N-(2-(neopentyloxy)ethyl)imidazo[1,2-b]pyridazin-6-amine | 353.5 | ** | -- |
| 3-(4-(aminomethyl)phenyl)-N-(2-(tert-butoxy)ethyl)imidazo[1,2-b]pyridazin-6-amine | 339.4 | ** | -- |
| 3-(4-(aminomethyl)phenyl)-N-(2-(trifluoromethoxy)phenethyl)imidazo[1,2-b]pyridazin-6-amine | 427.4 | *** | -- |
| 3-(4-(aminomethyl)phenyl)-N-(2-cyclohexylethyl)imidazo[1,2-b]pyridazin-6-amine | 349.5 | ** | -- |
| 3-(4-(aminomethyl)phenyl)-N-(2-cyclopropylethyl)imidazo[1,2-b]pyridazin-6-amine | 307.4 | * | -- |
| 3-(4-(aminomethyl)phenyl)-N-(2-ethoxyphenethyl)imidazo[1,2-b]pyridazin-6-amine | 387.5 | ** | -- |
| 3-(4-(aminomethyl)phenyl)-N-(2-fluorophenethyl)imidazo[1,2-b]pyridazin-6-amine | 361.4 | ** | -- |
| 3-(4-(aminomethyl)phenyl)-N-(2-isobutoxyethyl)imidazo[1,2-b]pyridazin-6-amine | 339.4 | ** | -- |
| 3-(4-(aminomethyl)phenyl)-N-(2-isopropoxyethyl)imidazo[1,2-b]pyridazin-6-amine | 325.4 | ** | -- |
| 3-(4-(aminomethyl)phenyl)-N-(2-methoxyphenethyl)imidazo[1,2-b]pyridazin-6-amine | 373.5 | *** | -- |
| 3-(4-(aminomethyl)phenyl)-N-(2-methylbutyl)imidazo[1,2-b]pyridazin-6-amine | 309.4 | * | -- |
| 3-(4-(aminomethyl)phenyl)-N-(2-phenoxyethyl)imidazo[1,2-b]pyridazin-6-amine | 359.4 | * | -- |
| 3-(4-(aminomethyl)phenyl)-N-(3-(2-fluorophenyl)propyl)imidazo[1,2-b]pyridazin-6-amine | 375.4 | ** | -- |
| 3-(4-(aminomethyl)phenyl)-N-(3-(3-fluorophenyl)propyl)imidazo[1,2-b]pyridazin-6-amine | 375.4 | ** | -- |
| 3-(4-(aminomethyl)phenyl)-N-(3-(4-fluorophenyl)propyl)imidazo[1,2-b]pyridazin-6-amine | 375.4 | ** | -- |
| 3-(4-(aminomethyl)phenyl)-N-(3-(cyclopentyloxy)propyl)imidazo[1,2-b]pyridazin-6-amine | 365.5 | ** | -- |
| 3-(4-(aminomethyl)phenyl)-N-(3-(tert-butoxy)propyl)imidazo[1,2-b]pyridazin-6-amine | 353.5 | ** | -- |
| 3-(4-(aminomethyl)phenyl)-N-(3-(trifluoromethyl)phenethyl)imidazo[1,2-b]pyridazin-6-amine | 411.4 | ** | -- |
| 3-(4-(aminomethyl)phenyl)-N-(3,3-dimethylbutyl)imidazo[1,2-b]pyridazin-6-amine | 323.4 | ** | -- |
| 3-(4-(aminomethyl)phenyl)-N-(3-fluorophenethyl)imidazo[1,2-b]pyridazin-6-amine | 361.4 | ** | -- |
| 3-(4-(aminomethyl)phenyl)-N-(3-fluoropropyl)imidazo[1,2-b]pyridazin-6-amine | 299.3 | * | -- |
| 3-(4-(aminomethyl)phenyl)-N-(3-methoxyphenethyl)imidazo[1,2-b]pyridazin-6-amine | 373.5 | ** | -- |
| 3-(4-(aminomethyl)phenyl)-N-(3-phenylpropyl)imidazo[1,2-b]pyridazin-6-amine | 357.5 | ** | -- |
| 3-(4-(aminomethyl)phenyl)-N-(4-fluorobutyl)imidazo[1,2-b]pyridazin-6-amine | 313.4 | ** | -- |
| 3-(4-(aminomethyl)phenyl)-N-(4-fluorophenethyl)imidazo[1,2-b]pyridazin-6-amine | 361.4 | * | -- |
| 3-(4-(aminomethyl)phenyl)-N-(4-methoxyphenethyl)imidazo[1,2-b]pyridazin-6-amine | 373.5 | ** | -- |
| 3-(4-(aminomethyl)phenyl)-N-(4-phenylbutyl)imidazo[1,2-b]pyridazin-6-amine | 371.5 | ** | -- |
| 3-(4-(aminomethyl)phenyl)-N-(5-fluoropentyl)imidazo[1,2-b]pyridazin-6-amine | 327.4 | * | -- |
| 3-(4-(aminomethyl)phenyl)-N-(cyclobutylmethyl)imidazo[1,2-b]pyridazin-6-amine | 307.4 | * | -- |
| 3-(4-(aminomethyl)phenyl)-N-(cyclohexylmethyl)imidazo[1,2-b]pyridazin-6-amine | 335.4 | * | -- |
| 3-(4-(aminomethyl)phenyl)-N-(cyclopentylmethyl)imidazo[1,2-b]pyridazin-6-amine | 321.4 | * | -- |
| 3-(4-(aminomethyl)phenyl)-N-(cyclopropylmethyl)imidazo[1,2-b]pyridazin-6-amine | 293.4 | * | -- |
| 3-(4-(aminomethyl)phenyl)-N-butyl-7-methylimidazo[1,2-b]pyridazin-6-amine | 309.4 | ** | -- |
| 3-(4-(aminomethyl)phenyl)-N-butylimidazo[1,2-b]pyridazin-6-amine | 295.4 | * | -- |
| 3-(4-(aminomethyl)phenyl)-N-butyl-N-methylimidazo[1,2-b]pyridazin-6-amine | 309.4 | * | -- |
| 3-(4-(aminomethyl)phenyl)-N-cyclohexylimidazo[1,2-b]pyridazin-6-amine | 321.4 | * | -- |
| 3-(4-(aminomethyl)phenyl)-N-isobutylimidazo[1,2-b]pyridazin-6-amine | 295.4 | * | -- |
| 3-(4-(aminomethyl)phenyl)-N-isopentylimidazo[1,2-b]pyridazin-6-amine | 309.4 | * | -- |
| 3-(4-(aminomethyl)phenyl)-N-neopentylimidazo[1,2-b]pyridazin-6-amine | 309.4 | ** | -- |
| 3-(4-(aminomethyl)phenyl)-N-pentylimidazo[1,2-b]pyridazin-6-amine | 309.4 | * | -- |
| 3-(4-(aminomethyl)phenyl)-N-phenethylimidazo[1,2-b]pyridazin-6-amine | 343.4 | * | -- |
| 3-(4-(aminomethyl)phenyl)-N-propylimidazo[1,2-b]pyridazin-6-amine | 281.4 | * | -- |
| 3-(6-((2-(cyclopentyloxy)ethyl)amino)imidazo[1,2-b]pyridazin-3-yl)phenol | 338.4 | * | *** |
| 3-(benzo[d][1,3]dioxol-5-yl)-N-(2-(cyclopentyloxy)ethyl)imidazo[1,2-b]pyridazin-6-amine | 366.4 | * | ** |
| 3,3-dimethyl-1-(4-(3-phenylimidazo[1,2-b]pyridazin-6-yl)piperazin-1-yl)butan-1-one | 377.5 | ** | *** |
| 4-((3-(4-(aminomethyl)phenyl)imidazo[1,2-b]pyridazin-6-yl)amino)-2-methylbutan-2-ol | 325.4 | ** | -- |
| 4-(3-bromoimidazo[1,2-b]pyridazin-6-yl)-N-(tert-butyl)piperazine-1-carboxamide | 381.3 | ** | *** |
| 4-(6-((2-(N,3,3-trimethylbutanamido)ethyl)amino)imidazo[1,2-b]pyridazin-3-yl)benzamide | 408.5 | ** | -- |
| 4-(6-((2-(tert-butoxy)ethyl)amino)imidazo[1,2-b]pyridazin-3-yl)benzamide | 353.4 | ** | -- |
| 4-(6-((2-(tert-butoxy)ethyl)amino)imidazo[1,2-b]pyridazin-3-yl)-N-(2-(diethylamino)ethyl)benzamide | 452.6 | ** | -- |
| 4-(6-((2-(tert-butoxy)ethyl)amino)imidazo[1,2-b]pyridazin-3-yl)-N-methylbenzamide | 367.4 | ** | -- |
| 4-(6-((2-(trifluoromethoxy)phenethyl)amino)imidazo[1,2-b]pyridazin-3-yl)benzamide | 441.4 | *** | -- |
| 4-(6-((2-ethoxyphenethyl)amino)imidazo[1,2-b]pyridazin-3-yl)-N-methylbenzamide | 415.5 | *** | -- |
| 4-(6-((2-isobutoxyethyl)amino)imidazo[1,2-b]pyridazin-3-yl)-N-(2-(pyrrolidin-1-yl)ethyl)benzamide | 450.6 | ** | -- |
| 4-(6-((2-isopropoxyethyl)amino)imidazo[1,2-b]pyridazin-3-yl)-N-(2-(pyrrolidin-1-yl)ethyl)benzamide | 436.5 | ** | -- |
| 4-(6-((2-methoxyphenethyl)amino)imidazo[1,2-b]pyridazin-3-yl)benzamide | 387.4 | *** | -- |
| 4-(6-((3-(trifluoromethyl)phenethyl)amino)imidazo[1,2-b]pyridazin-3-yl)benzamide | 425.4 | ** | -- |
| 4-(6-((3-methoxyphenethyl)amino)imidazo[1,2-b]pyridazin-3-yl)benzamide | 387.4 | ** | -- |
| 4-(6-(butylamino)imidazo[1,2-b]pyridazin-3-yl)-2-fluoro-N-(2-(methylamino)ethyl)benzamide | 384.5 | ** | -- |
| 4-(6-(butylamino)imidazo[1,2-b]pyridazin-3-yl)benzamide | 309.4 | ** | -- |
| 4-(6-(butylamino)imidazo[1,2-b]pyridazin-3-yl)-N-(2-(diethylamino)ethyl)benzamide | 408.5 | *** | -- |
| 4-(6-(butylamino)imidazo[1,2-b]pyridazin-3-yl)-N-(2-(dimethylamino)ethyl)benzamide | 380.5 | ** | -- |
| 4-(6-(butylamino)imidazo[1,2-b]pyridazin-3-yl)-N-(2-(methylamino)ethyl)benzamide | 366.5 | ** | -- |
| 4-(6-(butylamino)imidazo[1,2-b]pyridazin-3-yl)-N-(2-(pyrrolidin-1-yl)ethyl)benzamide | 406.5 | ** | -- |
| 5-(3-(4-(aminomethyl)phenyl)imidazo[1,2-b]pyridazin-6-yl)-N-isobutylnicotinamide | 400.5 | ** | -- |
| 5-(6-((2-(cyclopentyloxy)ethyl)amino)imidazo[1,2-b]pyridazin-3-yl)thiophene-2-carbonitrile | 353.4 | ** | *** |
| 5-(6-(butylamino)imidazo[1,2-b]pyridazin-3-yl)thiophene-2-carboxamide | 315.4 | * | -- |
| 5-(6-(propylamino)imidazo[1,2-b]pyridazin-3-yl)thiophene-2-carbaldehyde | 286.4 | * | -- |
| 6-(butylamino)-N-(1H-pyrazol-4-yl)imidazo[1,2-b]pyridazine-3-carboxamide | 299.3 | * | -- |
| 6-(butylamino)-N-(4-((2-(dimethylamino)ethyl)carbamoyl)phenyl)imidazo[1,2-b]pyridazine-3-carboxamide | 423.5 | ** | -- |
| cyclopentyl (2-((3-(4-carbamoylphenyl)imidazo[1,2-b]pyridazin-6-yl)amino)ethyl)(isopropyl)carbamate | 450.5 | ** | -- |
| ethyl (2-((3-(3-methoxypyridin-4-yl)imidazo[1,2-b]pyridazin-6-yl)amino)ethyl)(methyl)carbamate | 370.4 | *** | -- |
| ethyl (2-((3-(4-(isopentylcarbamoyl)phenyl)imidazo[1,2-b]pyridazin-6-yl)amino)ethyl)(methyl)carbamate | 452.5 | ** | -- |
| ethyl (2-((3-(4-(tert-butylcarbamoyl)phenyl)imidazo[1,2-b]pyridazin-6-yl)amino)ethyl)(methyl)carbamate | 438.5 | ** | -- |
| ethyl (2-((3-(4-carbamoylphenyl)imidazo[1,2-b]pyridazin-6-yl)amino)ethyl)(methyl)carbamate | 382.4 | ** | -- |
| ethyl (2-((3-(4-carbamoylphenyl)imidazo[1,2-b]pyridazin-6-yl)amino)ethyl)(isopropyl)carbamate | 410.5 | ** | -- |
| ethyl (3-((3-(5-acetylthiophen-2-yl)imidazo[1,2-b]pyridazin-6-yl)amino)propyl)(methyl)carbamate | 401.5 | * | -- |
| ethyl 4-((3-(4-(aminomethyl)phenyl)imidazo[1,2-b]pyridazin-6-yl)amino)butanoate | 353.4 | ** | -- |
| ethyl 4-((3-(5-acetylthiophen-2-yl)imidazo[1,2-b]pyridazin-6-yl)amino)butanoate | 372.4 | ** | -- |
| ethyl 4-(3-(methylcarbamoyl)imidazo[1,2-b]pyridazin-6-yl)piperazine-1-carboxylate | 332.4 | -- | ** |
| ethyl 4-(3-phenylimidazo[1,2-b]pyridazin-6-yl)piperazine-1-carboxylate | 351.4 | ** | *** |
| ethyl isopropyl(2-((3-(3-methoxypyridin-4-yl)imidazo[1,2-b]pyridazin-6-yl)amino)ethyl)carbamate | 398.5 | *** | -- |
| ethyl methyl(2-((3-(4-(methylcarbamoyl)phenyl)imidazo[1,2-b]pyridazin-6-yl)amino)ethyl)carbamate | 396.4 | ** | -- |
| ethyl methyl(2-((3-(5-(methylcarbamoyl)thiophen-2-yl)imidazo[1,2-b]pyridazin-6-yl)amino)ethyl)carbamate | 402.5 | ** | -- |
| isobutyl (2-((3-(4-carbamoylphenyl)imidazo[1,2-b]pyridazin-6-yl)amino)ethyl)(methyl)carbamate | 410.5 | ** | -- |
| isobutyl isopropyl(2-((3-(3-methoxypyridin-4-yl)imidazo[1,2-b]pyridazin-6-yl)amino)ethyl)carbamate | 426.5 | ** | -- |
| isobutyl methyl(2-((3-(4-(methylcarbamoyl)phenyl)imidazo[1,2-b]pyridazin-6-yl)amino)ethyl)carbamate | 424.5 | ** | -- |
| isopropyl (2-((3-(1H-pyrazol-4-yl)imidazo[1,2-b]pyridazin-6-yl)amino)ethyl)(methyl)carbamate | 343.4 | ** | -- |
| isopropyl (2-((3-(2,4-dimethylthiazol-5-yl)imidazo[1,2-b]pyridazin-6-yl)amino)ethyl)(methyl)carbamate | 388.5 | ** | -- |
| isopropyl (2-((3-(2-aminopyridin-4-yl)imidazo[1,2-b]pyridazin-6-yl)amino)ethyl)(methyl)carbamate | 369.4 | ** | -- |
| isopropyl (2-((3-(2-fluorophenyl)imidazo[1,2-b]pyridazin-6-yl)amino)ethyl)(methyl)carbamate | 371.4 | ** | -- |
| isopropyl (2-((3-(2-methoxyphenyl)imidazo[1,2-b]pyridazin-6-yl)amino)ethyl)(methyl)carbamate | 383.4 | ** | -- |
| isopropyl (2-((3-(3-methoxypyridin-4-yl)imidazo[1,2-b]pyridazin-6-yl)amino)ethyl)(methyl)carbamate | 384.4 | ** | -- |
| isopropyl (2-((3-(3-methoxypyridin-4-yl)imidazo[1,2-b]pyridazin-6-yl)amino)ethyl)carbamate | 370.4 | ** | -- |
| isopropyl (2-((3-(4,5-difluoro-2-methoxyphenyl)imidazo[1,2-b]pyridazin-6-yl)amino)ethyl)(isopropyl)carbamate | 447.5 | ** | -- |
| isopropyl (2-((3-(4-carbamoylphenyl)imidazo[1,2-b]pyridazin-6-yl)amino)ethyl)(isopropyl)carbamate | 424.5 | ** | -- |
| isopropyl (2-((3-(4-carbamoylphenyl)imidazo[1,2-b]pyridazin-6-yl)amino)ethyl)(methyl)carbamate | 396.4 | *** | -- |
| isopropyl (2-((3-(4-fluoro-2-methoxyphenyl)imidazo[1,2-b]pyridazin-6-yl)amino)ethyl)(methyl)carbamate | 401.4 | ** | -- |
| isopropyl (2-((3-(5-fluoro-2-methoxyphenyl)imidazo[1,2-b]pyridazin-6-yl)amino)ethyl)(methyl)carbamate | 401.4 | *** | -- |
| isopropyl (2-((3-(5-fluoro-2-methoxyphenyl)imidazo[1,2-b]pyridazin-6-yl)amino)ethyl)(isopropyl)carbamate | 429.5 | ** | -- |
| isopropyl (3-((3-(4-(aminomethyl)phenyl)imidazo[1,2-b]pyridazin-6-yl)amino)propyl)(methyl)carbamate | 396.5 | ** | -- |
| isopropyl (3-((3-(5-acetylthiophen-2-yl)imidazo[1,2-b]pyridazin-6-yl)amino)propyl)(methyl)carbamate | 415.5 | ** | -- |
| isopropyl 4-(3-(2-(difluoromethoxy)phenyl)imidazo[1,2-b]pyridazin-6-yl)piperazine-1-carboxylate | 431.4 | ** | *** |
| isopropyl 4-(3-(2-(difluoromethoxy)pyridin-3-yl)imidazo[1,2-b]pyridazin-6-yl)piperazine-1-carboxylate | 432.4 | ** | *** |
| isopropyl 4-(3-(2,2-difluorobenzo[d][1,3]dioxol-4-yl)imidazo[1,2-b]pyridazin-6-yl)piperazine-1-carboxylate | 445.4 | * | *** |
| isopropyl 4-(3-(2,3-dihydrobenzofuran-7-yl)imidazo[1,2-b]pyridazin-6-yl)piperazine-1-carboxylate | 407.5 | ** | -- |
| isopropyl 4-(3-(2,3-dimethoxyphenyl)imidazo[1,2-b]pyridazin-6-yl)piperazine-1-carboxylate | 425.5 | ** | *** |
| isopropyl 4-(3-(2,5-dimethoxyphenyl)imidazo[1,2-b]pyridazin-6-yl)piperazine-1-carboxylate | 425.5 | ** | *** |
| isopropyl 4-(3-(2-ethoxyphenyl)imidazo[1,2-b]pyridazin-6-yl)piperazine-1-carboxylate | 409.5 | ** | *** |
| isopropyl 4-(3-(2-fluoro-3-methoxyphenyl)imidazo[1,2-b]pyridazin-6-yl)piperazine-1-carboxylate | 413.4 | ** | *** |
| isopropyl 4-(3-(2-fluorophenyl)imidazo[1,2-b]pyridazin-6-yl)piperazine-1-carboxylate | 383.4 | ** | *** |
| isopropyl 4-(3-(2-methoxyphenyl)imidazo[1,2-b]pyridazin-6-yl)piperazine-1-carboxylate | 395.5 | *** | *** |
| isopropyl 4-(3-(2-methoxypyridin-3-yl)imidazo[1,2-b]pyridazin-6-yl)piperazine-1-carboxylate | 396.4 | *** | *** |
| isopropyl 4-(3-(2-methoxypyridin-4-yl)imidazo[1,2-b]pyridazin-6-yl)piperazine-1-carboxylate | 396.4 | ** | *** |
| isopropyl 4-(3-(2-oxo-1,2-dihydropyridin-3-yl)imidazo[1,2-b]pyridazin-6-yl)piperazine-1-carboxylate | 382.4 | ** | *** |
| isopropyl 4-(3-(3-chlorophenyl)imidazo[1,2-b]pyridazin-6-yl)piperazine-1-carboxylate | 399.9 | * | *** |
| isopropyl 4-(3-(3-methoxyphenyl)imidazo[1,2-b]pyridazin-6-yl)piperazine-1-carboxylate | 395.5 | ** | *** |
| isopropyl 4-(3-(3-methoxypyridin-4-yl)imidazo[1,2-b]pyridazin-6-yl)piperazine-1-carboxylate | 396.4 | *** | -- |
| isopropyl 4-(3-(benzo[d][1,3]dioxol-4-yl)imidazo[1,2-b]pyridazin-6-yl)piperazine-1-carboxylate | 409.4 | ** | -- |
| isopropyl 4-(3-(methylcarbamoyl)imidazo[1,2-b]pyridazin-6-yl)piperazine-1-carboxylate | 346.4 | -- | ** |
| isopropyl 4-(3-(pyridin-4-yl)imidazo[1,2-b]pyridazin-6-yl)piperazine-1-carboxylate | 366.4 | ** | -- |
| isopropyl 4-(3-phenylimidazo[1,2-b]pyridazin-6-yl)piperazine-1-carboxylate | 365.4 | ** | -- |
| isopropyl ethyl(2-((3-(3-methoxypyridin-4-yl)imidazo[1,2-b]pyridazin-6-yl)amino)ethyl)carbamate | 398.5 | ** | -- |
| isopropyl ethyl(2-((3-(4-(methylcarbamoyl)phenyl)imidazo[1,2-b]pyridazin-6-yl)amino)ethyl)carbamate | 424.5 | ** | -- |
| isopropyl isopropyl(2-((3-(2-methoxyphenyl)imidazo[1,2-b]pyridazin-6-yl)amino)ethyl)carbamate | 411.5 | ** | -- |
| isopropyl isopropyl(2-((3-(3-methoxypyridin-4-yl)imidazo[1,2-b]pyridazin-6-yl)amino)ethyl)carbamate | 412.5 | *** | -- |
| isopropyl isopropyl(2-((3-(4-(methylcarbamoyl)phenyl)imidazo[1,2-b]pyridazin-6-yl)amino)ethyl)carbamate | 438.5 | ** | -- |
| isopropyl isopropyl(2-((3-(pyridin-4-yl)imidazo[1,2-b]pyridazin-6-yl)amino)ethyl)carbamate | 382.5 | ** | -- |
| isopropyl isopropyl(2-((3-phenylimidazo[1,2-b]pyridazin-6-yl)amino)ethyl)carbamate | 381.5 | ** | -- |
| isopropyl methyl(2-((3-(4-(methylcarbamoyl)phenyl)imidazo[1,2-b]pyridazin-6-yl)amino)ethyl)carbamate | 410.5 | ** | -- |
| isopropyl methyl(2-((3-(4-(pyrrolidin-2-yl)phenyl)imidazo[1,2-b]pyridazin-6-yl)amino)ethyl)carbamate | 422.5 | *** | -- |
| isopropyl methyl(2-((3-(thiazol-4-yl)imidazo[1,2-b]pyridazin-6-yl)amino)ethyl)carbamate | 360.4 | ** | -- |
| methyl isopropyl(2-((3-(3-methoxypyridin-4-yl)imidazo[1,2-b]pyridazin-6-yl)amino)ethyl)carbamate | 384.4 | *** | -- |
| N-((1R,2S)-2-aminocyclohexyl)-4-(6-(butylamino)imidazo[1,2-b]pyridazin-3-yl)benzamide | 406.5 | ** | -- |
| N-(2-((3-(4-cyanophenyl)imidazo[1,2-b]pyridazin-6-yl)amino)ethyl)-N,3,3-trimethylbutanamide | 390.5 | ** | -- |
| N-(2-((3-(4-cyanophenyl)imidazo[1,2-b]pyridazin-6-yl)amino)ethyl)-N-methylpivalamide | 376.5 | ** | -- |
| N-(2-(cyclopentyloxy)ethyl)-3-(1H-pyrazol-3-yl)imidazo[1,2-b]pyridazin-6-amine | 312.4 | * | ** |
| N-(2-(cyclopentyloxy)ethyl)-3-(2-fluoropyridin-3-yl)imidazo[1,2-b]pyridazin-6-amine | 341.4 | * | *** |
| N-(2-(cyclopentyloxy)ethyl)-3-(2-methoxypyridin-3-yl)imidazo[1,2-b]pyridazin-6-amine | 353.4 | ** | -- |
| N-(2-(cyclopentyloxy)ethyl)-3-(3-fluoropyridin-4-yl)imidazo[1,2-b]pyridazin-6-amine | 341.4 | ** | -- |
| N-(2-(cyclopentyloxy)ethy)-3-(3-methoxyphenyl)imidazo[1,2-b]pyridazin-6-amine | 352.4 | -- | ** |
| N-(2-(cyclopentyloxy)ethyl)-3-(3-methoxypyridin-4-yl)imidazo[1,2-b]pyridazin-6-amine | 353.4 | * | -- |
| N-(2-(cyclopentyloxy)ethyl)-3-(4-(pyrrolidin-2-yl)phenyl)imidazo[1,2-b]pyridazin-6-amine | 391.5 | ** | -- |
| N-(2-(cyclopentyloxy)ethyl)-3-(4-methylthiophen-2-yl)imidazo[1,2-b]pyridazin-6-amine | 342.5 | -- | ** |
| N-(2-(cyclopentyloxy)ethyl)-3-(5-methoxypyridin-3-yl)imidazo[1,2-b]pyridazin-6-amine | 353.4 | ** | *** |
| N-(2-(cyclopentyloxy)ethyl)-3-(5-methylthiophen-2-yl)imidazo[1,2-b]pyridazin-6-amine | 342.5 | -- | ** |
| N-(2-(cyclopentyloxy)ethyl)-3-(pyridin-2-yl)imidazo[1,2-b]pyridazin-6-amine | 323.4 | ** | *** |
| N-(2-(cyclopentyloxy)ethyl)-3-(thiophen-2-yl)imidazo[1,2-b]pyridazin-6-amine | 328.4 | -- | *** |
| N-(2-(cyclopentyloxy)ethyl)-3-(thiophen-3-yl)imidazo[1,2-b]pyridazin-6-amine | 328.4 | -- | ** |
| N-(2-(diethylamino)ethyl)-4-(6-((2-isobutoxyethyl)amino)imidazo[1,2-b]pyridazin-3-yl)benzamide | 452.6 | ** | -- |
| N-(2-(diethylamino)ethyl)-4-(6-((2-isopropoxyethyl)amino)imidazo[1,2-b]pyridazin-3-yl)benzamide | 438.6 | ** | -- |
| N-(2-(methylamino)ethyl)-4-(6-((3-(N-methylpivalamido)propyl)amino)imidazo[1,2-b]pyridazin-3-yl)benzamide | 465.6 | * | -- |
| N-(2-(tert-butoxy)ethyl)-3-(3-methoxypyridin-4-yl)imidazo[1,2-b]pyridazin-6-amine | 341.4 | ** | -- |
| N-(2-(tert-butoxy)ethyl)-3-(4-(pyrrolidin-2-yl)phenyl)imidazo[1,2-b]pyridazin-6-amine | 379.5 | ** | -- |
| N-(2-aminoethyl)-4-(6-((3-(tert-butoxy)propyl)amino)imidazo[1,2-b]pyridazin-3-yl)benzamide | 410.5 | * | -- |
| N-(2-aminoethyl)-4-(6-((3,3-dimethylbutyl)amino)imidazo[1,2-b]pyridazin-3-yl)benzamide | 380.5 | * | -- |
| N-(2-aminoethyl)-4-(6-((3-phenylpropyl)amino)imidazo[1,2-b]pyridazin-3-yl)benzamide | 414.5 | * | -- |
| N-(2-aminoethyl)-4-(6-(butylamino)imidazo[1,2-b]pyridazin-3-yl)-2-methoxybenzamide | 382.5 | * | -- |
| N-(2-aminoethyl)-4-(6-(butylamino)imidazo[1,2-b]pyridazin-3-yl)-2-fluorobenzamide | 370.4 | ** | -- |
| N-(2-aminoethyl)-4-(6-(butylamino)imidazo[1,2-b]pyridazin-3-yl)-3-fluorobenzamide | 370.4 | * | -- |
| N-(2-aminoethyl)-4-(6-(butylamino)imidazo[1,2-b]pyridazin-3-yl)benzamide | 352.4 | * | -- |
| N-(2-aminopropyl)-4-(6-(butylamino)imidazo[1,2-b]pyridazin-3-yl)benzamide | 366.5 | * | -- |
| N-(2-ethoxyphenethyl)-3-(3-methoxypyridin-4-yl)imidazo[1,2-b]pyridazin-6-amine | 389.5 | ** | -- |
| N-(2-isobutoxyethyl)-3-(2-methoxyphenyl)imidazo[1,2-b]pyridazin-6-amine | 340.4 | * | -- |
| N-(2-isobutoxyethyl)-3-(3-methoxypyridin-4-yl)imidazo[1,2-b]pyridazin-6-amine | 341.4 | ** | -- |
| N-(2-isobutoxyethyl)-3-(pyridin-4-yl)imidazo[1,2-b]pyridazin-6-amine | 311.4 | * | -- |
| N-(2-isopropoxyethyl)-3-(1H-pyrazol-3-yl)imidazo[1,2-b]pyridazin-6-amine | 286.3 | * | -- |
| N-(2-isopropoxyethyl)-3-(3-methoxypyridin-4-yl)imidazo[1,2-b]pyridazin-6-amine | 327.4 | *** | -- |
| N-(2-isopropoxyethyl)-3-(4-(pyrrolidin-2-yl)phenyl)imidazo[1,2-b]pyridazin-6-amine | 365.5 | ** | -- |
| N-(2-isopropoxyethyl)-3-(isothiazol-5-yl)imidazo[1,2-b]pyridazin-6-amine | 303.4 | ** | -- |
| N-(2-isopropoxyethyl)-3-(thiazol-4-yl)imidazo[1,2-b]pyridazin-6-amine | 303.4 | * | -- |
| N-(3-((3-(4-(aminomethyl)phenyl)imidazo[1,2-b]pyridazin-6-yl)amino)propyl)-N-methylpivalamide | 394.5 | * | -- |
| N-(3-(6-((2-(cyclopentyloxy)ethyl)amino)imidazo[1,2-b]pyridazin-3-yl)phenyl)acetamide | 379.5 | ** | ** |
| N-(3-(tert-butoxy)propyl)-3-(3-methoxypyridin-4-yl)imidazo[1,2-b]pyridazin-6-amine | 355.4 | * | -- |
| N-(3,3-dimethylbutyl)-3-(4-((2-(methylamino)ethoxy)methyl)phenyl)imidazo[1,2-b]pyridazin-6-amine | 381.5 | ** | -- |
| N-(3,3-dimethylbutyl)-3-(4-(pyrrolidin-2-yl)phenyl)imidazo[1,2-b]pyridazin-6-amine | 363.5 | * | -- |
| N-(3-aminopropyl)-4-(6-(butylamino)imidazo[1,2-b]pyridazin-3-yl)benzamide | 366.5 | * | -- |
| N-(3-fluoropropyl)-3-(4-(pyrrolidin-2-yl)phenyl)imidazo[1,2-b]pyridazin-6-amine | 339.4 | ** | -- |
| N-(3-phenylpropyl)-3-(4-(pyrrolidin-2-yl)phenyl)imidazo[1,2-b]pyridazin-6-amine | 397.5 | * | -- |
| N-(4-(2-aminoethoxy)phenyl)-6-(butylamino)imidazo[1,2-b]pyridazine-3-carboxamide | 368.4 | * | -- |
| N-(4-(aminomethyl)phenyl)-6-(butylamino)imidazo[1,2-b]pyridazine-3-carboxamide | 338.4 | * | -- |
| N-(cyclopentylmethyl)-3-(3-methoxypyridin-4-yl)imidazo[1,2-b]pyridazin-6-amine | 323.4 | ** | -- |
| N-(tert-butyl)-4-(3-phenylimidazo[1,2-b]pyridazin-6-yl)piperazine-1-carboxamide | 378.5 | ** | *** |
| N,N-dimethyl-3-(6-(4-(pyrrolidine-1-carbonyl)piperazin-1-yl)imidazo[1,2-b]pyridazin-3-yl)benzamide | 447.5 | ** | *** |
| N1-(3-(4-(aminomethyl)phenyl)imidazo[1,2-b]pyridazin-6-yl)-N2-methyl-N2-phenylethane-1,2-diamine | 372.5 | ** | -- |
| N1-(3-(4-(aminomethyl)phenyl)imidazo[1,2-b]pyridazin-6-yl)-N3-methyl-N3-phenylpropane-1,3-diamine | 386.5 | ** | -- |
| N1-(3-(5-(aminomethyl)thiophen-2-yl)imidazo[1,2-b]pyridazin-6-yl)-N3-methyl-N3-phenylpropane-1,3-diamine | 392.5 | * | -- |
| N1-(4-(6-((3,3-dimethylbutyl)amino)imidazo[1,2-b]pyridazin-3-yl)benzyl)ethane-1,2-diamine | 366.5 | * | -- |
| N1-(4-(6-(butylamino)imidazo[1,2-b]pyridazin-3-yl)benzyl)ethane-1,2-diamine | 338.4 | * | -- |
| N1-(4-(6-(butylamino)imidazo[1,2-b]pyridazin-3-yl)benzyl)propane-1,3-diamine | 352.5 | * | -- |
| N-butyl-3-(1H-pyrazol-3-yl)imidazo[1,2-b]pyridazin-6-amine | 256.3 | ** | -- |
| N-butyl-3-(2,4-dimethylthiazol-5-yl)imidazo[1,2-b]pyridazin-6-amine | 301.4 | ** | -- |
| N-butyl-3-(3-methoxypyridin-4-yl)imidazo[1,2-b]pyridazin-6-amine | 297.4 | ** | -- |
| N-butyl-3-(4-((((tetrahydrofuran-2-yl)methyl)amino)methyl)phenyl)imidazo[1,2-b]pyridazin-6-amine | 379.5 | * | -- |
| N-butyl-3-(4-(((2-methoxyethyl)amino)methyl)phenyl)imidazo[1,2-b]pyridazin-6-amine | 353.5 | * | -- |
| N-butyl-3-(4-(((cyclopropylmethyl)amino)methyl)phenyl)imidazo[1,2-b]pyridazin-6-amine | 349.5 | * | -- |
| N-butyl-3-(4-((isopropylamino)methyl)phenyl)imidazo[1,2-b]pyridazin-6-amine | 337.5 | * | -- |
| N-butyl-3-(4-((propylamino)methyl)phenyl)imidazo[1,2-b]pyridazin-6-amine | 337.5 | * | -- |
| N-butyl-3-(4-((tert-butylamino)methyl)-2-fluorophenyl)imidazo[1,2-b]pyridazin-6-amine | 369.5 | * | -- |
| N-butyl-3-(4-((tert-butylamino)methyl)-3-fluorophenyl)imidazo[1,2-b]pyridazin-6-amine | 369.5 | ** | -- |
| N-butyl-3-(4-(pyrrolidin-2-yl)phenyl)imidazo[1,2-b]pyridazin-6-amine | 335.4 | * | -- |
| N-butyl-3-(isoindolin-5-yl)imidazo[1,2-b]pyridazin-6-amine | 307.4 | * | -- |
| N-butyl-3-(isoquinolin-6-yl)imidazo[1,2-b]pyridazin-6-amine | 317.4 | ** | -- |
| N-cyclohexyl-3-(3-methoxypyridin-4-yl)imidazo[1,2-b]pyridazin-6-amine | 323.4 | | *** |
| N-isobutyl-3-(3-(3-methoxypyridin-4-yl)imidazo[1,2-b]pyridazin-6-yl)benzamide | 401.5 | ** | -- |
| N-isobutyl-3-(3-(4-(methylcarbamoyl)phenyl)imidazo[1,2-b]pyridazin-6-yl)benzamide | 427.5 | ** | -- |
| N-isopentyl-3-(3-methoxypyridin-4-yl)imidazo[1,2-b]pyridazin-6-amine | 311.4 | ** | -- |
| N-isopropyl-4-(3-(2-methoxyphenyl)imidazo[1,2-b]pyridazin-6-yl)-N-methylpiperazine-1-carboxamide | 408.5 | ** | *** |
| N-isopropyl-4-(3-(2-methoxyphenyl)imidazo[1,2-b]pyridazin-6-yl)piperazine-1-carboxamide | 394.5 | * | *** |
| N-isopropyl-4-(3-phenylimidazo[1,2-b]pyridazin-6-yl)piperazine-1-carboxamide | 364.4 | ** | *** |
| N-methyl-4-(6-((2-(trifluoromethoxy)phenethyl)amino)imidazo[1,2-b]pyridazin-3-yl)benzamide | 455.4 | *** | -- |
| N-pentyl-3-(4-(pyrrolidin-2-yl)phenyl)imidazo[1,2-b]pyridazin-6-amine | 349.5 | * | -- |
| piperidin-1-yl(4-(3-(pyridin-2-yl)imidazo[1,2-b]pyridazin-6-yl)piperazin-1-yl)methanone | 391.5 | ** | *** |
| propyl isopropyl(2-((3-(3-methoxypyridin-4-yl)imidazo[1,2-b]pyridazin-6-yl)amino)ethyl)carbamate | 412.5 | * | -- |
| S-isopropyl 4-(3-phenylimidazo[1,2-b]pyridazin-6-yl)piperazine-1-carbothioate | 381.5 | * | *** |
| tert-butyl (1-(3-(2-methoxyphenyl)imidazo[1,2-b]pyridazin-6-yl)pyrrolidin-3-yl)(methyl)carbamate | 423.5 | * | *** |
| tert-butyl (1-(3-(2-methoxypyridin-3-yl)imidazo[1,2-b]pyridazin-6-yl)pyrrolidin-3-yl)(methyl)carbamate | 424.5 | ** | *** |
| tert-butyl (1-(3-(2-methoxypyridin-3-yl)imidazo[1,2-b]pyridazin-6-yl)azetidin-3-yl)(methyl)carbamate | 410.5 | -- | * |
| tert-butyl (2-((3-(2-aminopyridin-4-yl)imidazo[1,2-b]pyridazin-6-yl)amino)ethyl)(methyl)carbamate | 383.4 | *** | -- |
| tert-butyl (2-((3-(2-fluorophenyl)imidazo[1,2-b]pyridazin-6-yl)amino)ethyl)(methyl)carbamate | 385.4 | ** | -- |
| tert-butyl (2-((3-(2-fluoropyridin-4-yl)imidazo[1,2-b]pyridazin-6-yl)amino)ethyl)(methyl)carbamate | 386.4 | ** | -- |
| tert-butyl (2-((3-(2-methoxyphenyl)imidazo[1,2-b]pyridazin-6-yl)amino)ethyl)(methyl)carbamate | 397.5 | ** | -- |
| tert-butyl (2-((3-(3-carbamoylphenyl)imidazo[1,2-b]pyridazin-6-yl)amino)ethyl)(methyl)carbamate | 410.5 | ** | -- |
| tert-butyl (2-((3-(3-methoxyphenyl)imidazo[1,2-b]pyridazin-6-yl)amino)ethyl)(methyl)carbamate | 397.5 | ** | -- |
| tert-butyl (2-((3-(3-methoxypyridin-4-yl)imidazo[1,2-b]pyridazin-6-yl)amino)ethyl)(methyl)carbamate | 398.5 | * | -- |
| tert-butyl (2-((3-(4-(aminomethyl)phenyl)imidazo[1,2-b]pyridazin-6-yl)amino)ethyl)carbamate | 382.5 | ** | -- |
| tert-butyl (2-((3-(4-(cyanomethyl)phenyl)imidazo[1,2-b]pyridazin-6-yl)amino)ethyl)(methyl)carbamate | 406.5 | ** | -- |
| tert-butyl (2-((3-(4-carbamoylphenyl)imidazo[1,2-b]pyridazin-6-yl)amino)ethyl)(methyl)carbamate | 410.5 | ** | *** |
| tert-butyl (2-((3-(4-carbamoylphenyl)imidazo[1,2-b]pyridazin-6-yl)amino)ethyl)(isopropyl)carbamate | 438.5 | *** | -- |
| tert-butyl (2-((3-(4-cyanophenyl)imidazo[1,2-b]pyridazin-6-yl)amino)ethyl)(methyl)carbamate | 392.5 | ** | -- |
| tert-butyl (2-((3-(5-acetylthiophen-2-yl)imidazo[1,2-b]pyridazin-6-yl)amino)ethyl)(methyl)carbamate | 415.5 | *** | -- |
| tert-butyl (3-((3-(4-(aminomethyl)phenyl)imidazo[1,2-b]pyridazin-6-yl)amino)propyl)(methyl)carbamate | 410.5 | ** | -- |
| tert-butyl (3-((3-(5-acetylthiophen-2-yl)imidazo[1,2-b]pyridazin-6-yl)amino)propyl)(methyl)carbamate | 429.5 | ** | -- |
| tert-butyl 2-(2-((3-(4-(aminomethyl)phenyl)imidazo[1,2-b]pyridazin-6-yl)amino)ethyl)pyrrolidine-1-carboxylate | 436.5 | *** | -- |
| tert-butyl2-(2-((3-(4-carbamoylphenyl)imidazo[1,2-b]pyridazin-6-yl)amino)ethyl)pyrrolidine-1-carboxylate | 450.5 | ** | -- |
| tert-butyl 4-(3-(3-methoxypyridin-4-yl)imidazo[1,2-b]pyridazin-6-yl)piperazine-1-carboxylate | 410.5 | *** | -- |
| tert-butyl 4-(3-chloroimidazo[1,2-b]pyridazin-6-yl)piperazine-1-carboxylate | 337.8 | ** | -- |
| tert-butyl 4-(3-iodoimidazo[1,2-b]pyridazin-6-yl)piperazine-1-carboxylate | 429.3 | ** | *** |
| tert-butyl 4-(3-phenylimidazo[1,2-b]pyridazin-6-yl)piperazine-1-carboxylate | 379.5 | ** | *** |
| tert-butyl isopropyl(2-((3-(3-methoxypyridin-4-yl)imidazo[1,2-b]pyridazin-6-yl)amino)ethyl)carbamate | 426.5 | ** | -- |
| tert-butyl methyl(1-(3-phenylimidazo[1,2-b]pyridazin-6-yl)pyrrolidin-3-yl)carbamate | 393.5 | ** | ** |
| tert-butyl methyl(2-((3-(4-(methylcarbamoyl)phenyl)imidazo[1,2-b]pyridazin-6-yl)amino)ethyl)carbamate | 424.5 | ** | -- |
| tert-butyl methyl(2-((3-(4-(pyrrolidin-2-yl)phenyl)imidazo[1,2-b]pyridazin-6-yl)amino)ethyl)carbamate | 436.5 | *** | -- |
| tert-butyl methyl(2-((3-(pyridin-4-yl)imidazo[1,2-b]pyridazin-6-yl)amino)ethyl)carbamate | 368.4 | ** | -- |
| tert-butyl methyl(2-((3-phenylimidazo[1,2-b]pyridazin-6-yl)amino)ethyl)carbamate | 367.4 | ** | -- |

### 5.4.6. Pyrazolo[1,5-a]pyrimidine-based Inhibitor In Vitro Data

*In vitro* data obtained for various compounds of the invention are provided below in Table 1, wherein "MW" means molecular weight, "P81 Assay" refers to the P81 filter plate assay described above, "CBA" refers to the HEK281 cell-based assay described above, "--" means that results for the given assay were not obtained, "*" means less than or equal to 1.0 µM, "**" means a value of less than or equal to 0.1 µM, and "***" means less than or equal to 0.01 µM.

**Table 1**

| **Compound** | **MW** | **CBA IC₅₀ µM** | **P81 IC₅₀ µM** |
|---|---|---|---|
| (4-(3-(2-methoxypyridin-3-yl)pyrazolo[1,5-a]pyrimidin-5-yl)piperazin-1-yl)(pyrrolidin-1-yl)methanone | 407.5 | ** | *** |
| (S)-1-(2-(((3-(2-methoxyphenyl)pyrazolo[1,5-a]pyrimidin-5-yl)amino)methyl)pyrrolidin-1-yl)butan-1-one | 393.5 | ** | *** |
| (S)-1-(2-(((3-(2-methoxyphenyl)pyrazolo[1,5-a]pyrimidin-5-yl)amino)methyl)pyrrolidin-1-yl)-3,3-dimethylbutan-1-one | 421.5 | *** | *** |
| (S)-1-(3,3-dimethylbutyl)-5-(((3-(2-ethoxyphenyl)pyrazolo[1,5-a]pyrimidin-5-yl)amino)methyl)pyrrolidin-2-one | 435.6 | ** | *** |
| (S)-1-(3,3-dimethylbutyl)-5-(((3-(2-methoxyphenyl)pyrazolo[1,5-a]pyrimidin-5-yl)amino)methyl)pyrrolidin-2-one | 421.5 | *** | *** |
| (S)-1-(3,3-dimethylbutyl)-5-(((3-(2-methoxypyridin-3-yl)pyrazolo[1,5-a]pyrimidin-5-yl)amino)methyl)pyrrolidin-2-one | 422.5 | ** | *** |
| (S)-1-(3,3-dimethylbutyl)-5-(((3-(3-methoxypyridin-4-yl)pyrazolo[1,5-a]pyrimidin-5-yl)amino)methyl)pyrrolidin-2-one | 422.5 | ** | *** |
| (S)-1-(3,3-dimethylbutyl)-5-(((3-phenylpyrazolo[1,5-a]pyrimidin-5-yl)amino)methyl)pyrrolidin-2-one | 391.5 | -- | *** |
| (S)-2-(((3-bromopyrazolo[1,5-a]pyrimidin-5-yl)amino)methyl)-N-(tert-butyl)pyrrolidine-1-carboxamide | 395.3 | *** | *** |
| (S)-2-cyclopropyl-N-methyl-N-(1-(3-(pyridin-2-yl)pyrazolo[1,5-a]pyrimidin-5-yl)pyrrolidin-3-yl)acetamide | 376.5 | -- | ** |
| (S)-3,3,3-trifluoro-1-(2-(((3-(2-methoxyphenyl)pyrazolo[1,5-a]pyrimidin-5-yl)amino)methyl)pyrrolidin-1-yl)propan-1-one | 433.4 | ** | *** |
| (S)-3,3,3-trifluoro-1-(2-(((3-(pyridin-4-yl)pyrazolo[1,5-a]pyrimidin-5-yl)amino)methyl)pyrrolidin-1-yl)propan-1-one | 404.4 | ** | *** |
| (S)-3,3,3-trifluoro-N-(1-(3-(2-methoxyphenyl)pyrazolo[1,5-a]pyrimidin-5-yl)pyrrolidin-3-yl)-N-methylpropanamide | 433.4 | -- | ** |
| (S)-3,3,3-trifluoro-N-(1-(3-(2-methoxypyridin-3-yl)pyrazolo[1,5-a]pyrimidin-5-yl)pyrrolidin-3-yl)-N-methylpropanamide | 434.4 | *** | *** |
| (S)-3,3,3-trifluoro-N-methyl-N-(1-(3-(pyridin-2-yl)pyrazolo[1,5-a]pyrimidin-5-yl)pyrrolidin-3-yl)propanamide | 404.4 | *** | *** |
| (S)-5-(((3-bromopyrazolo[1,5-a]pyrimidin-5-yl)amino)methyl)-1-(3,3-dimethylbutyl)pyrrolidin-2-one | 394.3 | ** | *** |
| (S)-ethyl 2-(((3-(2-methoxyphenyl)pyrazolo[1,5-a]pyrimidin-5-yl)amino)methyl)pyrrolidine-1-carboxylate | 395.5 | *** | *** |
| (S)-isopropyl (1-(3-(2-methoxyphenyl)pyrazolo[1,5-a]pyrimidin-5-yl)pyrrolidin-3-yl)(methyl)carbamate | 409.5 | -- | ** |
| (S)-isopropyl (1-(3-(2-methoxypyridin-3-yl)pyrazolo[1,5-a]pyrimidin-5-yl)pyrrolidin-3-yl)(methyl)carbamate | 410.5 | -- | ** |
| (S)-isopropyl2-(((3-(2-methoxyphenyl)pyrazolo[1,5-a]pyrimidin-5-yl)amino)methyl)pyrrolidine-1-carboxylate | 409.5 | *** | *** |
| (S)-isopropyl methyl(1-(3-(pyridin-2-yl)pyrazolo[1,5-a]pyrimidin-5-yl)pyrrolidin-3-yl)carbamate | 380.4 | -- | * |
| (S)-isopropyl methyl(1-(3-(pyridin-4-yl)pyrazolo[1,5-a]pyrimidin-5-yl)pyrrolidin-3-yl)carbamate | 380.4 | -- | ** |
| (S)-methyl 2-(((3-(2-methoxyphenyl)pyrazolo[1,5-a]pyrimidin-5-yl)amino)methyl)pyrrolidine-1-carboxylate | 381.4 | ** | *** |
| (S)-N-(1-(3-(2-methoxyphenyl)pyrazolo[1,5-a]pyrimidin-5-yl)pyrrolidin-3-yl)-N-methylbutyramide | 393.5 | * | *** |
| (S)-N-(1-(3-(2-methoxypyridin-3-yl)pyrazolo[1,5-a]pyrimidin-5-yl)pyrrolidin-3-yl)-N-methylbutyramide | 394.5 | * | *** |
| (S)-N-(1-(3-bromopyrazolo[1,5-a]pyrimidin-5-yl)pyrrolidin-3-yl)-3,3,3-trifluoro-N-methylpropanamide | 406.2 | -- | ** |
| (S)-N-(1-(3-bromopyrazolo[1,5-a]pyrimidin-5-yl)pyrrolidin-3-yl)-N-methylpyrrolidine-1-carboxamide | 393.3 | -- | ** |
| (S)-N-(tert-butyl)-2-(((3-(2-methoxyphenyl)pyrazolo[1,5-a]pyrimidin-5-yl)amino)methyl)pyrrolidine-1-carboxamide | 422.5 | ** | *** |
| (S)-N-methyl-N-(1-(3-(pyridin-2-yl)pyrazolo[1,5-a]pyrimidin-5-yl)pyrrolidin-3-yl)butyramide | 364.4 | -- | * |
| (S)-tert-butyl (1-(3-(2-methoxyphenyl)pyrazolo[1,5-a]pyrimidin-5-yl)pyrrolidin-3-yl)(methyl)carbamate | 423.5 | ** | *** |
| (S)-tert-butyl 2-(((3-(2-(methoxymethyl)phenyl)pyrazolo[1,5-a]pyrimidin-5-yl)amino)methyl)pyrrolidine-1-carboxylate | 437.5 | ** | *** |
| (S)-tert-butyl 2-(((3-(2-ethylphenyl)pyrazolo[1,5-a]pyrimidin-5-yl)amino)methyl)pyrrolidine-1-carboxylate | 421.5 | ** | *** |
| (S)-tert-butyl 2-(((3-(2-hydroxyphenyl)pyrazolo[1,5-a]pyrimidin-5-yl)amino)methyl)pyrrolidine-1-carboxylate | 409.5 | ** | *** |
| (S)-tert-butyl 2-(((3-(2-isopropoxyphenyl)pyrazolo[1,5-a]pyrimidin-5-yl)amino)methyl)pyrrolidine-1-carboxylate | 451.6 | *** | *** |
| (S)-tert-butyl 2-(((3-(2-methoxyphenyl)pyrazolo[1,5-a]pyrimidin-5-yl)amino)methyl)pyrrolidine-1-carboxylate | 423.5 | *** | -- |
| (S)-tert-butyl 2-(((3-(3-methoxyphenyl)pyrazolo[1,5-a]pyrimidin-5-yl)amino)methyl)pyrrolidine-1-carboxylate | 423.5 | *** | *** |
| (S)-tert-butyl 2-(((3-(4-(aminomethyl)phenyl)pyrazolo[1,5-a]pyrimidin-5-yl)amino)methyl)pyrrolidine-1-carboxylate | 422.5 | *** | *** |
| (S)-tert-butyl 2-(((3-(4-carbamoylphenyl)pyrazolo[1,5-a]pyrimidin-5-yl)amino)methyl)pyrrolidine-1-carboxylate | 436.5 | * | *** |
| (S)-tert-butyl 2-(((3-(4-methoxyphenyl)pyrazolo[1,5-a]pyrimidin-5-yl)amino)methyl)pyrrolidine-1-carboxylate | 423.5 | *** | *** |
| (S)-tert-butyl 2-(((3-(4-methoxypyridin-3-yl)pyrazolo[1,5-a]pyrimidin-5-yl)amino)methyl)pyrrolidine-1-carboxylate | 424.5 | *** | *** |
| (S)-tert-butyl 2-(((3-(pyridin-4-yl)pyrazolo[1,5-a]pyrimidin-5-yl)amino)methyl)pyrrolidine-1-carboxylate | 394.5 | *** | -- |
| (S)-tert-butyl 2-(((3-(pyridin-4-yl)pyrazolo[1,5-a]pyrimidin-5-yl)amino)methyl)pyrrolidine-1-carboxylate | 394.5 | *** | -- |
| (S)-tert-butyl 2-(((3-(trifluoromethyl)pyrazolo[1,5-a]pyrimidin-5-yl)amino)methyl)pyrrolidine-1-carboxylate | 385.4 | ** | *** |
| (S)-tert-butyl 2-(((3-iodopyrazolo[1,5-a]pyrimidin-5-yl)amino)methyl)pyrrolidine-1-carboxylate | 443.3 | ** | -- |
| (S)-tert-butyl 2-(((3-phenylpyrazolo[1,5-a]pyrimidin-5-yl)amino)methyl)pyrrolidine-1-carboxylate | 393.5 | ** | *** |
| (S)-tert-butyl methyl(1-(3-(2-methylpyridin-4-yl)pyrazolo[1,5-a]pyrimidin-5-yl)pyrrolidin-3-yl)carbamate | 408.5 | ** | *** |
| (S)-tert-butyl methyl(1-(3-(pyridin-2-yl)pyrazolo[1,5-a]pyrimidin-5-yl)pyrrolidin-3-yl)carbamate | 394.5 | -- | * |
| 1-(4-(3-(2-methoxypyridin-3-yl)pyrazolo[1,5-a]pyrimidin-5-yl)piperazin-1-yl)-3-methylbutan-1-one | 394.5 | ** | *** |
| 2,2,2-trifluoroethyl 4-(3-(2-methoxypyridin-3-yl)pyrazolo[1,5-a]pyrimidin-5-yl)piperazine-1-carboxylate | 436.4 | *** | *** |
| 2-fluoroethyl 4-(3-(2-methoxypyridin-3-yl)pyrazolo[1,5-a]pyrimidin-5-yl)piperazine-1-carboxylate | 400.4 | *** | *** |
| 2-methoxyethyl 4-(3-(2-methoxyphenyl)pyrazolo[1,5-a]pyrimidin-5-yl)piperazine-1-carboxylate | 411.5 | -- | *** |
| 2-methoxyethyl 4-(3-(2-methoxypyridin-3-yl)pyrazolo[1,5-a]pyrimidin-5-yl)piperazine-1-carboxylate | 412.4 | ** | *** |
| 3-(2-methoxypyridin-3-yl)-N-(4,4,4-trifluorobutyl)pyrazolo[1,5-a]pyrimidin-5-amine | 351.3 | ** | *** |
| 3-(3-methoxypyridin-4-yl)-N-(2-(trifluoromethoxy)phenethyl)pyrazolo[1,5-a]pyrimidin-5-amine | 429.4 | *** | -- |
| 3-(4-(aminomethyl)phenyl)-N-(2-(cyclopentyloxy)ethyl)pyrazolo[1,5-a]pyrimidin-5-amine | 351.4 | ** | -- |
| 3-(4-(aminomethyl)phenyl)-N-(2-(neopentyloxy)ethyl)pyrazolo[1,5-a]pyrimidin-5-amine | 353.5 | ** | -- |
| 3-(4-(aminomethyl)phenyl)-N-(2-(tert-butoxy)ethyl)pyrazolo[1,5-a]pyrimidin-5-amine | 339.4 | ** | -- |
| 3-(4-(aminomethyl)phenyl)-N-(2-(trifluoromethoxy)phenethyl)pyrazolo[1,5-a]pyrimidin-5-amine | 427.4 | *** | -- |
| 3-(4-(aminomethyl)phenyl)-N-(2-methoxyethyl)pyrazolo[1,5-a]pyrimidin-5-amine | 297.4 | ** | -- |
| 3-(4-(aminomethyl)phenyl)-N-(3-(cyclopentyloxy)propyl)pyrazolo[1,5-a]pyrimidin-5-amine | 365.5 | ** | -- |
| 3-(4-(aminomethyl)phenyl)-N-butylpyrazolo[1,5-a]pyrimidin-5-amine | 295.4 | ** | -- |
| 3-(4-methoxypyridin-3-yl)-N-(2-(trifluoromethoxy)phenethyl)pyrazolo[1,5-a]pyrimidin-5-amine | 429.4 | ** | -- |
| 3-bromo-N-((1-(2,2,2-trifluoroethyl)pyrrolidin-2-yl)methyl)pyrazolo[1,5-a]pyrimidin-5-amine | 378.2 | ** | *** |
| 3-bromo-N-(3-(cyclopentyloxy)propyl)pyrazolo[1,5-a]pyrimidin-5-amine | 339.2 | * | -- |
| 4-(5-(butylamino)pyrazolo[1,5-a]pyrimidin-3-yl)-N-(2-(methylamino)ethyl)benzamide | 366.5 | ** | -- |
| 5-(4-(isobutylsulfonyl)piperazin-1-yl)-3-(2-methoxypyridin-3-yl)pyrazolo[1,5-a]pyrimidine | 430.5 | | ** |
| cyclopentyl 4-(3-(2-methoxypyridin-3-yl)pyrazolo[1,5-a]pyrimidin-5-yl)piperazine-1-carboxylate | 422.5 | *** | *** |
| ethyl 4-(3-(2-ethoxypyridin-3-yl)pyrazolo[1,5-a]pyrimidin-5-yl)piperazine-1-carboxylate | 396.4 | *** | *** |
| ethyl 4-(3-(2-methoxypyridin-3-yl)pyrazolo[1,5-a]pyrimidin-5-yl)piperazine-1-carboxylate | 382.4 | ** | *** |
| ethyl 5-(4-(isopropoxycarbonyl)piperazin-1-yl)pyrazolo[1,5-a]pyrimidine-3-carboxylate | 361.4 | | ** |
| ethyl methyl(2-((3-(4-(methylcarbamoyl)phenyl)pyrazolo[1,5-a]pyrimidin-5-yl)amino)ethyl)carbamate | 396.4 | ** | -- |
| isobutyl (2-((3-(4-carbamoylphenyl)pyrazolo[1,5-a]pyrimidin-5-yl)amino)ethyl)(methyl)carbamate | 410.5 | *** | -- |
| isobutyl methyl(2-((3-(4-(methylcarbamoyl)phenyl)pyrazolo[1,5-a]pyrimidin-5-yl)amino)ethyl)carbamate | 424.5 | * | -- |
| isopropyl (1-(3-(2-methoxypyridin-3-yl)pyrazolo[1,5-a]pyrimidin-5-yl)azetidin-3-yl)(methyl)carbamate | 396.4 | -- | ** |
| isopropyl (2-((3-(3-methoxypyridin-4-yl)pyrazolo[1,5-a]pyrimidin-5-yl)amino)ethyl)(methyl)carbamate | 384.4 | *** | -- |
| isopropyl (2-((3-(4-(aminomethyl)phenyl)pyrazolo[1,5-a]pyrimidin-5-yl)amino)ethyl)(methyl)carbamate | 382.5 | ** | -- |
| isopropyl (2-((3-(4-carbamoylphenyl)pyrazolo[1,5-a]pyrimidin-5-yl)amino)ethyl)(methyl)carbamate | 396.4 | *** | -- |
| isopropyl (2-((3-(4-methoxypyridin-3-yl)pyrazolo[1,5-a]pyrimidin-5-yl)amino)ethyl)(methyl)carbamate | 384.4 | ** | -- |
| isopropyl 4-(3-(1,3,5-trimethyl-1H-pyrazol-4-yl)pyrazolo[1,5-a]pyrimidin-5-yl)piperazine-1-carboxylate | 397.5 | -- | ** |
| isopropyl 4-(3-(1-isobutyl-1H-pyrazol-4-yl)pyrazolo[1,5-a]pyrimidin-5-yl)piperazine-1-carboxylate | 411.5 | -- | ** |
| isopropyl 4-(3-(1-methyl-2-oxo-1,2-dihydropyridin-3-yl)pyrazolo[1,5-a]pyrimidin-5-yl)piperazine-1-carboxylate | 396.4 | ** | *** |
| isopropyl 4-(3-(2-(methoxymethyl)phenyl)pyrazolo[1,5-a]pyrimidin-5-yl)piperazine-1-carboxylate | 409.5 | -- | * |
| isopropyl 4-(3-(2-(methylthio)pyridin-3-yl)pyrazolo[1,5-a]pyrimidin-5-yl)piperazine-1-carboxylate | 412.5 | -- | ** |
| isopropyl 4-(3-(2,6-dimethoxypyridin-3-yl)pyrazolo[1,5-a]pyrimidin-5-yl)piperazine-1-carboxylate | 426.5 | *** | *** |
| isopropyl 4-(3-(2-aminopyridin-3-yl)pyrazolo[1,5-a]pyrimidin-5-yl)piperazine-1-carboxylate | 381.4 | -- | ** |
| isopropyl 4-(3-(2-chloropyridin-3-yl)pyrazolo[1,5-a]pyrimidin-5-yl)piperazine-1-carboxylate | 400.9 | * | ** |
| isopropyl 4-(3-(2-ethoxypyridin-3-yl)pyrazolo[1,5-a]pyrimidin-5-yl)piperazine-1-carboxylate | 410.5 | ** | *** |
| isopropyl 4-(3-(2-fluoropyridin-3-yl)pyrazolo[1,5-a]pyrimidin-5-yl)piperazine-1-carboxylate | 384.4 | ** | *** |
| isopropyl 4-(3-(2-hydroxypyridin-3-yl)pyrazolo[1,5-a]pyrimidin-5-yl)piperazine-1-carboxylate | 382.4 | ** | *** |
| isopropyl 4-(3-(2-isopropoxypyridin-3-yl)pyrazolo[1,5-a]pyrimidin-5-yl)piperazine-1-carboxylate | 424.5 | ** | *** |
| isopropyl 4-(3-(2-methoxy-5-methylpyridin-3-yl)pyrazolo[1,5-a]pyrimidin-5-yl)piperazine-1-carboxylate | 410.5 | *** | *** |
| isopropyl 4-(3-(2-methoxy-6-methylpyridin-3-yl)pyrazolo[1,5-a]pyrimidin-5-yl)piperazine-1-carboxylate | 410.5 | ** | *** |
| isopropyl 4-(3-(2-methoxyphenyl)pyrazolo[1,5-a]pyrimidin-5-yl)piperazine-1-carboxylate | 395.5 | -- | *** |
| isopropyl 4-(3-(2-methoxypyridin-3-yl)pyrazolo[1,5-a]pyrimidin-5-yl)piperazine-1-carboxylate | 396.4 | -- | *** |
| isopropyl 4-(3-(2-methoxypyridin-3-yl)pyrazolo[1,5-a]pyrimidin-5-yl)-5,6-dihydropyridine-1(2H)-carboxylate | 393.4 | *** | *** |
| isopropyl 4-(3-(2-methoxypyridin-3-yl)pyrazolo[1,5-a]pyrimidin-5-yl)-3-oxopiperazine-1-carboxylate | 410.4 | *** | *** |
| isopropyl 4-(3-(2-methoxypyridin-3-yl)pyrazolo[1,5-a]pyrimidin-5-yl)-2,2-dimethylpiperazine-1-carboxylate | 424.5 | -- | ** |
| isopropyl 4-(3-(2-methylpyridin-3-yl)pyrazolo[1,5-a]pyrimidin-5-yl)piperazine-1-carboxylate | 380.4 | -- | ** |
| isopropyl 4-(3-(3,6-dimethoxypyridazin-4-yl)pyrazolo[1,5-a]pyrimidin-5-yl)piperazine-1-carboxylate | 427.5 | *** | *** |
| isopropyl 4-(3-(3-ethoxyphenyl)pyrazolo[1,5-a]pyrimidin-5-yl)piperazine-1-carboxylate | 409.5 | -- | *** |
| isopropyl 4-(3-(3-fluoro-2-methoxyphenyl)pyrazolo[1,5-a]pyrimidin-5-yl)piperazine-1-carboxylate | 413.4 | -- | ** |
| isopropyl 4-(3-(3-methoxypyridin-2-yl)pyrazolo[1,5-a]pyrimidin-5-yl)piperazine-1-carboxylate | 396.4 | -- | * |
| isopropyl 4-(3-(3-methoxypyridin-4-yl)pyrazolo[1,5-a]pyrimidin-5-yl)piperazine-1-carboxylate | 396.4 | ** | *** |
| isopropyl 4-(3-(4-fluoropyridin-3-yl)pyrazolo[1,5-a]pyrimidin-5-yl)piperazine-1-carboxylate | 384.4 | -- | ** |
| isopropyl 4-(3-(4-methoxypyridin-2-yl)pyrazolo[1,5-a]pyrimidin-5-yl)piperazine-1-carboxylate | 396.4 | -- | *** |
| isopropyl 4-(3-(4-methoxypyridin-3-yl)pyrazolo[1,5-a]pyrimidin-5-yl)piperazine-1-carboxylate | 396.4 | ** | *** |
| isopropyl 4-(3-(5-fluoro-2-methoxyphenyl)pyrazolo[1,5-a]pyrimidin-5-yl)piperazine-1-carboxylate | 413.4 | *** | *** |
| isopropyl 4-(3-(5-fluoro-2-methoxypyridin-3-yl)pyrazolo[1,5-a]pyrimidin-5-yl)piperazine-1-carboxylate | 414.4 | ** | *** |
| isopropyl 4-(3-(5-fluoropyridin-3-yl)pyrazolo[1,5-a]pyrimidin-5-yl)piperazine-1-carboxylate | 384.4 | -- | *** |
| isopropyl 4-(3-(5-methoxypyridin-2-yl)pyrazolo[1,5-a]pyrimidin-5-yl)piperazine-1-carboxylate | 396.4 | *** | *** |
| isopropyl 4-(3-(5-methoxypyridin-3-yl)pyrazolo[1,5-a]pyrimidin-5-yl)piperazine-1-carboxylate | 396.4 | ** | *** |
| isopropyl 4-(3-(6-fluoropyridin-3-yl)pyrazolo[1,5-a]pyrimidin-5-yl)piperazine-1-carboxylate | 384.4 | -- | ** |
| isopropyl 4-(3-(6-methoxypyridin-3-yl)pyrazolo[1,5-a]pyrimidin-5-yl)piperazine-1-carboxylate | 396.4 | * | *** |
| isopropyl 4-(3-(ethylcarbamoyl)pyrazolo[1,5-a]pyrimidin-5-yl)piperazine-1-carboxylate | 360.4 | -- | ** |
| ²isopropyl4-(3-(isopropylcarbamoyl)pyrazolo[1,5-a]pyrimidin-5-yl)piperazine-1-carboxylate | 374.4 | -- | * |
| isopropyl4-(3-(methylcarbamoyl)pyrazolo[1,5-a]pyrimidin-5-yl)piperazine-1-carboxylate | 346.4 | -- | ** |
| isopropyl4-(3-(pyrazin-2-yl)pyrazolo[1,5-a]pyrimidin-5-yl)piperazine-1-carboxylate | 367.4 | -- | ** |
| isopropyl4-(3-(pyridin-2-yl)pyrazolo[1,5-a]pyrimidin-5-yl)piperazine-1-carboxylate | 366.4 | ** | *** |
| isopropyl 4-(3-(pyridin-3-yl)pyrazolo[1,5-a]pyrimidin-5-yl)piperazine-1-carboxylate | 366.4 | -- | *** |
| isopropyl4-(3-(pyridin-4-yl)pyrazolo[1,5-a]pyrimidin-5-yl)piperazine-1-carboxylate | 366.4 | ** | *** |
| isopropyl 4-(3-(pyrimidin-5-yl)pyrazolo[1,5-a]pyrimidin-5-yl)piperazine-1-carboxylate | 367.4 | -- | ** |
| isopropyl4-(3-isopropylpyrazolo[1,5-a]pyrimidin-5-yl)piperazine-1-carboxylate | 331.4 | -- | ** |
| isopropyl 4-(pyrazolo[1,5-a]pyrimidin-5-yl)piperazine-1-carboxylate | 289.3 | -- | *** |
| isopropyl methyl(2-((3-(4-((2-(methylamino)ethyl)carbamoyl)phenyl)pyrazolo[1,5-a]pyrimidin-5-yl)amino)ethyl)carbamate | 453.5 | ** | -- |
| isopropyl methyl(2-((3-(4-(methylcarbamoyl)phenyl)pyrazolo[1,5-a]pyrimidin-5-yl)amino)ethyl)carbamate | 410.5 | *** | -- |
| isopropyl methyl(2-((3-(4-(pyrrolidin-2-yl)phenyl)pyrazolo[1,5-a]pyrimidin-5-yl)amino)ethyl)carbamate | 422.5 | *** | -- |
| methyl 4-(3-(2-methoxypyridin-3-yl)pyrazolo[1,5-a]pyrimidin-5-yl)piperazine-1-carboxylate | 368.4 | ** | ** |
| N-(2-(cyclopentyloxy)ethyl)-3-(3-methoxypyridin-4-yl)pyrazolo[1,5-a]pyrimidin-5-amine | 353.4 | ** | -- |
| N-(2-(tert-butoxy)ethyl)-3-(3-methoxypyridin-4-yl)pyrazolo[1,5-a]pyrimidin-5-amine | 341.4 | ** | -- |
| N-(2-aminoethyl)-4-(5-((2-methoxyethyl)amino)pyrazolo[1,5-a]pyrimidin-3-yl)benzamide | 354.4 | * | -- |
| N-(2-aminoethyl)-4-(5-((3,3-dimethylbutyl)amino)pyrazolo[1,5-a]pyrimidin-3-yl)benzamide | 380.5 | * | -- |
| N-(2-aminoethyl)-4-(5-(butylamino)pyrazolo[1,5-a]pyrimidin-3-yl)benzamide | 352.4 | ** | -- |
| N-(2-methoxyethyl)-3-phenylpyrazolo[1,5-a]pyrimidin-5-amine | 268.3 | * | -- |
| N-(3-(2-methoxypyridin-3-yl)pyrazolo[1,5-a]pyrimidin-5-yl)-N-(4,4,4-trifluorobutyl)acetamide | 393.4 | -- | * |
| N-(3-(cyclopentyloxy)propyl)-3-(3-methoxypyridin-4-yl)pyrazolo[1,5-a]pyrimidin-5-amine | 367.4 | ** | -- |
| N-(tert-butyl)-4-(3-(2-methoxypyridin-3-yl)pyrazolo[1,5-a]pyrimidin-5-yl)piperazine-1-carboxamide | 409.5 | ** | *** |
| N-(tert-butyl)-4-(3-(2-methoxypyridin-3-yl)pyrazolo[1,5-a]pyrimidin-5-yl)-N-methylpiperazine-1-carboxamide | 423.5 | ** | *** |
| N-isopropyl-4-(3-(2-methoxypyridin-3-yl)pyrazolo[1,5-a]pyrimidin-5-yl)piperazine-1-carboxamide | 395.5 | *** | *** |
| N-isopropyl-4-(3-(2-methoxypyridin-3-yl)pyrazolo[1,5-a]pyrimidin-5-yl)-N-methylpiperazine-1-carboxamide | 409.5 | ** | *** |
| tert-butyl (1-(3-(2-methoxypyridin-3-yl)pyrazolo[1,5-a]pyrimidin-5-yl)azetidin-3-yl)(methyl)carbamate | 410.5 | -- | * |
| tert-butyl (2-((3-(3-methoxypyridin-4-yl)pyrazolo[1,5-a]pyrimidin-5-yl)amino)ethyl)(methyl)carbamate | 398.5 | *** | -- |
| tert-butyl (2-((3-(4-carbamoylphenyl)pyrazolo[1,5-a]pyrimidin-5-yl)amino)ethyl)(methyl)carbamate | 410.5 | *** | -- |
| tert-butyl (2-(4-(5-((2-methoxyethyl)amino)pyrazolo[1,5-a]pyrimidin-3-yl)benzamido)ethyl)carbamate | 454.5 | * | -- |
| tert-butyl4-(3-(2-methoxypyridin-3-yl)pyrazolo[1,5-a]pyrimidin-5-yl)piperazine-1-carboxylate | 410.5 | *** | *** |
| tert-butyl4-(pyrazolo[1,5-a]pyrimidin-5-yl)-5,6-dihydropyridine-1(2H)-carboxylate | 300.4 | -- | ** |
| tert-butyl methyl(2-((3-(4-(methylcarbamoyl)phenyl)pyrazolo[1,5-a]pyrimidin-5-yl)amino)ethyl)carbamate | 424.5 | *** | -- |

### 5.4.7. Aryl ether-based Inhibitor In Vitro Data

The Caliper and HEK281 assays described above were used to obtain *in vitro* data for various aryl ether-based compounds:

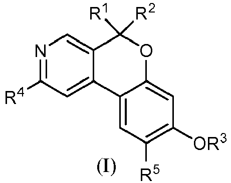

| **R¹** | **R²** | **R⁴** | **R⁵** | **R³** | **Caliper IC₅₀ (nM)** | **CBA IC₅₀** |
|---|---|---|---|---|---|---|
| H | H | H | H | 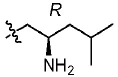 | c | |
| H | H | H | H | 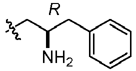 | 293 | |
| H | H | H | H | 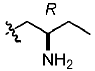 | d | |
| H | H | H | H | 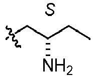 | c | |
| H | H | H | H | 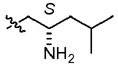 | 3.3 | 8.4 |
| Me | H | H | H | 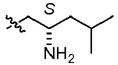 | 4.9 | |
| Me | H | H | H | 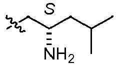 | c | 25 |
| H | Me | H | H | 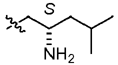 | c | b |
| Me | H | H | H | 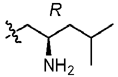 | c | >300 |
| H | Me | H | H | 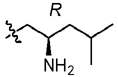 | c | |
| *Cyc*-Pr | H | H | H | 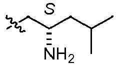 | c | c |
| *Cyc*-Pr | H | H | H | 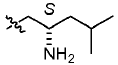 | d | |
| H | Cyc-Pr | H | H | 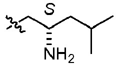 | b | 18 |
| *Cyc*-Pr | H | H | H | 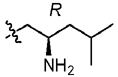 | d | |
| *Cyc*-Pr | H | H | H | 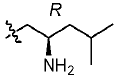 | d | d |
| H | Cyc-Pr | H | H | 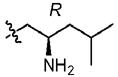 | d | d |
| Et | H | H | Et | 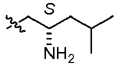 | c | 18 |
| Et | H | H | H | 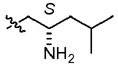 | c | c |
| H | Et | H | H | 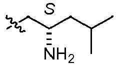 | 12 | 56 |
| =0 | | H | H | 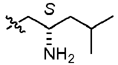 | c | c |
| H | H | NHAc | H | 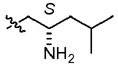 | c | c |
| H | H | NH₂ | H | 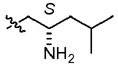 | b | b |
| H | H | H | Br | 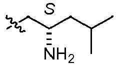 | 0.86 | 2.5 |

| | | | | | | |
|---|---|---|---|---|---|---|
| where: a = <1nM;b = 1-10 nM; c = 10-100 nM; d = 100-1000 nM. | | | | | | |

### 5.4.8. Pharmacological Effects

Studies of AAK1 knockout mice showed that disruption of the AAK1 gene affects pain response as measured using the formalin paw test. See example 5.4.1, above. The same test was used to confirm that the administration of an AAK1 inhibitor can also affect pain response.

Mice were tested for nociception with Automatic Nociception Analyzers (purchased from the Ozaki lab at University of California, San Diego). A metal band was placed around the left hind paw of each mouse with superglue 30 minutes prior to testing. After the 30-minute acclimation period, 20 µl of 5% formalin was subcutaneously injected in the dorsal surface of the left hind paw. Mice were individually housed in cylindrical chambers for 45 minutes. Fresh 5 % formalin solution was prepared by diluting formaldehyde (Formalde-fresh 20%, Fisher Scientific, Fair Lawn, NJ) with distilled water. Investigatory compounds were administered 30 minutes prior to formalin injection.

A computer software recorded flinches per minute, total flinches for Phase I (acute phase = first 8 minutes), and total flinches for Phase II (tonic phase between 20 - 40 minutes) through an electromagnetic field. See Yaksh TL, Ozaki G, McCumber D, Rathbun M, Svensson C, Malkmus S, Yaksh MC. An automated flinch detecting system for use in the formalin nociceptive bioassay. J Appl Physiol., 2001; 90:2386-402.

Various compounds of the invention were tested at different doses. Gabapentin and pregabalin were used as positive controls. Results are shown below in Table 2, wherein "*" means an effect equal to or greater than 50 percent of that of gabapentin at 200 mpk, "**" means an effect equal to or greater than 100 percent of that of gabapentin at 200 mpk, "sc" means subcutaneous administration, "ip" means in intraperitoneal administration, and "po" means oral administration.

**Table 2**

| **Compound** | **Dose (mpk)** | **Effect** |
|---|---|---|
| Gabapentin | 50 sc | * |
| Gabapentin | 200 sc | ** |
| Pregabalin | 50 sc | * |
| (S)-isopropyl (1-(3-(2-methoxypyridin-3-yl)imidazo[1,2-b]pyridazin-6-yl)pyrrolidin-3-yl)(methyl)carbamate | 10 po | * |
| (S)-isopropyl (1-(3-(2-methoxypyridin-3-yl)imidazo[1,2-b]pyridazin-6-yl)pyrrolidin-3-yl)(methyl)carbamate | 30 po | ** |
| (S)-isopropyl (1-(3-(2-methoxypyridin-3-yl)imidazo[1,2-b]pyridazin-6-yl)pyrrolidin-3-yl)(methyl)carbamate | 60 po | ** |
| (S)-tert-butyl 2-(((3-(4-(aminomethyl)phenyl)imidazo[1,2-b]pyridazin-6-yl)amino)methyl)pyrrolidine-1-carboxylate | 60 sc | ** |
| (S)-tert-butyl 2-(((3-bromoimidazo[1,2-b]pyridazin-6-yl)amino)methyl)pyrrolidine-1-carboxylate | 30 sc | ** |
| (S)-tert-butyl 2-(((3-bromoimidazo[1,2-b]pyridazin-6-yl)amino)methyl)pyrrolidine-1-carboxylate | 60 sc | ** |
| 3-(4-(1H-tetrazol-5-yl)phenyl)-N-butylimidazo[1,2-b]pyridazin-6-amine | 60 ip | ** |
| 3-(4-(aminomethyl)phenyl)-N-(2-(cyclopentyloxy)ethyl)imidazo[1,2-b]pyridazin-6-amine | 60 sc | ** |
| 3-(4-(aminomethyl)phenyl)-N-(2-(cyclopentyloxy)ethyl)imidazo[1,2-b]pyridazin-6-amine | 100 sc | ** |
| 3-(4-(aminomethyl)phenyl)-N-(2-(tert-butoxy)ethyl)imidazo[1,2-b]pyridazin-6-amine | 60 sc | ** |
| 3-(4-(aminomethyl)phenyl)-N-(3-(tert-butoxy)propyl)imidazo[1,2-b]pyridazin-6-amine | 60 sc | ** |
| 3-(4-(aminomethyl)phenyl)-N-(3-phenylpropyl)imidazo[1,2-b]pyridazin-6-amine | 30 ip | ** |
| isopropyl 4-(3-(2-methoxyphenyl)imidazo[1,2-b]pyridazin-6-yl)piperazine-1-carboxylate | 30 sc | ** |
| isopropyl 4-(3-(2-methoxyphenyl)imidazo[1,2-b]pyridazin-6-yl)piperazine-1-carboxylate | 60 sc | ** |
| N-(2-aminoethyl)-4-(6-(butylamino)imidazo[1,2-b]pyridazin-3-yl)benzamide | 30 ip | ** |
| tert-butyl (2-((3-(4-carbamoylphenyl)imidazo[1,2-b]pyridazin-6-yl)amino)ethyl)(methyl)carbamate | 100 po | ** |
| tert-butyl (2-((3-(4-carbamoylphenyl)imidazo[1,2-b]pyridazin-6-yl)amino)ethyl)(methyl)carbamate | 60 sc | ** |
| (S)-tert-butyl 2-(((3-(2-methoxyphenyl)pyrazolo[1,5-a]pyrimidin-5-yl)amino)methyl)pyrrolidine-1-carboxylate | 10 sc | * |
| (S)-tert-butyl 2-(((3-(2-methoxyphenyl)pyrazolo[1,5-a]pyrimidin-5-yl)amino)methyl)pyrrolidine-1-carboxylate | 60 sc | * |
| (S)-tert-butyl 2-(((3-(pyridin-4-yl)pyrazolo[1,5-a]pyrimidin-5-yl)amino)methyl)pyrrolidine-1-carboxylate | 30 sc | ** |
| isopropyl 4-(3-(2-methoxyphenyl)pyrazolo[1,5-a]pyrimidin-5-yl)piperazine-1-carboxylate | 30 sc | ** |
| isopropyl 4-(3-(2-methoxypyridin-3-yl)pyrazolo[1,5-a]pyrimidin-5-yl)piperazine-1-carboxylate | 10 po | * |
| isopropyl 4-(3-(2-methoxypyridin-3-yl)pyrazolo[1,5-a]pyrimidin-5-yl)piperazine-1-carboxylate | 30 po | ** |
| (*R*)-1-(5*H*-chromeno[3,4-*c*]pyridin-8-yloxy)-4-methylpentan-2-amine | 10 sc | 45% |
| (*R*)-1-(5*H*-chromeno[3,4-*c*]pyridin-8-yloxy)-4-methylpentan-2-amine | 30 sc | * |

These results demonstrate that AAK1 inhibitors can be used to treat pain.

## Claims

1. An inhibitor of adaptor associated kinase 1 (AAK1) activity for use in treating or managing fibromyalgia, wherein said inhibitor is a compound selected from the group consisting of:
a) compounds of the formula: 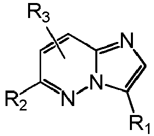 and pharmaceutically acceptable salts thereof, wherein: R₁ is R_{1A} or optionally substituted C₁₋₁₂ hydrocarbyl or 2-12-membered heterocarbyl, which optional substitution is with one or more R_{1A}; each R_{1A} is independently -OR_{1C}, -N(R_{1C})₂, -C(O)R_{1C}, -C(O)OR_{1C},-C(O)N(R_{1C})₂, -N(R_{1C})C(O)OR_{1C}, cyano, halo, or optionally substituted C₁₋₁₂ hydrocarbyl or 2-12-membered heterocarbyl, which optional substitution is with one or more R_{1B}; each R_{1B} is independently -OR_{1C}, -N(R_{1C})₂, -C(O)R_{1C}, -C(O)OR_{1C}, -C(O)N(R_{1C})₂, -N(R_{1C})C(O)OR_{1C}, cyano or halo; each R_{1C} is independently hydrogen or optionally substituted C₁₋₁₂ hydrocarbyl or 2-12-membered heterocarbyl, which optional substitution is with one or more of cyano, halo or hydroxyl; R₂ is -NR_{2A}R_{2B}, wherein R_{2A} is hydrogen and R_{2B} is optionally substituted C₁₋₁₂ hydrocarbyl or 2-12-membered heterocarbyl, which optional substitution is with one or more R_{2C}; or R_{2A} and R_{2B} are taken together to form a 4-7-membered heterocycle optionally substituted with one or more R_{2C}; each R_{2C} is independently -OR_{2D}, -N(R_{2D})₂, -C(O)R_{2D},-C(O)OR_{2D}, -C(O)N(R_{2D})₂, -N(R_{2D})C(O)OR_{2D}, cyano, halo, or optionally substituted C₁₋₁₂ hydrocarbyl or 2-12-membered heterocarbyl, which optional substitution is with one or more with one or more R_{2D}; each R_{2D} is independently hydrogen or optionally substituted C₁₋₁₂ hydrocarbyl or 2-12-membered heterocarbyl, which optional substitution is with one or more of cyano, halo or hydroxyl; and R₃ is hydrogen or C₁₋₆ alkyl optionally substituted with one or more of cyano, halo or hydroxyl;
b) compounds of the formula: 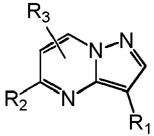 and pharmaceutically acceptable salts thereof, wherein: R₁ is R_{1A} or optionally substituted C₁₋₁₂ hydrocarbyl or 2-12-membered heterocarbyl, which optional substitution is with one or more R_{1A}; each R_{1A} is independently -OR_{1C}, -N(R_{1C})₂, -C(O)R_{1C}, -C(O)OR_{1C},-C(O)N(R_{1C})₂, -N(R_{1C})C(O)OR_{1C}, cyano, halo, or optionally substituted C₁₋₁₂ hydrocarbyl or 2-12-membered heterocarbyl, which optional substitution is with one or more R_{1B}; each R_{1B} is independently -OR_{1C}, -N(R_{1C})₂, -C(O)R_{1C}, -C(O)OR_{1C}, -C(O)N(R_{1C})₂, -N(R_{1C})C(O)OR_{1C}, cyano or halo; each R_{1C} is independently hydrogen or optionally substituted C₁₋₁₂ hydrocarbyl or 2-12-membered heterocarbyl, which optional substitution is with one or more of cyano, halo or hydroxyl; R₂ is -NR_{2A}R_{2B}, wherein R_{2A} is hydrogen and R_{2B} is optionally substituted C₁₋₁₂ hydrocarbyl or 2-12-membered heterocarbyl, which optional substitution is with one or more R_{2C}; or R_{2A} and R_{2B} are taken together to form a 4-7-membered heterocycle optionally substituted with one or more R_{2C}; each R_{2C} is independently -OR_{2D}, -N(R_{2D})₂, -C(O)R_{2D},-C(O)OR_{2D}, -C(O)N(R_{2D})₂, -N(R_{2D})C(O)OR_{2D}, cyano, halo, or optionally substituted C₁₋₁₂ hydrocarbyl or 2-12-membered heterocarbyl, which optional substitution is with one or more with one or more R_{2D}; each R_{2D} is independently hydrogen or optionally substituted C₁₋₁₂ hydrocarbyl or 2-12-membered heterocarbyl, which optional substitution is with one or more of cyano, halo or hydroxyl; and R₃ is hydrogen or C₁₋₆ alkyl optionally substituted with one or more of cyano, halo or hydroxyl; and
c) compounds of the formula: 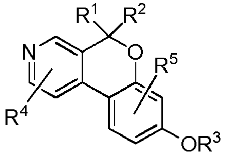 and pharmaceutically acceptable salts thereof, wherein: R¹ and R² are independently selected from hydrogen, C₃-C₆cycloalkyl, and C₁-C₃alkyl wherein the C₁-C₃alkyl is optionally substituted with one, two, or three groups independently selected from C₁-C₃alkoxy, C₁-C₃alkylamino, amino, cyano, C₁-C₃dialkylamino, halo, and hydroxy; or R¹ and R² together are oxo; R³ is C₁-C₃alkyl-Y or C₂-C₈alkyl, wherein the C₂-C₈alkyl is optionally substituted with one, two, or three groups independently selected from C₁-C₃alkoxy, C₁-C₃alkylamino, C₁-C₃alkoxyC₂-C₃alkylamino, amino, aryl, halo, C₁-C₃haloalkylamino, C₁-C₃haloalkylcarbonylamino, hydroxy, -NR^{x}R^{y}, and C₃-C₈cycloalkyl, wherein the cycloalkyl is further optionally substituted with one, two, or three groups independently selected from C₁-C₃alkoxy, C₁-C₃alkyl, C₁-C₃alkylamino, C₁-C₃alkoxyC₂-C₃alkylamino, amino, aryl, arylC₁-C₃alkyl, halo, C₁-C₃haloalkyl, C₁-C₃haloalkylamino and hydroxy; R⁴ is selected from hydrogen, C₁-C₃alkoxy, C₁-C₃alkoxycarbonylamino, C₁-C₃alkyl, C₁-C₃alkylamino, C₁-C₃alkylcarbonylamino, amino, arylamino, arylcarbonylamino, C₃-C₆cycloalkylamino, C₃-C₆cycloalkylcarbonylamino, C₃-C₆cycloalkyloxy, halo, C₁-C₃haloalkoxy, C₂-C₃haloalkylamino, C₂-C₃haloalkylcarbonylamino, and hydroxy; R⁵ is selected from hydrogen, C₁-C₃alkyl, cyano, C₃cycloalkyl, and halo; R^{x} and R^{y}, together with the nitrogen atom to which they are attached, form a three- to six-membered ring; and Y is selected from 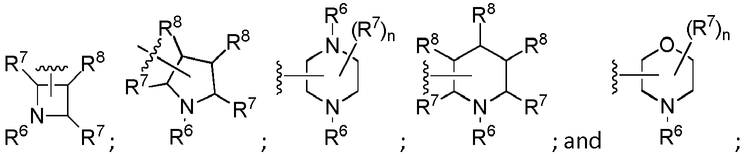 wherein R⁶ is selected from hydrogen, C₁-C₆alkyl, C₃-C₆cycloalkyl, and C₁-C₆alkylcarbonyl; n is 0, 1, 2, or 3; each R⁷ is independently selected from hydrogen, C₁-C₆alkyl, aryl, arylC₁-C₃alkyl, C₃-C₆cycloalkyl, halo, and C₁-C₃haloalkyl; and each R⁸ is independently selected from hydrogen, C₁-C₃alkoxy and hydroxy.

## Patentansprüche

1. Hemmer (Inhibitor) der Adapter- assoziierten Kinase 1 (AAK1)- Aktivität zur Verwendung bei der Behandlung oder dem Management von Fibromyalgie, wobei der Hemmer eine Verbindung ist, die aus der Gruppe ausgewählt ist, welche besteht aus:
a) Verbindungen der Formel: 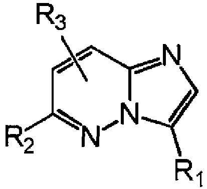 und pharmazeutisch akzeptablen Salzen derselben, wobei: R₁ für R_{1A} steht oder ein gegebenenfalls substituiertes C₁₋₁₂-Hydrocarbyl oder 2-12- gliedriges Heterocarbyl ist, wobei die gegebenenfalls vorhandene Substitution mit einem R_{1A} oder mehreren R_{1A} erfolgt; jedes R_{1A} unabhängig -OR_{1c}, -N(R_{1c})₂,-C(O)R_{1c}, -C(O)OR_{1c}, -C(O)N(R_{1c})₂, -N(R_{1c})C(O)OR_{1c}, ein Cyano, ein Halogen oder ein gegebenenfalls substituiertes C₁₋₁₂-Hydrocarbyl oder 2-12- gliedriges Heterocarbyl ist, wobei die gegebenenfalls vorhandene Substitution mit einem R_{1B} oder mehreren R_{1B} erfolgt; jedes R_{1B} unabhängig -OR_{1c}, -N(R_{1c})₂,-C(O)R_{1c}, -C(O)OR_{1c}, -C(O)N(R_{1c})₂, -N(R_{1c})C(O)OR_{1c}, ein Cyano oder ein Halogen ist; jedes R_{1C} unabhängig ein Wasserstoffatom oder ein gegebenenfalls substituiertes C₁₋₁₂- Hydrocarbyl oder 2-12-gliedriges Heterocarbyl ist, wobei die gegebenenfalls vorhandene Substitution mit einem oder mehreren von einem Cyano, einem Halogen oder einem Hydroxyl erfolgt; R₂ für -NR_{2A}R_{2B} steht, wobei R_{2A} ein Wasserstoffatom ist und R_{2B} ein gegebenenfalls substituiertes C₁₋₁₂- Hydrocarbyl oder 2-12-gliedriges Heterocarbyl ist, wobei die gegebenenfalls vorhandene Substitution mit einem R_{2C} oder mehreren R_{2C} erfolgt; oder R_{2A} und R_{2B} zusammengenommen werden, um einen 4-7-gliedrigen Heterocyclus zu bilden, der gegebenenfalls mit einem R_{2C} oder mehreren R_{2C} substituiert ist ; jedes R_{2c} unabhängig -OR_{2D}, -N(R_{2D})₂, -C(O)R_{2D}, -C(O)OR_{2D}, -C(O)N(R_{2D})₂,-N(R_{2D})C(O)OR_{2D}, ein Cyano, ein Halogen oder ein gegebenenfalls substituiertes C₁₋₁₂- Hydrocarbyl oder 2-12- gliedriges Heterocarbyl ist, wobei die gegebenenfalls vorhandene Substitution mit einem R_{2D} oder mehreren R_{2D} erfolg ; jedes R_{2D} unabhängig ein Wasserstoffatom oder ein gegebenenfalls substituiertes C₁₋₁₂- Hydrocarbyl oder 2-12- gliedriges Heterocarbyl ist, wobei die gegebenenfalls vorhandene Substitution mit einem oder mehreren von einem Cyano, einem Halogen oder einem Hydroxyl erfolgt; und R₃ ein Wasserstoffatom ist oder ein C₁₋₆- Alkyl, das gegebenenfalls mit einem oder mehreren von einem Cyano, einem Halogen oder einem Hydroxyl substituiert ist, ist;
b) Verbindungen der Formel: 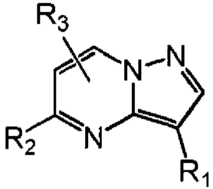 und pharmazeutisch akzeptablen Salzen derselben, wobei: R₁ für R_{1A} steht oder ein gegebenenfalls substituiertes C₁₋₁₂-Hydrocarbyl oder 2-12- gliedriges Heterocarbyl ist, wobei die gegebenenfalls vorhandene Substitution mit einem R_{1A} oder mehreren R_{1A} erfolgt; jedes R_{1A} unabhängig -OR_{1c},-N(R_{1c})₂, -C(O)R_{1c}, -C(O)OR_{1c}, -C(O)N(R_{1c})₂, -N(R_{1c})C(O)OR_{1c}, ein Cyano, ein Halogen oder ein gegebenenfalls substituiertes C₁₋₁₂-Hydrocarbyl oder 2-12- gliedriges Heterocarbyl ist, wobei die gegebenenfalls vorhandene Substitution mit einem R_{1B} oder mehreren R_{1B} erfolgt; jedes R_{1B} unabhängig -OR_{1c}, -N(R_{1c})₂,-C(O)R_{1c}, -C(O)OR_{1c}, -C(O)N(R_{1c})₂, -N(R_{1c})C(O)OR_{1c}, ein Cyano oder ein Halogen ist; jedes R_{1C} unabhängig ein Wasserstoffatom oder ein gegebenenfalls substituiertes C₁₋₁₂- Hydrocarbyl oder 2-12-gliedriges Heterocarbyl ist, wobei die gegebenenfalls vorhandene Substitution mit einem oder mehreren von einem Cyano, einem Halogen oder einem Hydroxyl erfolgt; R₂ für -NR_{2A}R_{2B} steht, wobei R_{2A} ein Wasserstoffatom ist und R_{2B} ein gegebenenfalls substituiertes C₁₋₁₂- Hydrocarbyl oder 2-12-gliedriges Heterocarbyl ist, wobei die gegebenenfalls vorhandene Substitution mit einem R_{2C} oder mehreren R_{2C} erfolgt ; oder R_{2A} und R_{2B} zusammengenommen werden, um einen 4-7-gliedrigen Heterocyclus zu bilden, der gegebenenfalls mit einem R_{2C} oder mehreren R_{2C} substituiert ist; jedes R_{2c} unabhängig -OR_{2D}, -N(R_{2D})₂, -C(O)R_{2D}, -C(O)OR_{2D}, -C(O)N(R_{2D})₂,-N(R_{2D})C(O)OR_{2D}, ein Cyano, ein Halogen oder ein gegebenenfalls substituiertes C₁₋₁₂- Hydrocarbyl oder 2-12- gliedriges Heterocarbyl ist, wobei die gegebenenfalls vorhandene Substitution mit einem R_{2D} oder mehreren R_{2D} erfolgt; jedes R_{2D} unabhängig ein Wasserstoffatom oder ein gegebenenfalls substituiertes C₁₋₁₂- Hydrocarbyl oder 2-12- gliedriges Heterocarbyl ist, wobei die gegebenenfalls vorhandene Substitution mit einem oder mehreren von einem Cyano, einem Halogen oder einem Hydroxyl erfolgt; und R₃ ein Wasserstoffatom ist oder ein C₁₋₆- Alkyl, das gegebenenfalls mit einem oder mehreren von einem Cyano, einem Halogen oder einem Hydroxyl substituiert ist, ist; und
c) Verbindungen der Formel: 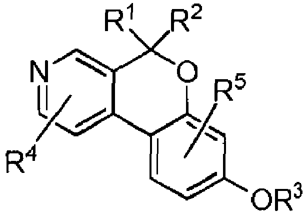 und pharmazeutisch akzeptablen Salzen derselben, wobei: R¹ und R² unabhängig voneinander ausgewählt sind aus: einem Wasserstoffatom, einem C₃-C₆- Cycloalkyl und einem C₁-C₃- Alkyl, wobei das C₁-C₃- Alkyl gegebenenfalls mit einer, zwei oder drei Gruppe(n) substituiert ist, die unabhängig aus einem C₁-C₃-Alkoxy, einem C₁-C₃- Alkylamino, einem Amino, einem Cyano, einem C₁-C₃- Dialkylamino, einem Halogen und einem Hydroxy ausgewählt ist / sind; oder R¹ und R² zusammen ein Oxo sind; R³ ein C₁-C₃- Alkyl-Y oder ein C₂-C₈- Alkyl ist, wobei das C₂-C₈-Alkyl gegebenenfalls mit einer, zwei oder drei Gruppe(n) substituiert ist, die unabhängig aus einem C₁-C₃- Alkoxy, einem C₁-C₃- Alkylamino, einem C₁-C₃- AlkoxyC₂-C₃- alkylamino, einem Amino, einem Aryl, einem Halogen, einem C₁-C₃-Halogenalkylamino, einem C₁-C₃- Halogenalkylcarbonylamino, einem Hydroxy, -NR^{x}R^{Y}, und einem C₃-C₈- Cycloalkyl ausgewählt ist / sind, wobei das Cycloalkyl ferner gegebenenfalls mit einer, zwei oder drei Gruppe(n) substituiert ist, die unabhängig aus einem C₁-C₃- Alkoxy, einem C₁-C₃- Alkyl, einem C₁-C₃- Alkylamino, einem C₁-C₃- AlkoxyC₂-C₃- alkylamino, einem Amino, einem Aryl, einem ArylC₁-C₃- alkyl, einem Halogen, einem C₁-C₃- Halogenalkyl, einem C₁-C₃- Halogenalkylamino und einem Hydroxy ausgewählt ist / sind; R⁴ aus einem Wasserstoffatom, einem C₁-C₃- Alkoxy, einem C₁-C₃- Alkoxycarbonylamino, einem C₁-C₃- Alkyl, einem C₁-C₃- Alkylamino, einem C₁-C₃-Alkylcarbonylamino, einem Amino, einem Arylamino, einem Arylcarbonylamino, einem C₃-C₆- Cycloalkylamino, einem C₃-C₆-Cycloalkylcarbonylamino, einem C₃-C₆- Cycloalkyloxy, einem Halogen, einem C₁-C₃- Halogenalkoxy, einem C₂-C₃-Halogenalkylamino, einem C₂-C₃- Halogenalkylcarbonylamino, und einem Hydroxy ausgewählt ist; R⁵ aus einem Wasserstoffatom, einem C₁-C₃- Alkyl, einem Cyano, einem C₃- Cycloalkyl und einem Halogen ausgewählt ist; R^{x} und R^{Y}, zusammen mit dem Stickstoffatom, an das sie gebunden sind, einen drei- bis sechsgliedrigen Ring bilden; und Y aus 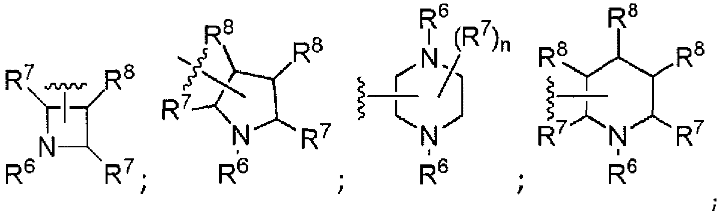 und 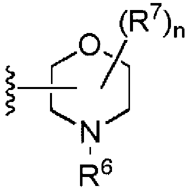 ausgewählt ist;
wobei R⁶ aus einem Wasserstoffatom, einem C₁-C₆- Alkyl, einem C₃-C₆- Cycloalkyl und einem C₁-C₆- Alkylcarbonyl ausgewählt ist; n 0, 1, 2 oder 3 ist; jedes R⁷ unabhängig aus einem Wasserstoffatom, einem C₁-C₆- Alkyl, einem Aryl, einem ArylC₁-C₃- alkyl, einem C₃-C₆- Cycloalkyl, einem Halogen, und einem C₁-C₃- Halogenalkyl ausgewählt ist; und jedes R⁸ unabhängig aus einem Wasserstoffatom, einem C₁-C₃- Alkoxy und einem Hydroxy ausgewählt ist.

## Revendications

1. Un inhibiteur de l'activité de la kinase associée à l'adaptateur 1 (AAK1) pour une utilisation dans le traitement ou la gestion de la fibromyalgie, dans lequel ledit inhibiteur est un composé choisi dans le groupe consistant en :
a) des composés présentant la formule: 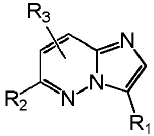 et les sels pharmaceutiquement acceptables en dérivant,
formule dans laquelle:
- R₁ est R_{1A} ou un groupe hydrocarbyle en C₁₋₁₂ éventuellement substitué ou un groupe hétérocarbyle comportant de 2 à 12 atomes éventuellement substitué, les substituants éventuels étant un ou plusieurs R_{1A};
- chaque R_{1A} est, indépendamment l'un de l'autre, -OR_{1C}, -N(R_{1C})₂, -C(O)R_{1C}, -C(O)OR_{1C}, -C(O)N(R_{1C})₂, -N(R_{1C})C(O)OR_{1C}, cyano, halo ou un groupe hydrocarbyle en C₁₋₁₂ éventuellement substitué ou un groupe hétérocarbyle comportant de 2 à 12 atomes éventuellement substitué, les substituants éventuels étant un ou plusieurs R_{1B};
- chaque R_{1B} est, indépendamment l'un de l'autre, -OR_{1C}, -N(R_{1C})₂, -C(O)R_{1C}, -C(O)OR_{1C}, -C(O)N(R_{1C})₂, -N(R_{1C})C(O)OR_{1C}, cyano ou halo;
- chaque R_{1C} est, indépendamment l'un de l'autre, un atome d'hydrogène ou un groupe hydrocarbyle en C₁₋₁₂ éventuellement substitué ou un groupe hétérocarbyle comportant de 2 à 12 atomes éventuellement substitué, les substituants éventuels étant un ou plusieurs groupes cyano, halo ou hydroxyle;
- R₂ est -NR_{2A}R_{2B}, où R_{2A} est un atome d'hydrogène et R_{2B} est un groupe hydrocarbyle en C₁₋₁₂ éventuellement substitué ou un groupe hétérocarbyle comportant de 2 à 12 atomes éventuellement substitué, les substituants éventuels étant un ou plusieurs R_{2C} ;
- ou R_{2A} et R_{2B}, pris ensemble forment un hétérocycle à 4-7 atomes, éventuellement substitué par un ou plusieurs R_{2C};
- chaque R_{2C} est, indépendamment l'un de l'autre, -OR_{2D}, -N(R_{2D})₂, -C(O)R_{2D}, -C(O)OR_{2D}, -C(O)N(R_{2D})₂, -N(R_{2D})C(O)OR_{2D}, cyano, halo ou un groupe hydrocarbyle en C₁₋₁₂ éventuellement substitué ou un groupe hétérocarbyle comportant de 2 à 12 atomes éventuellement substitué, les substituants éventuels étant un ou plusieurs R_{2D};
- chaque R_{2D} est, indépendamment l'un de l'autre, un atome d'hydrogène ou un groupe hydrocarbyle en C₁₋₁₂ éventuellement substitué ou un groupe hétérocarbyle comportant de 2 à 12 atomes éventuellement substitué, les substituants éventuels étant un ou plusieurs cyano, halo ou hydroxyle; et
- R₃ est un atome d'hydrogène ou un groupe C₁₋₆-alkyle éventuellement substitué par un ou plusieurs cyano, halo ou hydroxyle ;
b) composés présentant la formule : 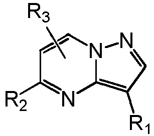 et les sels pharmaceutiquement acceptables en dérivant, dans lesquels:
- R₁ est R_{1A} ou un groupe hydrocarbyle en C₁₋₁₂ éventuellement substitué ou un groupe hétérocarbyle comportant de 2 à 12 atomes éventuellement substitué, les substituants éventuels étant un ou plusieurs R_{1A} ;
- chaque R_{1A} est, indépendamment l'un de l'autre, -OR_{1C}, -N(R_{1C})₂, -C(O)R_{1C}, -C(O)OR_{1C}, -C(O)N(R_{1C})₂, -N(R_{1C})C(O)OR_{1C}, cyano, halo ou un groupe hydrocarbyle en C₁₋₁₂ éventuellement substitué ou un groupe hétérocarbyle comportant de 2 à 12 atomes éventuellement substitué, les substituants éventuels étant un ou plusieurs R_{1B};
- chaque R_{1B} est, indépendamment l'un de l'autre, -OR_{1C}, -N(R_{1C}), -C(O)R_{1C}, -C(O)OR_{1C}, -C(O)N(R_{1C})₂, -N(R_{1C})C(O)OR_{1C}, cyano ou halo;
- chaque R_{1C} est, indépendamment l'un de l'autre, un atome d' hydrogène ou un groupe hydrocarbyle en C₁₋₁₂ éventuellement substitué ou un groupe hétérocarbyle comportant de 2 à 12 atomes éventuellement substitué, les substituants éventuels étant un ou plusieurs groupes cyano, halo ou hydroxyle;
- R₂ est -NR_{2A}R_{2B}, où R_{2A} est un atome d'hydrogène et R_{2B} est un groupe hydrocarbyle en C₁₋₁₂ éventuellement substitué ou un groupe hétérocarbyle comportant de 2 à 12 atomes éventuellement substitué, les substituants éventuels étant un ou plusieurs R_{2C} ;
- ou R_{2A} et R_{2B}, pris ensemble forment un hétérocycle à 4-7 atomes, éventuellement substitué par un ou plusieurs R_{2C};
- chaque R_{2C} est, indépendamment l'un de l'autre, -OR_{2D}, -N(R_{2D})₂, -C(O)R_{2D}, -C(O)OR_{2D}, -C(O)N(R_{2D})₂, -N(R_{2D})C(O)OR_{2D}, cyano, halo ou un groupe hydrocarbyle en C₁₋₁₂ éventuellement substitué ou un groupe hétérocarbyle comportant de 2 à 12 atomes éventuellement substitué, les substituants éventuels étant un ou plusieurs R_{2D};
- chaque R_{2D} est, indépendamment l'un de l'autre, un atome d'hydrogène ou un groupe hydrocarbyle en C₁₋₁₂ éventuellement substitué ou un groupe hétérocarbyle comportant de 2 à 12 atomes éventuellement substitué, les substituants éventuels étant un ou plusieurs cyano, halo ou hydroxyle ; et
- R₃ est un atome d'hydrogène ou un groupe C₁₋₆-alkyle éventuellement substitué par un ou plusieurs cyano, halo ou hydroxyle ; et
c) composés présentant la formule: 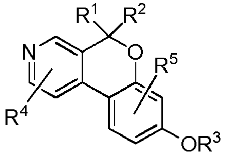 et les sels pharmaceutiquement acceptables en dérivant, dans lesquels:
- R¹ et R² sont choisis, indépendamment l'un de l'autre, parmi l'atome d'hydrogène et les groupes C₃-C₆cycloalkyle et C₁-C₃alkyle, le groupe C₁-C₃alkyle étant éventuellement substitué par un, deux ou trois groupes choisis, indépendamment l'un de l'autre, parmi C₁-C₃alkoxy, C₁-C₃alkylamino, amino, cyano, C₁-C₃dialkylamino, halo et hydroxy;
- ou R¹ et R² forment ensemble un groupe oxo,
- R³ est un groupe C₁-C₃alkyl-Y ou un groupe C₂-C₈alkyle, ce groupe C₂-C₈alkyle étant éventuellement substitué par un, deux ou trois groupes choisis, indépendamment l'un de l'autre, parmi C₁-C₃alkoxy, C₁-C₃alkylamino, C₁-C₃alkoxyC₂-C₃alkylamino, amino, aryle, halo, C₁-C₃haloalkylamino, C₁-C₃haloalkylcarbonylamino, hydroxy, -NR^{X}R^{Y}, et C₃-C₈ cycloalkyle, ce groupe cycloalkyle étant en outre éventuellement substitué par un, deux ou trois groupes choisis, indépendamment l'un de l'autre, parmi C₁-C₃ alkoxy, C₁-C₃alkyle, C₁-C₃alkylamino, C₁-C₃alkoxyC₂-C₃alkylamino, amino, aryle, arylC₁-C₃alkyle, halo, C₁-C₃haloalkyle, C₁-C₃haloalkylamino et hydroxy;
- R⁴ est choisi parmi l'atome d'hydrogène, les groupes C₁-C₃alkoxy, C₁-C₃-alkoxycarbonylamino, C₁-C₃-alkyle, C₁-C₃-alkylamino, C₁-C₃-alkyl-carbonylamino, amino, arylamino, arylcarbonylamino, C₃-C₆cycloalkylamino, C₃-C₆cycloalkylcarbonylamino, C₃-C₆cycloalkyloxy, halo, C₁-C₃haloalkoxy, C₂-C₃haloalkylamino, C₂-C₃haloalkylcarbonylamino, et hydroxy ;
- R⁵ est choisi parmi l'atome d'hydrogène, C₁-C₃alkyle, cyano, C₃cycloalkyle, et halo;
- R^{X} et R^{Y} forment ensemble avec l'atome d'azote auquel ils sont liés un noyau comprenant de 3 à 6 atomes ; et Y est choisi parmi les groups 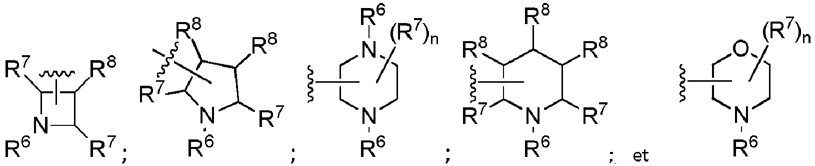 dans lesquels:
- R⁶ est choisi parmi l'atome d'hydrogène, les groupes C₁-C₆alkyle, C₃-C₆cycloalkyle, et C₁-C₆alkylcarbonyle ;
- n est 0, 1, 2, ou 3;
- chaque R⁷ est choisi, indépendamment l'un de l'autre, parmi l'atome d'hydrogène, C₁-C₆alkyle, aryle, arylC₁-C₃alkyle, C₃-C₆cycloalkyle, halo, et C₁-C₃haloalkyle; et
- chaque R⁸ est choisi, indépendamment l'un de l'autre, parmi l'atome d'hydrogène, C₁-C₃alkoxy et hydroxy.
